# EUROPEAN PATENT SPECIFICATION

(11) **EP 2 887 982 B1**
(45) Date of publication and mention of the grant of the patent: **23.11.2022**
(21) Application number: 13833318.2
(22) Date of filing: 27.08.2013
(51) Int. Cl.: A61M 37/00, A61F 9/00, A61B 5/15, A61M 5/32

(54) **APPARATUS FOR DRUG DELIVERY USING MICRONEEDLES**
VORRICHTUNG ZUR ARZNEIMITTELVERABREICHUNG MIT MIKRONADELN
APPAREIL D'ADMINISTRATION DE MÉDICAMENTS À L'AIDE DE MICRO-AIGUILLES

(30) Priority: 27.08.2012 US 201261693542 P; 07.09.2012 US 201261698254 P; 18.01.2013 US 201361754495 P; 01.02.2013 US 201361759771 P; 14.03.2013 US 201361784817 P
(43) Date of publication of application: 01.07.2015
(73) Proprietor: Clearside Biomedical, Inc., Alpharetta, GA 30005 (US); Georgia Tech Research Corporation, Atlanta, GA 30332 (US); Emory University, Atlanta, GA 30322 (US)
(72) Inventor: PRAUSNITZ, Mark R., Atlanta, GA 30307 (US); EDELHAUSER, Henry F., Atlanta, GA 30350 (US); ZARNITSYN, Vladimir, Atlanta, GA 30341 (US); PATEL, Samirkumar, Atlanta, GA 30318 (US); GROSSNIKLAUS, Hans, Atlanta, GA 30322 (US)
(74) Representative: Patentanwaltskanzlei Matschnig & Forsthuber OG
(86) International application number: PCT/US2013/056863
(87) International publication number: WO 2014/036009

(56) References cited:
- US-A- 5 788 679
- US-A1- 2003 009 113
- US-A1- 2007 060 927
- US-A1- 2007 260 201
- US-A1- 2009 030 381
- US-A1- 2009 105 749
- US-A1- 2009 105 749
- US-A1- 2011 202 012
- US-A1- 2012 083 727
- US-B1- 7 678 078
- US-B2- 8 197 435

## Description

### Background

The embodiments described herein relate generally to the field of ophthalmic therapies and more particularly to an apparatus comprising a microneedle for delivery and/or removal of a substance, such as a fluid therapeutic agent into and/or from ocular tissues for treatment of the eye.

Although needles have been used in transdermal and intraocular drug delivery, there remains a need for improved microneedle devices and methods, particularly for delivery of substances (e.g., drugs) into the targeted regions of the eye. Many inflammatory and proliferative diseases in the posterior region of the eye require long term pharmacological treatment. Examples of such diseases include macular degeneration, diabetic retinopathy, and uveitis. It is often difficult to deliver effective doses of a drug to the posterior region of the eye using conventional delivery methods such as topical application, which has poor efficacy, and systemic administration, which often causes significant side effects. For example, while eye drops are useful in treating conditions affecting the exterior surface of the eye or tissues at the front of the eye, the eye drops are not significantly carried to the back of the eye, as may be desired for the treatment of some of the retinal diseases listed above.

Although there have been advances in the past decade regarding the utilization of ocular injection and systemically delivered substances for the treatment of ocular disorders, obstacles still exist. For example, direct injection into the eye (e.g., into a portion of the sclera and/or the vitreous) using conventional 27 gauge or 30 gauge needles and syringes can be effective but often requires professional training and raises safety concerns. Moreover, the anatomy of the eye can make insertion of a conventional 27 gauge or 30 gauge needle into ocular tissue challenging. For example, the eye has a lower modulus of elasticity than skin, and thus will deform more readily in response to an applied force compared to deformation of the skin in response to the same applied force. Accordingly, conventional needles that are designed to pierce skin or other tissue may not be suitable for piercing ocular tissue.

In addition, many known methods of direct injection of a drug into the eye include inserting a needle or a cannula at an acute angle relative to a surface of the eye, which can make controlling the depth of insertion challenging. For example, some such methods include controlling the angular orientation of the needle such that the injected substance exits the needle at a particular locations. Moreover, some known methods of injecting substances into ocular tissue include using complicated visualization system or sensors to control the placement of the needle or cannula.

Moreover, in some instances, such as when treating intraocular tumors, tumors seeds and/or precancerous tissue within the vitreous can be spread through the passageway defined by the insertion of the needle (i.e., a needle tract), which can increase the risk of complications from the tumor. Thus, known methods that result in multiple needle tracts, needle tracts having a large diameter and/or length can result in increased risk of complications.

In some instances, the relative size of the anatomy of the eye can present challenges to treatment of ocular disease. For example, in the treatment of retinoblastoma in pediatric cases, the target insertion site of a needle (e.g., the ciliary body) is significantly smaller than a corresponding target insertion site in an adult case. In such instances, the precise placement of the needle can present a challenge for physicians, resulting in an increased chance of tissue damage and an increase in cost of the procedure. In addition, the anatomy of the eye in pediatric cases can be such that a standard 27 gauge or 30 gauge needle is too large, making insertion to a desired depth into ocular tissue a challenge.

Thus, a need exists for improved methods and devices for delivering substances to ocular tissue.

US 2012/0083727 A1 describes a drug delivery blade for delivering a drug depot to a treatment site in a patient. An intravitreal injection device for administering a pharmacological agent formulation to an intreavitreal department of an eye is disclosed in US 7,678,078 B1. US 2009/0105749 A1 relates to an implant insertion tool for use with a lacrimal implant. US 2003/0009113 A1 relates to micro-needles and devices for sampling of abiological fluid and/or delivery of a drug or other formulation across the skin. US 2009/0030381 A1 describes a hypodermic needle for delivering a bolus of a drug to the posterior chamber of the eye. US 2011/202012 describes a smart injection syringe system providing real-time user feedback of the correct needle position.

### Summary

Devices and methods described herein relate generally to intraocular treatment and more particularly to the use of microneedles for treatment of ocular tissue.

The present invention relates to an apparatus set out in the appended set of claims.

Also described is a method which is not part of the invention, but present for illustration purposes, for delivering a substance to a target tissue of an eye includes inserting a microneedle into an eye such that a distal edge defined by a beveled surface of the microneedle does not extend through the choroid of the eye. The beveled surface of the microneedle defines a tip angle of less than about 20 degrees. The beveled surface has a height such that an opening defined by the beveled surface is within at least one of a suprachoroidal space or a lower portion of the sclera. A substance is conveyed from a cartridge coupled to a proximal end portion of the microneedle into the suprachoroidal space via the opening defined by the beveled surface

### Brief Description of the Drawings

The present invention is set out in the appended set of claims. The following description of the drawings include embodiments according to the invention, but also embodiments which do not fall under the scope of independent claim 1 and, thus, are not part of the invention and are present for illustration purposes only. In particular, the described methods of treatment are not part of the invention and are present for illustration purposes only.
FIG. 1 is a cross-sectional view of an illustration of the human eye.
FIG. 2 is a cross-sectional view of a portion of the human eye of FIG. 1 taken along the line 2-2.
FIGS. 3 and 4 are cross-sectional views of a portion of the human eye of FIG. 1 taken along the line 3-3, illustrating the suprachoroidal space without and with, respectively, the presence of a fluid.
FIG. 5 is a block diagram of a delivery device according to an embodiment.
FIG. 6 is a front view illustration of a delivery device according to an embodiment.
FIG. 7 is a perspective view illustration of a portion of a microneedle according to an embodiment.
FIG. 8 is a side view illustration of a portion of a microneedle according to the invention.
FIG. 9 is a top view illustration of the portion of the microneedle of FIG. 8.
FIG. 10 is a cross-sectional view of the portion of the microneedle taken along the line 10-10 in FIG. 9.
FIG. 11 is a side view illustration of a portion of a microneedle according to another embodiment.
FIG. 12 is a top view illustration of the portion of the microneedle of FIG. 11.
FIG. 13 is side view illustration of a portion of a microneedle according to another embodiment.
FIGS. 14-16 are side view illustrations of various lumen and bevel configurations included in a microneedle according to various embodiments.
FIG. 17 is a schematic illustration of an infusion device in use, according to an embodiment.
FIG. 18 is an enlarged view of a portion of the human eye and a portion of the infusion device identified in FIG. 17 as region Z.
FIG. 19 is a schematic illustration of a microneedle in use, according to an embodiment.
FIG. 20 is a schematic illustration of a portion of the human eye illustrating certain dimensions.
FIG. 21 is a front view illustration of a delivery device according to an embodiment.
FIG. 22 is a schematic illustration of a delivery system according to an embodiment.
FIG. 23 is a schematic illustration of a kit including a delivery device and at least one microneedle according to an embodiment.
FIG. 24 is a schematic illustration of a microneedle array according to an embodiment.
FIG. 25 is a cross-sectional illustration of an eye with a microneedle, according to an embodiment, and a standard 30 gauge needle inserted into the vitreous.
FIG. 26 is a flow chart illustrating a method of delivering a drug to a target ocular tissue, according to an embodiment.
FIG. 27 is an image of a microneedle (shown in the middle), a 27 gauge standard needle (shown at the top), and a 30 gauge standard needle (shown at the bottom), according to an embodiment.
FIG. 28 is an image of a microneedle (shown at the bottom) and 34 gauge standard needle (shown at the top), according to an embodiment.
FIG. 29 is a set of images of human cadaver eyes prior to injection (top panels) and following injection (bottom panels) of triamcinolone with a 30 gauge standard needle (left panels) and a microneedle of the invention (right panels).
FIG. 30 is a graph showing the growth of WERI human retinoblastoma cells versus days in cell culture, following aspiration and passage of cells with a 26 gauge, 30 gauge or microneedle.
FIG. 31 is a set of images depicting stained WERI human retinoblastoma cells following aspiration and passage using 26 gauge, 30 gauge standard needle or a microneedle of the invention.
FIGS. 32 and 33 are schematic illustrations of a microneedle with and without a baffle in the chamber of the microneedle, respectively, according to an embodiment.
FIG. 34 is a graph showing cell density of WERI human retinoblastoma cells versus time, following aspiration and passage of cells with a standard needle with or without a baffle, or a microneedle with or without a baffle.
FIG. 35 are images of the rabbit eye. A. Vitreous seeds of retinoblastoma (*) in the rabbit model. B. The microneedle (arrow) is inserted at the pars plana. C. The microneedle (arrow) is inserted to its hub into the vitreous. D. After 3 weekly injections of 20 µg topotecan, the vitreous seeds disappeared.
FIG. 36 is a schematic depiction of a 30 gauge needle or a microneedle with baffle inserted into the pars plana of an enucleated eye (left panel); and a set of images of enucleated eyes stained with hematoxylin-eosin following aspiration of retinoblastoma with a 30 gauge needle (top panes) or microneedle (bottom panes). Images were taken at 25X (middle panes) and 100X (right panes) magnification. Needle tracts are indicated with black arrows in the 100X images.

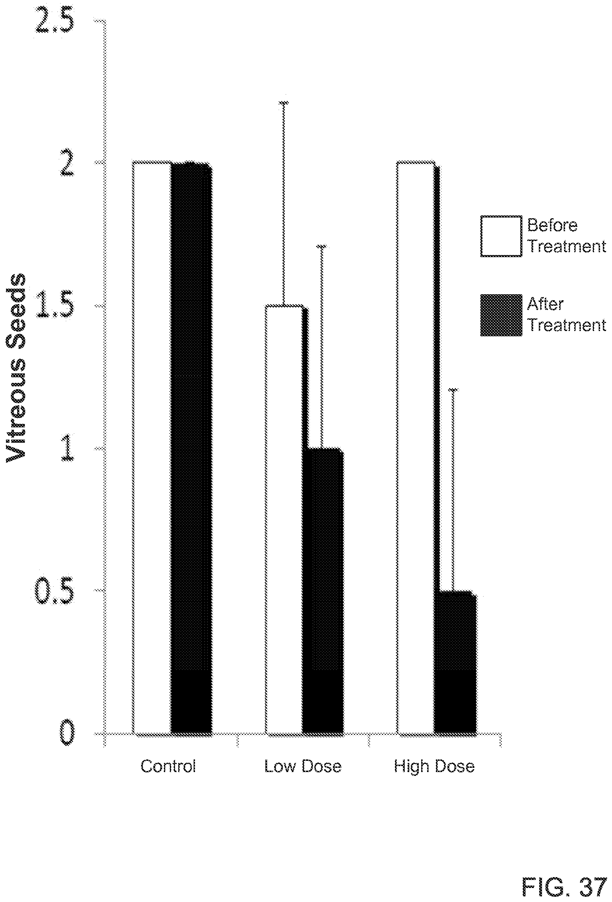

FIG. 37 is a bar graph showing vitreous seed score after control (PBS), low-dose topotecan (5µL/50µg topotecan), or high-dose topotecan (10µL/50µg topotecan) treatment in a rabbit retinoblastoma model. Dosages were administered once per week, for three weeks. Vitreous seed score (i.e., no score, (+), (++), or (+++) corresponding to a score of 0, 1, 2, or 3, respectively) was determined before and after topotecan treatment in each animal, and the average vitreous seed score in each group was calculated at both time points. Vitreous seeds were graded as 0 (no seed), 1 plus (+) with seeds filling less than 1/3 of the vitreous; 2 plus (++) with seeds filling 1/3-2/3 of the vitreous; and 3 plus (+++) with seeds filling the entire vitreous.
FIG. 38 is a bar graph depicting tumor area after administration of control (PBS), low-dose topotecan (5µL/50µg topotecan), or high-dose topotecan (10µL/50µg topotecan) in a rabbit retinoblastoma model. Dosages were administered once per week for three weeks. Tumor area was measured in mm².

### Detailed Description

The present invention is set out in the appended set of claims. The following description describes embodiments and aspects according to the invention, but also embodiments and aspects which do not fall under the scope of independent claim 1 and, thus, are not part of the invention and are present for illustration purposes only. In particular, the described methods of treatment are not part of the invention and are present for illustration purposes only.

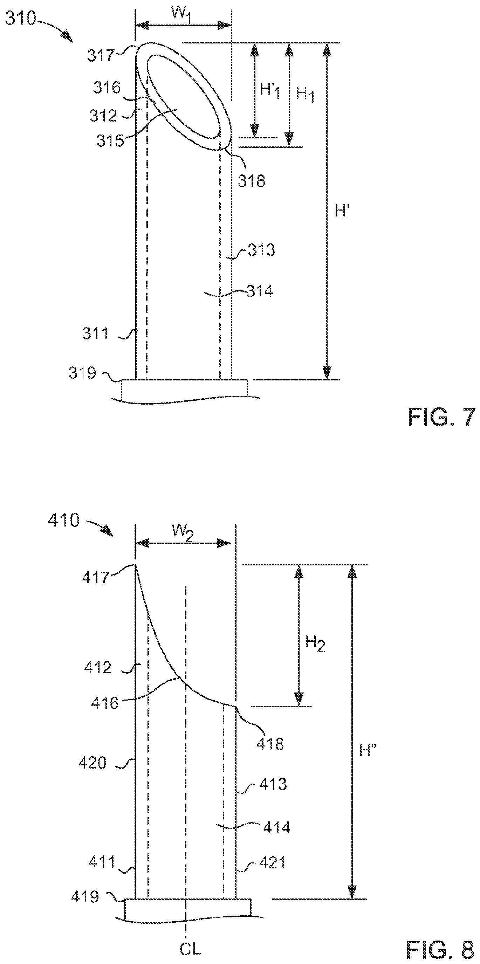

For example, embodiments of Figs.7, 14 and 16 do not form part of the invention as they fail to disclose the features of independent claim 1.

In some embodiments, a microneedle for ocular drug delivery and/or ocular tumor removal includes a beveled surface. The beveled surface of the microneedle defines a tip angle of less than about 20 degrees and a ratio of a bevel height to a bevel width of less than about 2.5. The beveled microneedle, in one embodiment, allows for accurate and reproducible drug delivery to the suprachoroidal space (SCS) of the eye. In other embodiments, the beveled microneedle is used in a pediatric ocular drug delivery methods to deliver one or more drugs to the vitreous of a pediatric eye. Advantageously, the beveled microneedle, when used in the pediatric eye, minimizes the length of the needle tract, thereby minimizing the opportunity for a tumor cell(s) to re-seed in the needle tract.

In some embodiments, a microneedle has a proximal end portion and a distal end portion and defines a lumen. The proximal end portion is configured to be coupled to a cartridge to place the lumen in fluid communication with the cartridge. The proximal end portion includes a base surface that is configured to be placed in contact with a surface of a target tissue. The distal end portion of the microneedle includes a beveled surface. The beveled surface defines a first bevel angle and a second bevel angle different from the first bevel angle. In some embodiments, the first bevel angle is less than the second bevel angle. In some embodiments, the first bevel angle is less than about 20 degrees and the second bevel angle is less than about 30 degrees.

In some embodiments, a microneedle has a proximal end portion and a distal end portion and defines a lumen. The proximal end portion is configured to be coupled to a cartridge to place the lumen in fluid communication with the cartridge. The proximal end portion includes a base surface that is configured to be placed in contact with a surface of a target tissue. The distal end portion of the microneedle includes a beveled surface. The beveled surface defines a tip angle of less than about 20 degrees and a ratio of a bevel height to a bevel width of less than about 2.5.

In some embodiments, a hollow microneedle and/or microneedle assembly for delivery of a drug to an eye is provided. In some embodiments, the hollow microneedle includes a distal end portion and a shaft extending from a cartridge housing. The needle can be disposed within a needle cap prior to use. A distal end of the microneedle includes a bevel that corresponds, at least partially, to a target location within the eye. The cartridge housing can receive a cartridge containing a therapeutic agent. In some embodiments, the microneedle is configured to allow the entire shaft or substantially the entire shaft of the microneedle to be inserted into the eye such that the distal end portion of the microneedle is disposed within the target location (e.g., the suprachoroidal space) of the eye.

In some embodiments, a microneedle for delivery of a drug to a pediatric eye is provided. The microneedle may be a hollow microneedle, or a solid microneedle. In some embodiments, the microneedle includes a bevel and a shaft extending from a base, and defines a lumen. The microneedle is configured to facilitate the insertion of the entire shaft or substantially the entire shaft of the microneedle into the pediatric eye such that a drug formulation can be deposited, injected and/or infused in the vitreous of the pediatric eye without damaging the lens or retina.

In some embodiments, a method, not being part of the invention, for delivering a substance to a target tissue of an eye includes inserting a microneedle into an eye such that a distal edge defined by a beveled surface of the microneedle does not extend through the choroid of the eye. The beveled surface of the microneedle defines a tip angle of less than about 20 degrees. The beveled surface has a height such that an opening defined by the beveled surface is within at least one of a suprachoroidal space or a lower portion of the sclera. A substance is conveyed from a cartridge coupled to a proximal end portion of the microneedle into the suprachoroidal space via the opening defined by the beveled surface.

In some embodiments, a method, not being part of the invention, for delivering a drug to the suprachoroidal space of an eye includes inserting a distal end of a hollow microneedle into the sclera, wherein the entire shaft or substantially the entire shaft of the microneedle is inserted into the eye at an angle of approximately 90 degrees. Upon insertion, a drug is conveyed, injected and/or infused through the microneedle, through the sclera, and into the suprachoroidal space without damaging the lens, retina, or other ocular tissue.

In some embodiments, a method, not being part of the invention, for delivering a drug to the suprachoroidal space of an eye includes inserting a distal end of a hollow microneedle into the sclera, wherein the entire shaft or substantially the entire shaft of the microneedle is inserted into the eye at an angle of approximately 90 degrees. Upon insertion, a drug is infused through the microneedle, through the sclera, and into the suprachoroidal space without damaging the lens, retina, or other ocular tissue. In a further embodiment, the microneedle, when inserted into the eye for suprachoroidal drug delivery, does not puncture the choroid.

In some embodiments, a method, not being part of the invention, for delivering a drug to the vitreous of a pediatric eye includes inserting the distal end of any of the microneedles described herein through the ciliary body of the pediatric eye, wherein the entire shaft or substantially the entire shaft of the microneedle is inserted into the eye at an angle of approximately 90 degrees. A drug is then injected and/or infused through the lumen of the microneedle into the vitreous.

In some embodiments, a method, not being part of the invention, for delivering a drug to the suprachoroidal space of an eye is provided. In some embodiments, the method comprises inserting a distal end of a hollow microneedle into the sclera or suprachoroidal space, wherein the entire shaft or substantially the entire shaft of the microneedle is inserted into the eye at an angle of approximately 90 degrees. Upon insertion, a drug is injected and/or infused through the microneedle into the suprachoroidal space without damaging the lens, retina, and/or other ocular tissue.

In some embodiments, a method, not being part of the invention, for treating retinoblastoma includes inserting a distal end of a microneedle defining a lumen through the ciliary body of the human eye, wherein the entire shaft or substantially the entire shaft of the microneedle is inserted into the eye, and infusing a topotecan formulation through the microneedle and into the vitreous of the eye.

In some embodiments, the methods provided herein, but not being part of the invention, are used to deliver a growth factor to the vitreous of the eye, for example, a pediatric eye. In some embodiments, the growth factor is vascular endothelial growth factor (VEGF). In other embodiments, the drug delivered with the methods provided herein is a VEGF inhibitor. In some embodiments, the VEGF inhibitor is an antibody, e.g., bevacizumab. In still other embodiments, both VEGF and a VEGF inhibitor are delivered to the vitreous of a pediatric eye via any of the methods and microneedles described herein. In a further embodiment, the length of the microneedle is such that the entire shaft or substantially the entire shaft of the microneedle is inserted into the eye without damaging the lens or retina, or other ocular substructures.

In some embodiments, a method, not being part of the invention, for decreasing the tumor size of an intraocular tumor in a patient includes infusing a topotecan formulation into an eye having one or more intraocular tumors, such as, for example, a retinoblastoma tumor. The topotecan formulation is infused using at least one microneedle provided herein. In some instances, the patient in need thereof is a pediatric patient. In one embodiment, the drug (e.g., a chemotherapeutic agent such as topotecan) is infused into the eye in an hourly, daily, or weekly dosing regimen. In one embodiment, the drug is infused into the eye once weekly. In a further embodiment, the drug is infused into the eye once weekly for two, three, four, five, or six weeks. In another embodiment, the drug is infused into the eye once weekly for three weeks. In another embodiment, the drug is infused into the eye at a dosage of about 10 µg, e.g., in a 50 µL volume. In one embodiment, the tumor area is reduced to a greater extent in comparison to the reduction in tumor area that occurs when topotecan is infused using a 30 gauge needle.

In another aspect, a method, not being part of the invention, for extraction of a biological tissue, fluid, or molecular sample from the vitreous, sclera or corneal stroma of a patient's eye (e.g., a pediatric eye) using any of the microneedles described herein is provided. In a further embodiment, the biological sample is a cancer cell or cells, for example, a retinoblastoma cell or cells. In a further embodiment, the extraction of the biological sample does not result in the accumulation of the biological tissue, fluid, or molecule in the needle tract. In another embodiment, the extraction of the biological sample results in less accumulation of the biological tissue, fluid, or molecule in comparison to the accumulation of the biological tissue, fluid, or molecule in the needle tract that occurs when the biological sample is extracted using a 27 gauge or 30 gauge needle.

In another embodiment, provided herein, but not being part of the invention, are methods for decreasing the number of vitreous seeds of an intraocular tumor in a patient, e.g., a retinoblastoma tumor. In a further embodiment, the number of vitreous tumor seeds in the patient is reduced to a greater extent compared to the reduction in the number of vitreous tumor seeds that are present after infusion of topotecan using a 30 gauge needle. In even a further embodiment, the patient is a pediatric patient.

In some embodiments, a microneedle, such as those described herein, is configured to be at least partially inserted into the sclera to deliver a therapeutic agent to a target region of the eye (e.g., the suprachoroidal space). The microneedles described herein include a bevel, which in comparison with bevels of standard needles, allows for ease of penetration into the sclera and/or suprachoroidal space with minimal collateral damage. The microneedles define a narrow lumen (e.g., greater than or equal to 30 gauge, 32 gauge, 34 gauge, 36 gauge, etc.) that can allow for suprachoroidal drug delivery while minimizing the diameter of the needle tract caused by the insertion of the microneedle. The lumen, the configuration of multiple bevel angles and the bevel aspect ratio of the microneedles described herein are distinct from the bevel included in standard 27 gauge and 30 gauge needles. Moreover, the entire shaft (or substantially the entire shaft) of the microneedle can be inserted into the eye using the methods provided herein, allowing for less uncertainty and less variability in drug delivery. In one embodiment, the microneedle has a length of about 4 mm compared to, for example, a length of about 10 mm for a 27 gauge and/or 30 gauge needle. In such embodiments, the size of the microneedle can be more appropriate for insertion into the pediatric eye than the size of a 27 gauge and/or 30 gauge needle.

The microneedle defines a lumen and includes a distal end portion and a shaft extending from a cartridge housing. The microneedle can be disposed within a needle cap prior to use. The cartridge housing receives a cartridge containing a therapeutic agent. The arrangement of the cartridge housing and the microneedle can allow the entire shaft or substantially the entire shaft of the microneedle to be inserted into the eye. In some embodiments, the arrangement of the distal end portion of the microneedle can correspond to a target tissue of the eye. For example, in some instances, the entire shaft or substantially the entire shaft can be inserted into the eye such that the distal end portion of the microneedle is disposed within the sclera or suprachoroidal space of the eye without damaging other ocular tissues. In some instances, the lumen of the microneedle defines a flow path through which a drug formulation is conveyed and/or infused when the microneedle is disposed within the sclera or the suprachoroidal space. For example, in some instances, the distal end portion of the microneedle can be inserted into a target region in or near the sclera. The relatively expandable suprachoroidal space can have a smaller resistance to flow than the relatively incompressible surrounding tissue. Thus, as a drug formulation is conveyed and/or infused into the target region, the drug formulation can naturally flow into and expand the suprachoroidal space. As a result, the drug formulation can be conveyed to an anterior region of the eye (e.g., the choroid, retina, etc.) without surgically accessing (e.g., cutting) the target region.

In some embodiments, the microneedle is hollow and defines a narrow lumen. The narrow lumen (e.g., greater than or equal to 32 gauge) of the microneedle can allow for drug delivery to the posterior segment of the eye, for example, to the vitreous, as well as aspiration of cellular material from the eye. The microneedle is much smaller than standard 27 gauge and 30 gauge needles which are now commonly used for intraocular injection. Moreover, the entire shaft (or substantially the entire shaft) of the microneedle is inserted into the eye in the methods provided herein, allowing for less uncertainty and less variability in drug delivery. Such embodiments can be particularly useful in pediatric patients, as the eyes have a very short ciliary body (e.g., as described herein with reference to Table 1 below). The embodiments described herein can achieve greater reproducibility in drug delivery and reduce the risk of damage to the lens and/or retina when compared to conventional needles.

In some embodiments, a method, not being part of the invention, includes inserting a hollow microneedle into an eye of a patient at an insertion site; the microneedle has a tip end that defines an opening. Upon insertion, a triamcinolone composition (e.g., triamcinolone particles) is delivered over a period of time through the inserted microneedle and into the suprachoroidal space of the eye. During the time period the delivered drug formulation flows within the suprachoroidal space away from the insertion site. In some embodiments, the composition comprises triamcinolone or triamcinolone acetonide nanoparticles or microparticles. In some embodiments, the microparticles in the composition have a D₅₀ of 2 µm or less and/or a D₉₀ of less than 10 µm.

In yet another aspect, a method, not being part of the invention, for delivering a drug into the vitreous of an eye is provided. In some embodiments, the method includes inserting a distal end of a microneedle into a vitreous of a human eye, e.g., a pediatric eye, wherein the entire shaft, or substantially the entire shaft of the microneedle is inserted into the eye. The distal end of the microneedle includes a bevel that corresponds, at least partially, to a target location within the eye. The microneedle defines a lumen configured to provide a flow path for a drug when the microneedle is disposed within the vitreous. The method further includes removing the microneedle after a desired amount of drug is delivered.

In some embodiments, a method for drug delivery to the pediatric eye is provided, the method not being part of the invention. The method includes inserting a microneedle into the pediatric eye, so that the entire shaft or substantially the entire shaft of the microneedle is inserted into the pediatric eye during drug delivery. In this regard, the user (e.g., a doctor, nurse, etc.) of the device is not required to determine the depth of insertion of the device, which allows for greater reproducibility in drug delivery methods and/or cellular aspiration methods. Moreover, the bevel structure (e.g., bevel length and bevel angle) of the devices presented herein eliminate or substantially reduce damage to the lens and retina when inserting the device through the ciliary body and into the vitreous of the eye.

As used in this specification, the singular forms "a," "an" and "the" include plural referents unless the context clearly dictates otherwise. Thus, for example, the term "a member" is intended to mean a single member or a combination of members, "a material" is intended to mean one or more materials, or a combination thereof.

As used herein, the words "proximal" and "distal" refer to the direction closer to and away from, respectively, an operator (e.g., surgeon, physician, nurse, technician, etc.) who would insert the medical device into the patient, with the tip-end (i.e., distal end) of the device inserted inside a patient's body first. Thus, for example, the end of a microneedle described herein first inserted inside the patient's body would be the distal end, while the opposite end of the microneedle (e.g., the end of the medical device being manipulated by the operator) would be the proximal end of the microneedle.

As used herein, a "set" can refer to multiple features or a singular feature with multiple parts. For example, when referring to set of walls, the set of walls can be considered as one wall with distinct portions, or the set of walls can be considered as multiple walls.

As used herein, the terms "about" and "approximately" generally mean plus or minus 10% of the value stated. For example, about 0.5 would include 0.45 and 0.55, about 10 would include 9 to 11, about 1000 would include 900 to 1100.

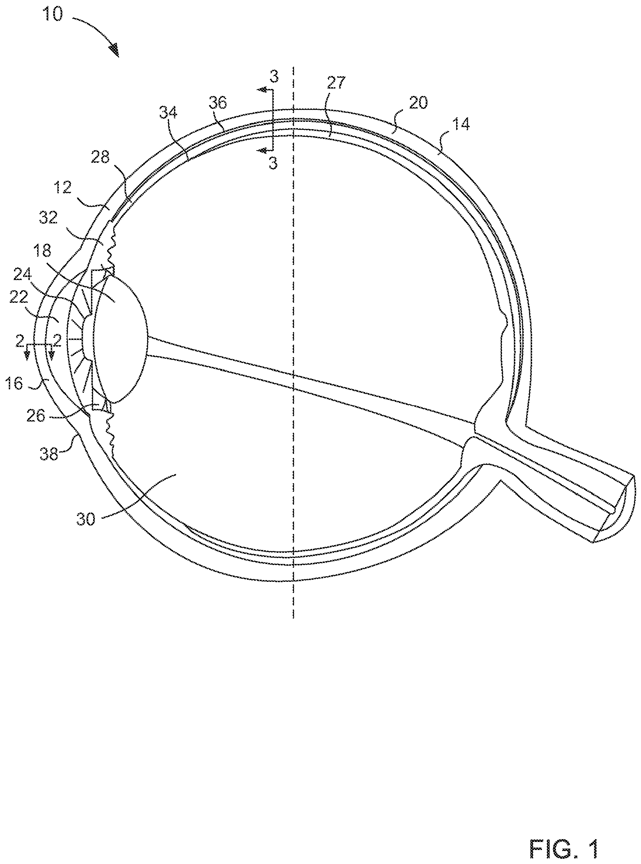

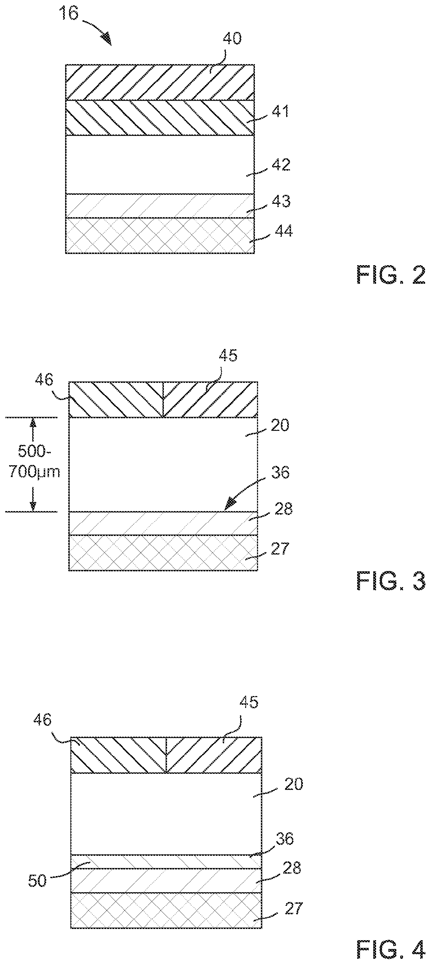

The embodiments and methods described herein can be used to treat, deliver substances to and/or aspirate substances from, various target tissues in the eye. For reference, FIGS. 1-4 are a various cross-sectional views of a human eye 10. While specific regions are identified, those skilled in the art will recognize that the proceeding identified regions do not solely constitute the eye 10, rather the identified regions are presented as a simplified example suitable for the discussion of the embodiments herein. The eye 10 includes both an anterior segment 12 (the portion of the eye in front of and including the lens) and a posterior segment 14 (the portion of the eye behind the lens). The anterior segment 12 is bounded by the cornea 16 and the lens 18, while the posterior segment 14 is bounded by the sclera 20 and the lens 18. The anterior segment 12 is further subdivided into the anterior chamber 22, between the iris 24 and the cornea 16, and the posterior chamber 26, between the lens 18 and the iris 24. The cornea 16 and the sclera 20 collectively form a limbus 38 at the point at which they meet. The exposed portion of the sclera 20 on the anterior segment 12 of the eye is protected by a clear membrane referred to as the conjunctiva 45 (see e.g., FIGS. 2 and 3). Underlying the sclera 20 is the choroid 28 and the retina 27, collectively referred to as retinachoroidal tissue. A vitreous humour 30 (also referred to as the "vitreous") is disposed between a ciliary body 32 (including a ciliary muscle and a ciliary process) and the retina 27. The anterior portion of the retina 27 forms an ora serrata 34. The loose connective tissue, or potential space, between the choroid 28 and the sclera 20 is referred to as the suprachoroid. FIG. 2 illustrates the cornea 16, which is composed of the epithelium 40, the Bowman's layer 41, the stroma 42, the Descemet's membrane 43, and the endothelium 44. FIG. 3 illustrates the sclera 20 with surrounding Tenon's Capsule 46 or conjunctiva 45, suprachoroidal space 36, choroid 28, and retina 27, substantially without fluid in the suprachoroidal space 36 (i.e., the in this configuration, the space is "potential" suprachoroidal space). As shown in FIG. 3, the sclera 20 has a thickness between about 500 µm and 700 µm. FIG. 4 illustrates the sclera 20 with the surrounding Tenon's Capsule 46 or the conjunctiva 45, suprachoroidal space 36, choroid 28, and retina 27, with fluid 50 in the suprachoroidal space 36.

As used herein, the term "suprachoroidal space," which is synonymous with suprachoroid, or suprachoroidia, describes the space (or volume) and/or potential space (or potential volume) in the region of the eye 10 disposed between the sclera 20 and choroid 28. This region primarily is composed of closely packed layers of long pigmented processes derived from each of the two adjacent tissues; however, a space can develop in this region as a result of fluid or other material buildup in the suprachoroidal space and the adjacent tissues. The suprachoroidal space can be expanded by fluid buildup because of some disease state in the eye or as a result of some trauma or surgical intervention. In some embodiments, the fluid buildup is intentionally created by the delivery, injection and/or infusion of a drug formulation into the suprachoroid to create and/or expand further the suprachoroidal space 36 (i.e., by disposing a drug formulation therein). This volume may serve as a pathway for uveoscleral outflow (i.e., a natural process of the eye moving fluid from one region of the eye to the other through) and may become a space in instances of choroidal detachment from the sclera.

The dashed line in FIG. 1 represents the equator of the eye 10. In some embodiments, the insertion site of any of the microneedles and/or methods described herein is between the equator and the limbus 38 (i.e., in the anterior portion 12 of the eye 10). For example, in some embodiments, the insertion site is between about two millimeters and 10 millimeters (mm) posterior to the limbus 38. In other embodiments, the insertion site of the microneedle is at about the equator of the eye 10. In still other embodiments, the insertion site is posterior the equator of the eye 10. In this manner, a drug formulation can be introduced (e.g., via the microneedle) into the suprachoroidal space 36 at the site of the insertion and can flow through the suprachoroidal space 36 away from the site of insertion during an infusion event (e.g., during injection).

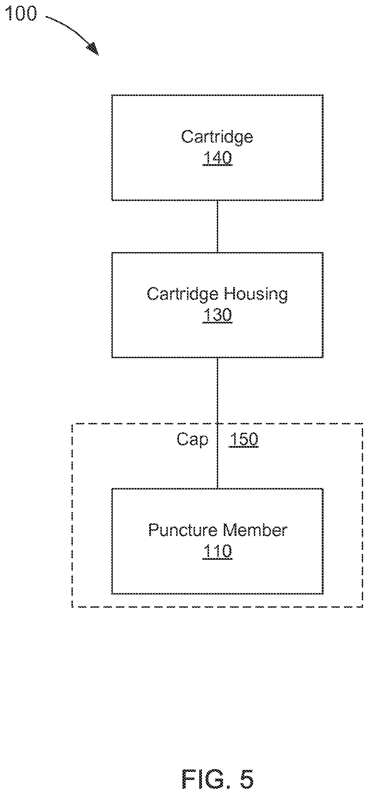

FIG. 5 is block diagram illustrating a delivery (e.g., infusion, injection) device 100 according to an embodiment. The delivery device 100 includes a delivery member 110, a cartridge housing 130, and a cartridge 140. The delivery member 110 is a microneedle as defined in the appended claims and is configured to puncture and/or pierce a target tissue of a patient, and deliver a substance to and/or away from the target tissue. For example, the delivery member 110 can be configured to puncture ocular tissue, deliver a substance thereto and/or remove a substance therefrom. In some embodiments, the shape and/or size of the delivery member 110 can correspond with at least a portion of a target tissue. For example, in some embodiments, the length of the delivery member 110 can correspond to a portion of ocular tissue such that when the delivery member 110 is inserted into the ocular tissue, at least a portion of the delivery member 110 is disposed within the sclera or suprachoroidal space of the eye. In other embodiments, a bevel geometry (e.g., bevel angle, bevel height, bevel aspect ratio or the like) of the delivery member 110 is configured to easily pierce the target tissue and maintain an opening (not shown) within a desired region. The delivery member 110 is physically and/or fluidically coupled to the cartridge housing 130. More specifically, the cartridge housing 130 can include a set of annular walls that define an inner volume that is in fluid communication with the delivery member 110.

The cartridge housing 130 can be coupled to and/or receive the cartridge 140 such that at least a portion of the cartridge 140 is disposed within the cartridge housing 130. The cartridge 140 can be any suitable device configured to house or contain a drug formulation (e.g., a prophylactic agent, a therapeutic agent, a diagnostic agent or any of the formulations described herein). More specifically, the cartridge 140 can include a set of walls that define an inner volume within which a drug formulation is disposed. The cartridge 140 can be moved between a first configuration and a second configuration to expel the drug formulation disposed within the inner volume. For example, in some embodiments, the cartridge 140 can be a prefilled syringe or the like.

In use, the delivery member 110 can be inserted into, for example, an ocular tissue such that at least a portion of the delivery member 110 is disposed within the sclera or suprachoroidal space of the eye. With the delivery member 110 disposed within the eye, the cartridge 140 can be moved within the inner volume of and/or relative to the cartridge housing 130 to place the inner volume of the cartridge 140 in fluid communication with the lumen defined by the delivery member 110. After the cartridge 140 is placed in fluid communication with the delivery member 110, the cartridge 140 can be moved from the first configuration to the second configuration to expel the drug formulation (contained within the inner volume) through the lumen of the delivery member 110. Thus, in this manner, the delivery device 100 can deliver a drug formulation to the suprachoroidal space of the eye and the drug formulation can flow within the suprachoroidal space to be delivered to, for example, the posterior region of the eye. In other embodiments, the delivery device 100 can deliver a drug formulation to any suitable location.

As shown in FIG. 5, in some embodiments, the delivery device 100 can include a cap 150 that is disposed about the delivery member 110 prior to the insertion of the delivery member 110 into the ocular tissue. In such embodiments, the cap 150 can be configured to maintain the sterility of the delivery member 110. Similarly stated, in some embodiments, the cap 150 can enclose the delivery member 110 such that delivery member 110 is sterile prior to insertion into the ocular tissue.

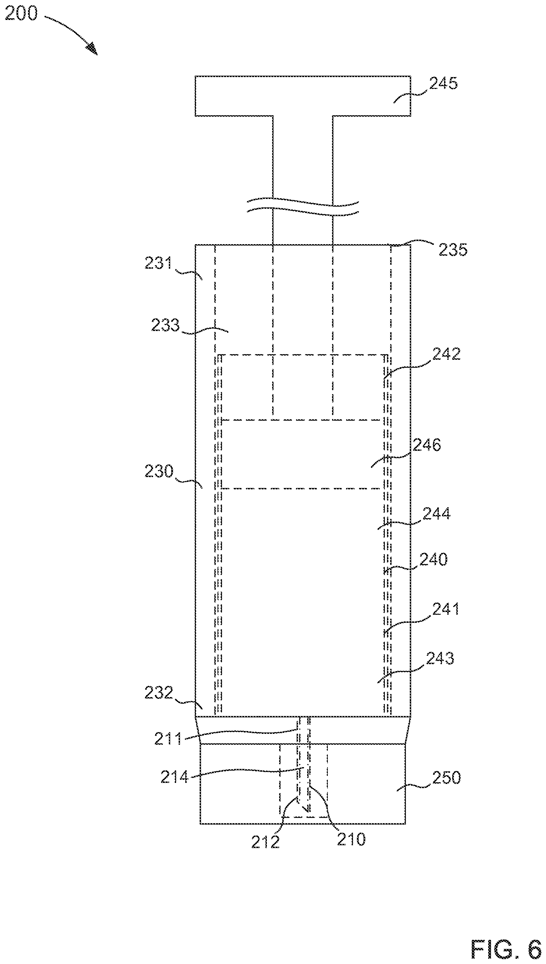

FIG. 6 is schematic illustration of medicament delivery device 200 according to an embodiment. The medicament delivery device 200 includes a microneedle 210, a cartridge housing 230, a cartridge 240, and a cap 250. The cap 250 is disposed adjacent to the cartridge housing 230 and is configured to house at least a portion of the microneedle 210. In this manner, the cap 250 can maintain the sterility of the microneedle 210 prior to use of the medicament delivery device 200. Therefore, a user (e.g., a doctor, technician, nurse, physician, ophthalmologist, etc.) can remove the cap 250 to expose at least a portion of the microneedle 210, as described in further detail herein.

The microneedle 210 is a microneedle as defined in the appended claims and is configured to puncture, a target tissue of a patient. For example, the microneedle 210 can be configured to puncture ocular tissue. In some embodiments, the microneedle 210 can be a 30 gauge microneedle, a 32 gauge microneedle or a 34 gauge microneedle. In some embodiments, the shape and/or size of the microneedle 210 can correspond with at least a portion of a target tissue. For example, in some embodiments, the length of the microneedle 210 can correspond with a portion of ocular tissue such that when the microneedle 210 is inserted into the ocular tissue, a portion of the microneedle 210 is disposed within the sclera or suprachoroidal space of the eye. In other embodiments, a bevel geometry (e.g., bevel angle, bevel height, bevel aspect ratio or the like) of the microneedle 210 is shaped such that the distal tip of the microneedle 210 can easily pierce the target tissue and the opening (not shown) of the microneedle 210 can be maintained within a desired region during an injection event.

The microneedle 210 defines a lumen 214 that extends through a proximal end portion 211 and a distal end portion 212 of the microneedle 210. The distal end portion 212 of the microneedle 210 includes a bevel configured to puncture, pierce and/or separate a target tissue of a patient (e.g., ocular tissue), as described in further detail herein. The proximal end portion 211 of the microneedle 210 is physically and fluidically coupled to the cartridge housing 230. In some embodiments, the microneedle 210 and the cartridge housing 230 can be monolithically or unitarily formed. In other embodiments, the microneedle 210 can be physically coupled to the cartridge housing 230 via a press fit, a friction fit, a threaded coupling, an adhesive, and/or any other suitable coupling means. In this manner, the lumen 214 defined by the microneedle 210 can be placed in fluid communication with an inner volume 233 defined by the cartridge housing 230, as described in further detail herein.

The cartridge housing 230 has a proximal end portion 231 and a distal end portion 232. The distal end portion 232 is physically and fluidically coupled to the microneedle 210, as described above. The proximal end portion 231 can be configured to receive and/or be coupled to the cartridge 240. More specifically, at least a portion of the cartridge 240 can be inserted through an opening 235 defined by the proximal end portion 231 of the cartridge housing 230 such that at least a portion of the cartridge 240 is disposed within the inner volume 233 of the cartridge housing 230.

The cartridge 240 includes a cartridge body 241 and a plunger 245. The cartridge body 241 has a proximal end portion 242 and a distal end portion 243 and defines an inner volume 244. The proximal end portion 242 of the cartridge body 241 is substantially open such that the cartridge body 241 can movably receive at least a portion of the plunger 245. More specifically, at least a portion of the plunger 245 is disposed within the inner volume 244 and can be moved between a first position (e.g., a proximal position) and a second position (e.g., a distal position). The plunger 245 includes a seal member 246 that forms a friction fit with one or more surfaces of the cartridge body 241 that define the inner volume 244. In this manner, the seal member 246 and the cartridge body 241 can form a substantially fluid-tight seal that substantially isolates a portion of the inner volume 244 that is distal to the seal member 246 from a portion of the inner volume 244 that is proximal to the seal member 246, as described in further detail herein.

In some embodiments, the distal end portion 243 of the cartridge body 241 can be at least temporarily closed (e.g., at least temporarily fluidically sealed). In this manner, the inner volume 244 (e.g., the portion of the inner volume 244 between the seal member 246 and the distal end portion 243) of the cartridge body 241 is fluidically isolated from a volume outside of the cartridge body 241. The inner volume 244 of the cartridge body 241 can further house or contain a drug formulation of the compositions described herein (e.g., a prophylactic agent, a therapeutic agent, or a diagnostic agent). More specifically, the drug formulation is disposed within the inner volume 244 of the cartridge body 241 in a distal position relative to the seal member 246. Thus, the drug formulation contained within the inner volume 244 is substantially fluidically isolated from a volume outside of the container body 241. In some embodiments, the inner volume 244 can contain a drug formulation with a volume of about 0.5 mL or less, for example about 0.1 mL to about 0.5 mL. In other embodiments, the inner volume 244 can contain a drug formulation with a volume of about 0.1 mL. In still other embodiments, the inner volume 244 can contain a drug formulation with a volume greater the about 0.5 mL.

In some embodiments, the distal end portion 243 of the cartridge body 241 can be moved between a first configuration (e.g., a closed or sealed configuration) and a second configuration (e.g., an open configuration). Expanding further, the distal end portion 243 of the container body 241 can include a surface that can be deformed (e.g., punctured, broken, opened, or otherwise reconfigured) to expel the drug formulation contained with the inner volume 244 of the cartridge body 241. For example, in some embodiments, the cartridge 240 can be inserted into the cartridge housing 230 such that the deformable surface of the distal end portion 243 of the cartridge body 241 is placed in contact with the proximal end portion 211 of the microneedle 210. In such embodiments, the proximal end portion 211 of the microneedle 210 can extend from a surface of the cartridge housing 230 that defines the inner volume 233 such that when the cartridge 240 is disposed within the inner volume 233, the proximal end portion of the microneedle 210 pierces, breaks, or otherwise reconfigures the deformable portion of the cartridge body 241. In this manner, the lumen 214 defined by the microneedle 210 can be placed in fluid communication with the inner volume 244 defined by the cartridge body 241. Therefore, when the plunger 245 is moved from its first position to its second position relative to the cartridge body 241, the drug formulation contained within the inner volume 244 of the cartridge body 241 can be expelled through the lumen 214 defined by the microneedle 210.

In other embodiments, however, the distal end portion 243 of the cartridge body 241 can be fluidically coupled to the microneedle 210. In this manner, the inner volume 244 (e.g., the portion of the inner volume 244 between the seal member 246 and the distal end portion 243) of the cartridge body 241 is fluidically coupled to a volume outside of the cartridge body 241 via the microneedle 210. For example, in some embodiments the distal end portion 243 can be devoid of a deformable portion or seal (e.g., a crimp seal), and in use the proximal end portion of the microneedle 210 need not pierce, break, or otherwise a surface prior to use.

In use, a user (e.g., a doctor, technician, nurse, physician, ophthalmologist, etc.) can remove the cap 250 to expose at least a portion of the microneedle 210 and can manipulate the infusion device 200 to insert the microneedle 210 into, for example, an ocular tissue. As described above, the length and/or the shape of the distal end portion 212 of the microneedle 210 (including, for example, a beveled surface) at least partially corresponds with the target tissue (e.g., the eye) such that the distal end portion 212 of the microneedle 210 is disposed within a lower portion of the sclera and/or the suprachoroidal space of the eye after being inserted. More specifically, the distal end portion 212 of the microneedle 210 is configured to pierce the sclera of the eye and be disposed within the sclera and/or suprachoroidal space without substantially piercing the choroid of the eye.

With the microneedle 210 disposed within the eye, the cartridge 240 can be moved within the inner volume 233 of the cartridge housing 230 to place the inner volume 244 of the cartridge body 241 in fluid communication with the lumen 214 defined by the microneedle 210. For example, in some embodiments, the proximal end portion 211 of the microneedle 210 can pierce or otherwise reconfigure the proximal end portion 211 to move the deformable surface from a sealed configuration to an unsealed or open configuration. Thus, the inner volume 244 of the cartridge body 241 is placed in fluid communication with the lumen 214 defined by the microneedle 210.

After the inner volume 244 of the cartridge body 241 is placed in fluid communication with the microneedle 210, the cartridge 240 can be moved from a first configuration (e.g., where the plunger 245 is disposed in its first position relative to the cartridge body 241) to a second configuration (e.g., where the plunger 245 is disposed in its second position relative to the cartridge body 241). With the seal member 246 forming a substantially fluid-tight or leak-proof seal (e.g., a substantially hermetic seal) with an inner surface of the cartridge body 241, the movement of the plunger 245 to its second position expels the drug formulation (contained within the inner volume 244) through the lumen 214 of the microneedle 210. Thus, the medicament delivery device 200 can deliver the drug formulation to the suprachoroidal space of the eye and the drug formulation can flow within the suprachoroidal space to be delivered to, for example, the posterior region of the eye.

Although the proximal end portion 211 of the microneedle 210 is described above as piercing or otherwise reconfiguring the deformable surface of the distal end portion 243 of the cartridge body 241, in other embodiments, the microneedle 210 need not physically contact the cartridge body 241. For example, in some embodiments, the distal end portion 243 of the cartridge body 241 can be in its closed configuration (e.g., undeformed configuration) when the plunger 245 is moved relative to the cartridge body 241. In such embodiments, the movement of the plunger 245 can increase the pressure within the inner volume 244 and the increase in pressure can move the deformable surface of the distal end portion 243 to its open configuration (e.g., deformed configuration). For example, the increase in pressure can open a valve or break (e.g., rupture) the deformable surface. Thus, the inner volume 244 can be placed in fluid communication with the lumen 214 of the microneedle 210 and the medicament delivery device 200 can deliver the drug formulation to the target tissue.

Although the microneedle 210 is described above as being inserted such that the distal end portion 212 is at least partially disposed in the suprachoroidal space, in other instances, the microneedle 210 can be inserted into various other regions of ocular tissue. For example, in some instances, a the microneedle 210 can be inserted through the ciliary body to dispose, at least partially, the distal end portion 212 of the microneedle 210 in the vitreous of, for example, a pediatric eye, as described in further detail herein.

Although not shown in FIG. 6, in some embodiments, the cartridge 240 can include a cap configured to enclose the deformable surface of the cartridge body 241. In such embodiments, the cap can maintain the sterility of the deformable surface and/or can prevent deformation (e.g., breaking, puncturing, etc.) of the deformable surface prior to use.

FIG. 7 is a schematic illustration of a microneedle 310 according to an embodiment. The microneedle 310 can be included in, for example, the medicament delivery device 200 described above with reference to FIG. 6, or any other delivery system described herein. The microneedle 310 can be configured to puncture, pierce and/or penetrate a portion of the eye to deliver a drug formulation to and/or remove a substance from a target location, such as, for example, the suprachoroidal space. The microneedle 310 includes a proximal end portion 311, a distal end portion 312, and a set of annular walls 313. The microneedle 310 has a shaft length H' that can be any suitable length. For example, in some embodiments, the shaft length H' can substantially correspond to at least a portion of the eye. For example, in some embodiments, the shaft length H' can correspond to and/or be within a range of the thickness of the sclera (see e.g., FIGS. 3 and 4). Thus, in some embodiments, the shaft length H' can be any suitable length such that when the microneedle is inserted into the eye, the distal end portion 312 of the microneedle 310 is disposed within the suprachoroidal space without puncturing and/or extending through the choroid. By way of example, the microneedle 310 shaft length H' can be about 1000 µm or less, about 900 µm or less, about 850 µm or less, about 800 µm or less, about 750 µm or less, about 700 µm or less, about 650 µm or less, or about 600 µm or less. In some embodiments, the microneedle 310 shaft length H' can be about 750 µm. In other embodiments, the microneedle 310 shaft length H' can be about 800 µm, or about 850 µm, or about 900 µm, or about 950 µm, or about 1 mm.

In other embodiments, the microneedle 310 can have a shaft length H' suitable for use in treatment of other portions of the eye, such as, the vitreous. For example, in some embodiments, the microneedle 310 can have a shaft length H' of about 1 mm to about 3 mm. In another embodiments, the microneedle 310 can have a shaft length H' from about 2.5 mm to about 5.5 mm. In yet another embodiment, the microneedle 310 can have a shaft length H' from about 3 mm to about 4 mm.

The walls 313 define a lumen 314 that extends through the proximal end portion 311 and the distal end portion 312. The proximal end portion 311 includes a base (or hub) 319 and/or can be coupled to (e.g., physically and/or fluidically) any suitable medical device. For example, in some embodiments, the proximal end portion 311 can be physically and fluidically coupled to the cartridge housing 230, as described above with reference to FIG. 6. In other embodiments, the proximal end portion 311 can be indirectly coupled to a medical device or cartridge housing via any suitable intervening structure such as, for example, a Luer-Lok^{®} (or other locking mechanism) or sterile flexible tubing. In this manner, the lumen 314 defined by the walls 313 of the microneedle 310 can be placed in fluid communication with a fluid source (e.g., the cartridge 240 described above or any other suitable source) to deliver a drug formulation to a target tissue.

The distal end portion 312 of the microneedle 310 defines an opening 315 configured to place the lumen 314 in fluid communication with a volume substantially outside the microneedle 310. The distal end portion 312 includes a bevel 316 (also referred to herein as "beveled surface") with a distal edge 317 and a proximal edge 318. Similarly stated, the distal end portion 312 includes a surface (i.e., the bevel 316) that is slanted, sloped, angled and/or inclined from an outer surface of the walls 313. Said another way, the beveled surface 316 intersects the outer surface of the walls 313 at one or more angles to define one or more sharp edges (e.g., the distal edge 317 and/or the proximal edge 318), as defined herein. This arrangement allows the distal end portion 312 of the microneedle to pierce, separate and/or deform the target tissue to facilitate penetration of the shaft microneedle 310 therethrough. Similarly stated, the sharp distal edge 317 is configured to pierce the target tissue, such as ocular tissue, to facilitate defining a passageway within the target tissue to the desired location (e.g., the suprachoroidal space and/or the vitreous, as described in further detail herein).

As shown in FIG. 7, the bevel 316 has a height H₁ defined and/or bounded by the distal edge 317 and the proximal edge 318. In other embodiments, the bevel 316 can have a height H'₁ that is defined between the distal edge 317 and an edge formed between an inner surface of the walls 313 that is circumferentially opposed to the distal edge 317 (e.g., an edge formed by the bevel 316 and an inner surface of the walls 313 that is adjacent to the proximal edge 318). The height H₁ and/or H'₁ can be any height that prevents and/or limits the likelihood of piercing the lens, retina, and/or choroid when the entire shaft length H' or substantially the entire shaft length H' of the microneedle 310 is inserted into the eye through the sclera. In other instances, the height H₁ and/or H'₁ of the bevel 316 can prevent and/or limit the likelihood of damaging the lens or other ocular tissue when the entire shaft length H' or substantially the entire shaft length H' is inserted into the eye through the ciliary body (e.g., such that the distal end portion 312 is disposed in the vitreous 30). In yet other embodiments, the height H₁ and/or H'₁ can be such that when the microneedle is disposed within the sclera, the opening 315 is at a desired location of the sclera and/or suprachoroidal space (see e.g., FIGS. 3 and 4). For example, if the bevel height H₁ and/or H'₁ is too large, a portion of the opening may be disposed outside of (e.g., above) the sclera and/or into the conjunctiva. Such positioning may result in the deposition of substances in an undesirable portion of the eye. Thus, in some embodiments, the bevel height H₁ and/or H'₁ is such that when the distal edge 317 is disposed within the suprachoroidal space and/or adjacent the choroid, the opening 315 does not extend beyond the innermost half of the sclera, third of the sclera, or quarter of the sclera. More particular, as described herein, in some embodiments, the microneedle can be inserted into the ocular tissue at an angle that is between about 80 degrees and about 100 degrees relative to a tangential surface of the insertion site of the eye. When inserted in such an orientation and with the distal edge 317 disposed within the suprachoroidal space and/or adjacent the choroid, the bevel height H₁ and/or H'₁ can be such that the opening 315 does not extend beyond the innermost half of the sclera, third of the sclera, or quarter of the sclera.

For example, in some embodiments, the height H₁ and/or H'₁ of the bevel 316 can be about 500 µm or less, about 450 µm or less, about 400 µm or less, about 350 µm or less, about 300 µm or less, about 250 µm or less, about 200 µm or less, about 150 µm or less, or about 100 µm or less. In other embodiments, the height H₁ and/or H'₁ of the bevel 316 is from about 50 µm to about 500 µm, from about 100 µm to about 500 µm, from about 150 µm to about 500 µm, from about 200 µm to about 500 µm, from about 250 µm to about 500 µm, from about 300 µm to about 500 µm, from about 50 µm to about 400 µm, from about 100 µm to about 400 µm, or from about 150 µm to about 400 µm. In some embodiments, the height H₁ and/or H'₁ of the bevel 316 can be about 485 µm. In still other embodiments, the height H₁ and/or H'₁ of the bevel 316 can be about 500 µm to about 1 mm. In another embodiment, the height H₁ and/or H'₁ of the bevel 316 is from about 600 µm to about 1 mm, from about 700 µm to about 1 mm, from about 800 µm to about 1 mm, from about 900 µm to about 1 mm, and/or any fraction there between.

While characterized above by the height H₁ and/or H'₁ of the bevel 316, in other embodiments, the microneedle 310 can be characterized by a bevel angle, or more particularly, a tip angle relative to an axis defined by the lumen 314. In some embodiments, the tip angle can be selected to facilitate insertion of the microneedle 310 within the desired type of tissue (e.g., ocular tissue). Similarly stated, in some embodiments, the tip angle can be selected to provide a sufficient "sharpness" such that the microneedle 310 can be inserted into the eye (e.g., the sclera) while minimizing the deformation of the eye resulting from the force of insertion. For example, in some embodiments, the bevel angle can be less than about 0.1 degree. In other embodiments, the bevel angle can be from approximately 0.1 degree to approximately 1 degree. In still other embodiments, the bevel angle can be from approximately 1 degree to approximately 5 degrees, including any fraction of a degree there between. In some embodiments, the microneedle 310 has a bevel angle from about 0.1 degree to about 30 degrees or from about 1 degree to about 25 degrees or from about 2 degrees to about 20 degrees or from about 10 degrees to about 20 degrees. In some embodiments, the tip angle can be less than about 18 degrees, 15 degrees or 12 degrees.

In yet other embodiments, the microneedle 310 can be characterized by the bevel height H₁ (or H'₁) to width W₁ ratio, (i.e., the bevel aspect ratio). In some embodiments, the bevel width W₁ corresponds with an outer diameter of the microneedle 310. In other embodiments, the bevel width W₁ can be associated with a diameter that is smaller than the outer diameter of the microneedle 310 (e.g., the microneedle 310 can have an outer diameter that is tapered from the proximal end portion 311 to the distal end portion 3212). In some embodiments, the bevel width W₁ is from about 50 µm to about 500 µm, from about 50 µm to about 400 µm, from about 100 µm to about 400 µm, from about 200 µm to about 400 µm, from about 200 µm to about 320 µm, or from about 100 µm to about 250 µm. As such, the microneedle 310 can have a bevel aspect ratio (height: width) is about 0.25:1, about 0.5:1, about 0.75:1, about 1:1, about 1.5:1, about 2:1, about 2.2:1, about 2.5:1 or about 3:1.

In this manner, the arrangement of the bevel 316 (e.g., the bevel can be such that the distal edge 317 is sufficiently sharp such as to pierce a target tissue and penetrate into sclera, the suprachoroidal space or the vitreous without (I) substantially causing the target tissue to elastically deform or (ii) damaging internal structures of the eye (e.g., the lens, retina, choroid, etc.). Similarly stated, the arrangement of the bevel 316 can be such that the distal edge 317 is sufficiently sharp such that prior to piercing the target tissue, the distal edge 317 does not substantially bend, compress, deform, or otherwise move the target tissue. Thus, the accuracy of insertion relative to the target tissue is increased and a potential for damaging surrounding tissue is minimized.

As described above, the microneedle 310 has a shaft length H'. The shaft length H' of the microneedle 310 is defined as the length from the distal edge 317 to the hub or base surface 319. In the drug delivery methods provided herein, the entire shaft length H' or substantially the entire shaft length H' of the microneedle 310 can be inserted into the eye. In this regard, the user need not determine the depth of insertion. The shaft length H' is such that when the microneedle 310 inserted through, for example, the ciliary body, the risk of piercing the lens or retina is greatly minimized or eliminated. Similarly, the shaft length H' is such that when the microneedle 310 is inserted through the sclera at about the ocular hemisphere (described above), the risk of piercing the choroid and/or retina is greatly minimized or eliminated. In this regard, the microneedles provided herein achieve greater reproducibility, and eliminate or substantially reduce uncertainty associated with drug delivery methods to the eye (e.g., to the suprachoroidal space and/or to the vitreous). The shaft length can be tailored depending on the age of the patient and the desired tissue for drug delivery or aspiration.

While the bevel 316 included in the microneedle 310 is shown in FIG. 7 as being substantially linear (e.g., the beveled surface is straight or planar), in other embodiments, a microneedle can include a bevel and/or beveled surface that is substantially curvilinear. FIGS. 8-10 are schematic illustrations of a microneedle 410 according to the invention. The microneedle 410 includes a proximal end portion 411, a distal end portion 412, and a set of annular walls 413. The microneedle 410 has a shaft length H" that can be any suitable length. For example, in some embodiments, the shaft length H" can substantially correspond to at least a portion of the eye. For example, in some embodiments, the shaft length H" can correspond to and/or be within a range of the thickness of the sclera (see e.g., FIGS. 3 and 4). In some instances, the shaft length H" can be substantially similar to or the same as the shaft length H' described above with reference to the microneedle 310.

The walls 413 of the microneedle 410 define a lumen 414 extending through the proximal end portion 411 and the distal end portion 412. Furthermore, the walls 413 can have and/or can define a width W₂ associated with an outer diameter and/or a beveled surface 416 of the microneedle 410, as described in further detail herein. The proximal end portion 411 can be substantially similar in form and function as the proximal end portion 311 included in the microneedle 310 described above with reference to FIG. 7. In this manner, the proximal end portion 411 can be physically and fluidically coupled to any suitable medical device, as described above. The distal end portion 412 of the microneedle 410 includes a bevel (or beveled surface) 416 having a distal edge 417 and a proximal edge 418. More particularly, as shown in FIGS. 8-10, the walls 413 of the microneedle 410 include a first portion 420 that intersects a first end of the bevel 416 to form and/or define the distal edge 417, and a second portion 421 that intersects a second end of the bevel 416, opposite the first end, to form and/or define the proximal edge 418. Similarly stated, the first portion 420 and the second portion 421 of the walls are circumferentially opposed and as such, the distal edge 417 of the bevel 416 and the proximal edge 418 of the bevel 416 are circumferentially opposed. The distal end portion 412 of the microneedle 410 also defines an opening 415. In this manner, the distal end portion 412 is configured to place the lumen 414 in fluid communication with a volume substantially outside the microneedle 410, such as for example, the suprachoroidal space.

As shown FIG. 8, the bevel 416 has a height H₂ defined and/or bounded by the distal edge 417 and the proximal edge 418. In some embodiments, the height H₂ of the bevel 416 is substantially similar in height to H₁ of the bevel 316. In other embodiments, the height H₂ of the bevel 416 can be any suitable height included in the range of lengths described above. For example, in some embodiments, the height H₂ can be such that when the microneedle 410 is disposed within the sclera, the opening 415 is at a desired location of the sclera and/or suprachoroidal space (see e.g., FIGS. 3 and 4). For example, if the bevel height H₂ is too large, a portion of the opening may be disposed outside of (e.g., above) the sclera and/or into the conjunctiva. Such positioning may result in the deposition of substances in an undesirable portion of the eye. Thus, in some embodiments, the bevel height H₂ is such that when the distal edge 417 is disposed within the suprachoroidal space and/or adjacent the choroid, the opening 415 does not extend beyond the innermost half of the sclera, third of the sclera, or quarter of the sclera. More particular, as described herein, in some embodiments, the microneedle 410 can be inserted into the ocular tissue at an angle that is between about 80 degrees and about 100 degrees relative to a tangential surface of the insertion site of the eye. When inserted in such an orientation and with the distal edge 417 disposed within the suprachoroidal space and/or adjacent the choroid, the bevel height H₂ can be such that the opening 415 does not extend beyond the innermost half of the sclera, third of the sclera, or quarter of the sclera. Thus, by maintaining the bevel height H₂ below a desired threshold, the length of the opening 415 can be such that the entire opening 415 is maintained within a desired portion of the sclera and/or suprachoroidal space.

In some embodiments, the height H₂ of the bevel can be about 500 µm to about 1 mm. In another embodiment, the height H₂ of the bevel 416 is about 500 µm or less, about 450 µm or less, about 400 µm or less, about 350 µm or less, about 300 µm or less, about 250 µm or less, about 200 µm or less, about 150 µm or less, or about 100 µm or less. In other embodiments, the height H₂ of the bevel 416 is from about 50 µm to about 500 µm, from about 100 µm to about 500 µm, from about 150 µm to about 500 µm, from about 200 µm to about 500 µm, from about 250 µm to about 500 µm, from about 300 µm to about 500 µm, from about 50 µm to about 400 µm, from about 100 µm to about 400 µm, or from about 150 µm to about 400 µm. The bevel height H₂ can be any bevel height that prevents piercing of the lens, retina, or choroid when the distal end 412 of the microneedle 410 is inserted through the sclera, and the entire shaft length H" or substantially the entire shaft length H" of the microneedle 410 is inserted into the eye.

As described above, the microneedle 410 has the width W₂. In some embodiments, the width W₂ can be from about 50 µm to about 500 µm, from about 50 µm to about 400 µm, from about 100 µm to about 400 µm, from about 200 µm to about 400 µm, or from about 100 µm to about 250 µm. In some embodiments, the microneedle 410 can be characterized by, for example, a bevel height H₂ to width W₂ ratio (i.e., a bevel aspect ratio (height: width)). In some embodiments, the microneedle 410 can have a bevel aspect ratio that is about 0.25:1, about 0.5:1, about 0.75:1, about 1:1, about 1.5:1, about 2:1, about 2.2:1, about 2.5:1, or about 3:1. In some embodiments, the microneedle 410 can have a bevel aspect ratio that is less than about 3.0:1, about 2.5:1, about 2.2:1, about 2.0:1 or about 1.5:1.

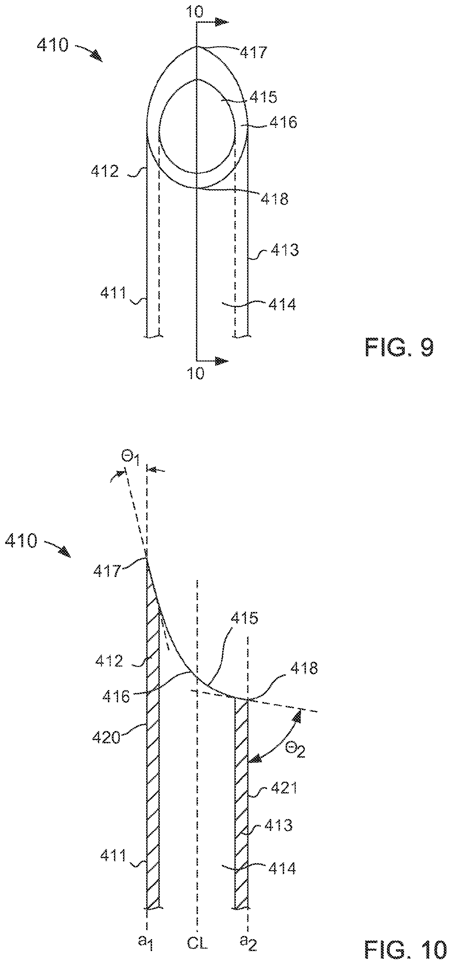

As shown in FIGS. 8 and 10, the bevel 416 is substantially curvilinear (e.g., the beveled surface is not planar). The bevel 416 can have any suitable radius of curvature (or radii of curvature). In some embodiments, the radius of curvature of the bevel 416 can be substantially consistent or continuous between the distal edge 417 and the proximal edge 418. In other embodiments, the radius of curvature can vary along the height H₂ of the bevel 416. As shown in FIG. 10, the first portion 420 of the walls 413 has and/or is associated with an axis ai that is substantially parallel to a centerline CL of the microneedle 410 and the second portion 421 of the walls 413 has and/or is associated with an axis a₂ that is substantially parallel to the centerline CL of the microneedle 410. The bevel 416 is arranged such that a first bevel angle Θ₁ (e.g., a tip angle or distal angle) is defined between the axis ai and a line that is tangent to the bevel 416 at or near the distal edge 417. Similarly, the bevel 416 is arranged such that a second bevel angle Θ₂ (e.g., a proximal angle or an inside angle) is defined between the axis a₂ and a line that is tangent to the bevel 416 at or near the proximal edge 418. In some embodiments, the first bevel angle Θ₁ can be smaller than the second bevel angle Θ₂. For example, in some embodiments, the first bevel angle Θ₁ can be less than about 20 degrees and the second bevel angle Θ₂ can be greater than about 30 degrees. In some embodiments, the first bevel angle Θ₁ can be less than 0.1 degree. In other embodiments, the first bevel angle Θ₁ can be from approximately 0.1 degree to approximately 1 degree. In still other embodiments, the first bevel angle Θ₁ can be from approximately 1 degree to approximately 5 degrees, including any fraction of a degree therebetween. In one embodiment, the microneedle 410 can have a first bevel angle Θ₁ from about 0.1 degree to about 30 degrees, or from about 1 degree to about 25 degrees, or from about 2 degrees to about 20 degrees, or from about 10 degrees to about 20 degrees. In some embodiments, the first bevel angle Θ₁ can be less than about 20 degrees or less than about 18 degrees. In still other embodiments, the first bevel angle Θ₁ can be about 12 degrees.

In some embodiments, the second bevel angle Θ₂ can be from about 20 degrees to about 30 degrees, or from about 20 degrees to about 45 degrees, or from about 20 degrees to about 60 degrees, or from about 20 degrees to about 75 degrees, or from about 20 degrees to about 90 degrees. In other embodiments, the second bevel angle Θ₂ can be less than 20 degrees yet still larger than the first bevel angle Θ₁. In some embodiments, the second bevel angle Θ₂ can be greater than about 30 degrees. In some embodiments, the second bevel angle Θ₂ can be between about 30 degrees and about 45 degrees. In other embodiments, the second bevel angle Θ₂ can be between about 45 degrees and about 90 degrees.

As a result, arrangement of the bevel 416 at or near the distal edge 417 can define a radius of curvature that is substantially larger than the arrangement of the bevel 416 at or near the proximal edge 418. In this manner, the bevel 416 and more particularly, the distal edge 417, can be sufficiently sharp (as described above), while the second portion of the bevel 417 can be configured to maintain the structural rigidity of the distal end portion 412 of the microneedle 410 and/or maintain the bevel height H₂ at or below a desired value. For example, the first bevel angle Θ₁ can be below a desired angle such that a thickness of the first portion 420 of the walls 413 is sufficiently thin to pierce ocular tissue without substantially bending, curving, deforming, and/or otherwise moving the ocular tissue prior to insertion. Conversely, the second bevel angle Θ₂ can be increased (e.g., relative to the first bevel angle Θ₁) to an extent such that a thickness of the second portion 421 of the walls 413 is sufficiently thick to maintain the structural rigidity of the distal end portion 412 of the microneedle 410 and/or such that the bevel height H₂ is at or below a desired value.

As shown in FIG. 8, the shaft length H" of the microneedle 410 is defined as the length from the distal edge 417 to the hub 419. In the drug delivery methods provided herein, the entire shaft length H" or substantially the entire shaft length H" of the microneedle 410 is inserted into the eye. In this regard, the user need not determine an optimal depth of microneedle insertion prior to insertion and/or employ visualization techniques during the insertion operation. The microneedle 410 can be defined by a shaft length H" that, in some embodiments, does not allow for piercing of the choroid when inserted into the sclera of the eye. In this regard, the microneedle 410 provided herein reduces variability associated with sclera and suprachoroidal drug delivery methods, and eliminates or substantially reduces the uncertainty associated with ocular drug delivery methods to the eye. In other embodiments, the microneedle 410 can be defined by a shaft length H" that does not allow for piercing of the lens when inserted into the vitreous of the eye. In this regard, the microneedles 410 provided herein reduce variability associated with intravitreal drug delivery methods, and eliminates or substantially reduces the uncertainty associated with ocular drug delivery methods to the eye.

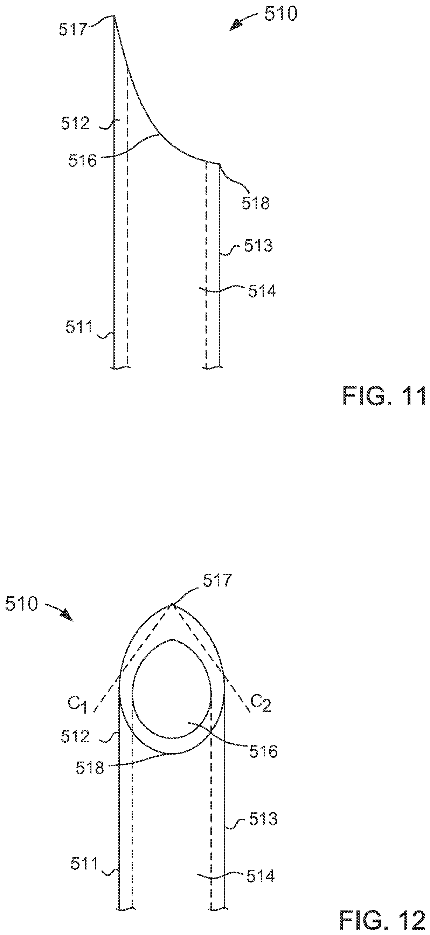

Although the microneedle 410 is shown in FIGS. 8-10 as including a bevel 416 or beveled surface that is substantially continuous, in other embodiments, a microneedle can have a bevel that is discontinuous and/or is defined in at least two planes. Similarly stated, although the microneedle is shown and described above as include a single bevel surface, in other embodiments, a microneedle can have multiple bevel surfaces. For example, FIGS. 11 and 12 illustrate a microneedle 510 according to another embodiment. As shown in FIG. 11, the microneedle 510 has a proximal end portion 511 and a distal end portion 512 and defines a lumen 514. The proximal end portion 511 can be substantially similar in form and function of the proximal end portion 311 of the microneedle 310 shown in FIG. 7. The distal end portion 512 includes and/or defines a bevel 516 having a distal edge 517 and a proximal edge 518. As shown in FIG. 11, the bevel 516 can be substantially curvilinear. In some embodiments, the bevel 516 can include a radius of curvature that is substantially similar to the radius of curvature of the bevel 416 described above with reference to FIGS. 8-10.

In some embodiments, the distal end portion 512 of the microneedle 510 can be cut or formed in a plane that is different from (e.g., substantially orthogonal to) the plane in which the bevel 516 is profiled (e.g., as shown in FIG. 11). For example, as shown in FIG. 12, the distal end portion 512 of the microneedle 510 can be cut along the lines Ci and C₂. As a result, the distal end portion 512 can be tapered between a first portion, which is near and/or adjacent to the proximal edge 518, and a second portion, which is near and/or adjacent to the distal edge 517. Thus, by cutting the distal end portion 512 along, for example, the lines C₁ and C₂ in FIG. 12, the distal edge 517 can be sharpened in two planes (e.g., by the bevel 516 and by the cuts along the lines Ci and C₂).

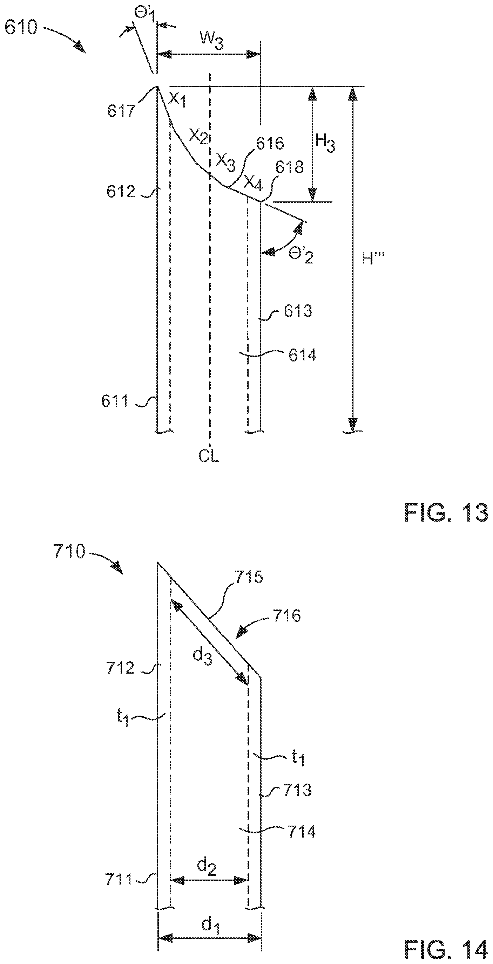

While the bevels 416 and 516 are shown as forming a substantially smooth curve or continuous surface, in other embodiments, a microneedle can include a bevel that is formed from any number of planes or segments (e.g., a three facet or a four facet bevel). For example, FIG. 13 is a schematic illustration of a microneedle 610 according to an embodiment. The microneedle 610 includes a proximal end portion 611, a distal end portion 612, and a set of annular walls 613. The microneedle 610 can have a shaft length H‴ that can substantially correspond to at least a portion of the eye. For example, in some embodiments, the shaft length H‴ can correspond to and/or be within a range of the thickness of the sclera (see e.g., FIGS. 3 and 4). Thus, in some embodiments, the shaft length H‴ can be any suitable length that does not allow for piercing of and/or extending through the choroid when the microneedle 610 is inserted into the posterior segment of the eye, as described above. In this regard, the microneedle 610 provided herein reduces variability associated with suprachoroidal drug delivery methods, and eliminates or substantially reduces the uncertainty associated with ocular drug delivery methods to and/or removal of substances from the eye. In some embodiments, the shaft length H"' can be any of the lengths described above with reference to the microneedle 310 or the microneedle 410, or any other shaft lengths referenced herein.

The walls 613 define a lumen 614 extending through the proximal end portion 611 and the distal end portion 612. The proximal end portion 611 can be substantially similar in form and function as the proximal end portion 311 included in the microneedle 310 described above with reference to FIG. 7. In this manner, the proximal end portion 611 can be physically and fluidically coupled to any suitable medical device, as described above. Although not shown in FIG. 13, in some embodiments, the proximal end portion 611 can be coupled and/or can include a base or hub. As described herein, in use the base or hub can include a surface that substantially circumscribes the shaft of the microneedle 610, and that can contact a portion of the target tissue (e.g., a surface of the eye) during use.

The distal end portion 612 of the microneedle 610 includes a bevel or beveled surface 616 having a distal edge 617 and a proximal edge 618. Similarly stated, the distal end portion 612 includes a surface (i.e., the bevel 316) that is slanted, sloped, angled and/or inclined from an outer surface of the walls 613. Said another way, the beveled surface 616 intersects the outer surface of the walls 613 at one or more angles to define one or more sharp edges (e.g., the distal edge 617 and/or the proximal edge 618), as defined herein. This arrangement allows the distal end portion 612 of the microneedle to pierce, separate and/or deform the target tissue to facilitate penetration of the shaft microneedle 610 therethrough. Similarly stated, the sharp distal edge 617 is configured to pierce the target tissue, such as ocular tissue, to facilitate defining a passageway within the target tissue to the desired location (e.g., the suprachoroidal space and/or the vitreous, as described herein).

While not shown in FIG. 13, the distal end portion 612 can define an opening substantially similar to the opening 315 or the opening 415 described above. In this manner, the distal end portion 612 can be configured to place the lumen 614 in fluid communication with a volume substantially outside the microneedle 610, such as for example, the suprachoroidal space.

In some embodiments, the microneedle 610 comprises multiple facets (also referred to as segments). For example, as shown in FIG. 13, the bevel 616 can include a first segment X₁, a second segment X₂, a third segment X₃, and a fourth segment X₄ (i.e., a four facet bevel). In another embodiment, the bevel includes a first segment X₁, a second segment X₂ and a third segment X₃ (i.e., a three facet bevel). In yet other embodiments, a microneedle can include any number of facets or bevel portions.

In some embodiments, the segments X₁, X₂, X₃, and X₄ can be substantially similar in length to each other. In other embodiments, the segments X₁, X₂, X₃, and X₄ can be substantially different lengths. Furthermore, the segments X₁, X₂, X₃, and X₄ can be configured to be substantially tangential to a radius of curvature (not shown) of the bevel 616. Therefore, as shown in FIG. 13, the segments X₁, X₂, X₃, and X₄ are arranged at various angles relative to a centerline CL of the microneedle 610 defined by the walls 613 of the microneedle 610. Expanding further, the segment X₁ can be arranged such that the distal edge 617 of the bevel 616 is sufficiently sharp (as described above) while the segments X₂, X₃, and X₄ can be arranged to provide structural rigidity to the distal end portion 612 of the microneedle 610 and/or maintain a bevel height H₃ (or a bevel aspect ratio) within a desired range.

In some embodiments, the first segment X₁ defines a first bevel angle Θ'₁ (e.g., a tip angle or distal angle) and another segment (e.g., segment X₄) defines a second bevel angle Θ'₂ (e.g., a proximal angle or an inside angle). In some embodiments, the first bevel angle Θ'₁ can be smaller than the second bevel angle Θ'₂. For example, in some embodiments, the first bevel angle Θ'₁ and/or the second bevel angle Θ'₂ can be any angle or within any range of angles described above with reference to the microneedle 410. As a result, arrangement of the bevel 616 at or near the distal edge 617 can be sufficiently sharp (as described herein), while the portion of the bevel 617 at or near the proximal edge 618 can be configured to maintain the structural rigidity of the microneedle 610 and/or maintain the bevel height H₃ at or below a desired value. For example, the first bevel angle Θ'₁ can be such that a thickness of a first portion of the walls 613 is sufficiently thin to pierce ocular tissue without substantially bending, curving, deforming, and/or otherwise moving the ocular tissue prior to insertion. Conversely, the second bevel angle Θ'₂ can be larger than the first bevel angle Θ'₁ to maintain the bevel height H₃ and/or the bevel aspect ratio (H₃:W₃) at or below any of the values specified herein.

In this manner, the height H₃ can be such that when the microneedle is disposed within the sclera, the opening (not shown in FIG. 13) is at a desired location of the sclera and/or suprachoroidal space (see e.g., FIGS. 3 and 4). For example, if the bevel height H₃ is too large, a portion of the opening may be disposed outside of (e.g., above) the sclera and/or into the conjunctiva. Such positioning may result in the deposition of substances in an undesirable portion of the eye. Thus, the multi-angle bevel arrangement maintains the bevel height H₃ such that when the distal edge 617 is disposed within the suprachoroidal space and/or adjacent the choroid, the opening does not extend beyond the innermost half of the sclera, third of the sclera, or quarter of the sclera. More particular, as described herein, in some embodiments, the microneedle 610 can be inserted into the ocular tissue at an angle that is between about 80 degrees and about 100 degrees relative to a tangential surface of the insertion site of the eye. When inserted in such an orientation and with the distal edge 617 disposed within the suprachoroidal space and/or adjacent the choroid, the bevel height H₃ can be such that the opening does not extend beyond the innermost half of the sclera, third of the sclera, or quarter of the sclera.

While shown in FIG. 13 as including four segments X₁, X₂, X₃, and X₄, in other embodiments, a microneedle can include any number of segments configured at any suitable angle relative to an axis defined by a set of walls of the microneedle. Moreover, in some embodiments, a microneedle can include additional bevel segments that are defined within a different plane, such as the "chisel cut" bevel surfaces shown and described in FIG. 12.

FIG. 14 is a schematic illustration of a microneedle 710 according to an embodiment. The microneedle 710 includes a proximal end portion 711, a distal end portion 712, and a set of annular walls 713. The proximal end portion 711 can be substantially similar in form and function as the proximal end portion 311 included in the microneedle 310 described above with reference to FIG. 7. In this manner, the proximal end portion 711 can be physically and fluidically coupled to any suitable medical device, as described above. The distal end portion 712 of the microneedle 710 includes a bevel 716. The bevel 716 can be substantially similar to the bevel 316 included in the microneedle 310, described above with reference to FIG. 7, and/or any of the other bevels or beveled surfaces described herein. The distal end portion 712 of the microneedle 710 is also configured to define an opening 715.

In some embodiments, the microneedle can have a shaft length (not identified in FIG. 14) that substantially corresponds to at least a portion of the eye. For example, in some embodiments, the shaft length can correspond to and/or be within a range of the thickness of the sclera (see e.g., FIGS. 3 and 4). Similarly stated, the microneedle 710 can have a shaft length that does not allow for piercing of and/or passing through the choroid when inserted into the posterior segment of the eye, as described herein. In this regard, the microneedle 710 provided herein reduces variability associated with scleral and suprachoroidal drug delivery methods, and eliminates or substantially reduces the uncertainty associated with ocular drug delivery methods to the eye. In other embodiments, the microneedle 710 can have a shaft length (not identified in FIG. 14) that does not allow for piercing of the lens when inserted into the posterior segment of the eye, as described above. In this regard, the microneedle 710 provided herein reduces variability associated with intravitreal drug delivery methods, and eliminates or substantially reduces the uncertainty associated with ocular drug delivery methods to the eye.

The walls 713 define a lumen 714 extending through the proximal end portion 711 and the distal end portion 712. The walls 713 have a thickness t₁ and define an outer diameter d₁ of the microneedle 710 and an inner diameter d₂ of the microneedle 710. In some embodiments, the outer diameter d₁ of the microneedle 710 is 30 gauge, 32 gauge, 34 gauge or 36 gauge. In some embodiments, the outer diameter d₁ can be from about 50 µm to about 500 µm, from about 50 µm to about 400 µm, from about 100 µm to about 400 µm, from about 200 µm to about 400 µm, or from about 100 µm to about 250 µm. In some embodiments, the inner diameter d₂ can be at least partially associated with a particle size of a drug to be conveyed therethrough. For example, in some embodiments, the inner diameter d₂ can be about 5 times the average particle size of a drug that is to be conveyed therethrough. In some embodiments, the inner diameter d₂ can be between about 100 µm and about 130 µm. In other embodiments, the inner diameter d₂ can be between about 110 µm and about 120 µm. In still other embodiments, the inner diameter d₂ can be less than 100 µm. In yet other embodiments, the inner diameter d₂ can be greater than 130 µm. The opening 715 defined by the distal end portion 712 has a diameter d₃ that is configured to be substantially greater than the inner diameter d₂ and substantially less than the outer diameter d₁.

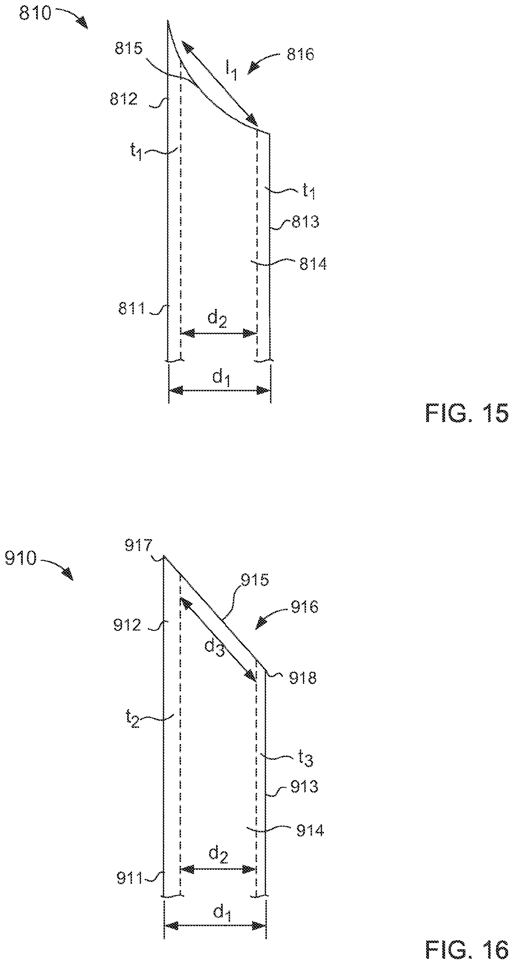

While the bevel 716 is shown in FIG. 14 as being substantially straight and/or planar, in some embodiments, a microneedle can include a curvilinear bevel and/or a multi-faceted beveled surface. For example, the microneedle 410 shown and described above has a curved bevel, and can have an inner diameter, an outer diameter and/or a wall thickness as described herein with reference to the microneedle 710. As another example, the microneedle 610 shown and described above has a multi-faceted bevel, and can have an inner diameter, an outer diameter and/or a wall thickness as described herein with reference to the microneedle 710s. As yet another example, FIG. 15 is a schematic illustration of a microneedle 810 according to an embodiment. The microneedle 810 includes a proximal end portion 811, a distal end portion 812, and a set of annular walls 813. The proximal end portion 811 can be substantially similar in form and function as the proximal end portion 311 included in the microneedle 310 described above with reference to FIG. 7. In this manner, the proximal end portion 811 can be physically and fluidically coupled to any suitable medical device, as described above. The distal end portion 812 of the microneedle 810 includes a bevel 816 that is substantially curvilinear. In some embodiments, the bevel 816 can be substantially similar to the bevel 416 included in the microneedle 410, described above with reference to FIGS. 8-10. The distal end portion 812 of the microneedle 810 is also configured to define an opening 815. The microneedle 810 can have a shaft length (not shown) that does not allow for piercing of the choroid when inserted into the posterior segment of the eye, as described above. In this regard, the microneedle 810 provided herein reduces variability associated with suprachoroidal drug delivery methods, and eliminates or substantially reduces the uncertainty associated with ocular drug delivery methods to the eye. In other embodiments, the microneedle 810 can have a shaft length (not shown) that does not allow for piercing of the lens when inserted into the posterior segment of the eye, as described above. In this regard, the microneedle 810 provided herein reduces variability associated with intravitreal drug delivery methods, and eliminates or substantially reduces the uncertainty associated with ocular drug delivery methods to the eye.

The walls 813 define a lumen 814 extending through the proximal end portion 811 and the distal end portion 812. The walls 813 have a thickness t₁ and define an outer diameter d₁ of the microneedle 810 and an inner diameter d₂ of the microneedle 810. In some embodiments, the outer diameter d₁ of the microneedle 810 is 30 gauge, 32 gauge, 34 gauge or 36 gauge. The opening 815 defined by the curvilinear bevel 816 has a length li that is configured to be greater than the inner diameter d₂ and less than the outer diameter d₁.

While walls 813 are shown in FIG. 15 as having the same thickness ti, in other embodiments, a thickness of a set of walls can be varied circumferentially about the microneedle. For example, FIG. 16 is a schematic illustration of a microneedle 910 according to an embodiment. The microneedle 910 includes a proximal end portion 911, a distal end portion 912, and a set of annular walls 913. The proximal end portion 911 can be substantially similar in form and function as the proximal end portion 311 included in the microneedle 310 described above with reference to FIG. 7. The distal end portion 912 of the microneedle 910 includes a bevel 916 having a distal edge 917 and a proximal edge 918. The bevel 916 can be substantially similar to the bevel 316 included in the microneedle 310, described above with reference to FIG. 7. The distal end portion 912 of the microneedle 910 is also configured to define an opening 915. The microneedle 910 can have a shaft length (not shown) that does not allow for piercing of the choroid when inserted into the posterior segment of the eye, as described above. In this regard, the microneedle 910 provided herein reduces variability associated with suprachoroidal drug delivery methods, and eliminates or substantially reduces the uncertainty associated with ocular drug delivery methods to the eye. In other embodiments, the microneedle 910 can have a shaft length (not shown) that does not allow for piercing of the lens when inserted into the posterior segment of the eye, as described above. In this regard, the microneedle 910 provided herein reduces variability associated with intravitreal drug delivery methods, and eliminates or substantially reduces the uncertainty associated with ocular drug delivery methods to the eye.

The annular walls 913 define a lumen 914 extending through the proximal end portion 911 and the distal end portion 912. The walls 913 define an outer diameter d₁ of the microneedle 910 and an inner diameter d₂ of the microneedle 910, as described above in reference to FIG. 14. The opening 915, defined by the distal end portion 912, has a diameter d₃ that is configured to be substantially greater than the inner diameter d₂ and substantially less than the outer diameter d₁.

As shown in FIG. 16, the walls 913 have a first thickness t₂ and a second thickness t₃. Expanding further, the thickness of the walls 913 can vary relative to an axis defined between a point (not shown) on the distal edge 917 and a point (not shown) of the proximal edge 918. For example, as shown, a first portion of the walls 913 that intersects a first end of the bevel 916 to form and/or define the distal edge 917 has the first thickness t₂. A second portion of the walls 913 that intersects a second end of the bevel 916, opposite the first end, to form and/or define the proximal edge 918 has the second thickness t₃. As show, the first thickness t₂ is greater than the second thickness t₃. In this manner, the first thickness t₂ can be sufficiently thick such that the bevel 916 can have a desired angle and surface area at the distal edge 917. Furthermore, the second thickness t₃ can be sufficiently thin such that the diameter d₃ is maintained.

While the first thickness t₂ and the second thickness t₃ are shown in FIG. 16 as being associated with the distal edge 917 and the proximal edge 918, in other embodiments, a microneedle can have a wall configured to have a thickness associated with a proximal edge that is thicker than a thickness associated with a distal edge. In such embodiments, the greater thickness at the proximal edge can be increase and/or maintain structural rigidity at a distal end portion of the microneedle. Furthermore, the smaller thickness at the distal edge can facilitate the insertion of the distal edge into ocular tissue without substantially elastically deforming the adjacent tissue (as described herein).

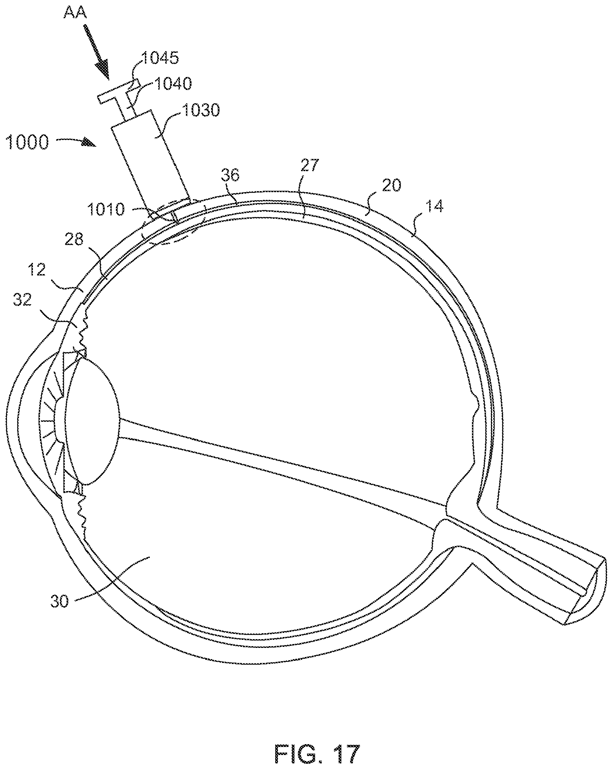

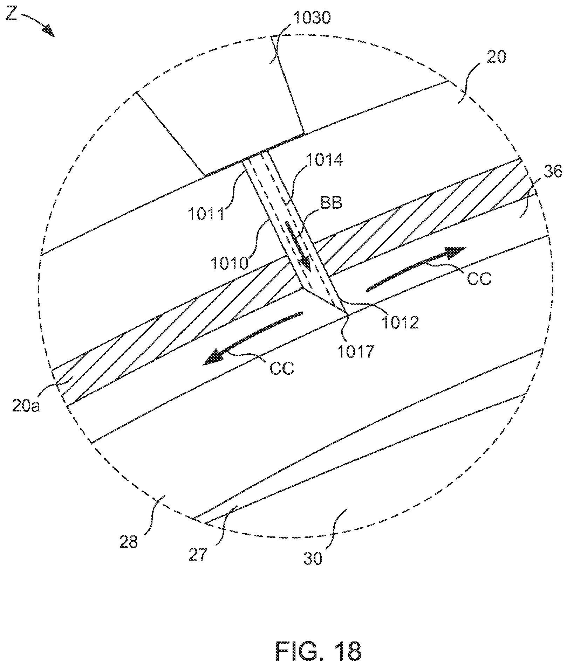

Any of the embodiments described above with reference to FIGS. 5-16 can be suitable for suprachoroidal drug delivery. For example, FIGS. 17 and 18 illustrate a medicament delivery device 1000 being used to deliver a drug formulation to the suprachoroidal space of an eye according to an embodiment. The medicament delivery device 1000 can be substantially similar to or the same as the medicament delivery device 200 described above with reference to FIG. 3, or any other delivery devices shown herein. In this manner, the infusion device 1000 includes a microneedle 1010, a cartridge housing 1030, and a cartridge 1040. The microneedle 1010 can be, for example, any of the microneedles described herein.

In some embodiments, a method of delivering a drug formulation to target ocular tissue includes inserting the microneedle 1010 into the sclera 20 of the eye 10 and advancing the microneedle 1010 through the sclera 20 such that the entire or substantially the entire shaft length (H' or H") of the microneedle 1010 between a proximal end portion 1011 and a distal end portion 1012 is disposed in the eye 10 (as shown in FIG. 17). More specifically, the microneedle 1010 can be inserted into the ocular tissue (e.g., the sclera) such that a base surface of the proximal end portion of the microneedle 1010 or a base surface of the cartridge is placed in contact with the ocular tissue. Thus, the entire shaft length of the microneedle 1010 can be inserted into the eye. The microneedle 1010 can have any suitable shaft length, such as the shaft lengths H' (see microneedle 310), H" (see microneedle 410)and H"' (see microneedle 610) described herein. Moreover, the base can substantially circumscribe the shaft. Thus, in some embodiments, as shown in FIG. 17, the microneedle 1010 can be inserted into the eye 10 at an angle that is between about 80 degrees and about 100 degrees relative to a surface of the insertion site of the eye. In some embodiments, the microneedle 1010 can be inserted into the eye 10 at an angle of about 90 degrees, and such that a portion of the base that circumscribes the shaft is in contact with the surface of the eye 10.

As shown in FIG. 18, the shaft length of the microneedle 1010 is such that the distal end portion 1012 of the microneedle 1010 is disposed within a lower portion 20a of the sclera 20 and/or the suprachoroidal space 36. Furthermore, the shaft length of the microneedle 1010 is such that the distal end portion 1012 and more specifically, a distal edge 1017, does not substantially pierce, extend through and/or deform the choroid 28. The shaft length can be any suitable length as described herein.

Moreover, the distal end portion 1012 of the microneedle 1010 includes a beveled surface, which can be similar to any of the beveled surfaces described herein. In particular, the beveled surface is configured such that the distal end opening (not identified) of the microneedle 1010 is at a desired location of the sclera 20 and/or suprachoroidal space 36. Thus, in some embodiments, the bevel height and/or the bevel aspect ratio is such that when the distal edge 1017 is disposed within the suprachoroidal space and/or adjacent the choroid, the opening does not extend outside of the lower portion 20a of the sclera 20. The lower portion 20a of the sclera 20 can include the lower half of the sclera 20, third of the sclera 20, or quarter of the sclera 20. More particularly, because the microneedle 1010 can be inserted into the ocular tissue at an angle that is between about 80 degrees and about 100 degrees relative to a tangential surface of the insertion site of the eye, the circumferential angular orientation (i.e., the angle of rotation about the axis of the microneedle) need not be controlled.

With the distal end portion 1012 of the microneedle 1010 disposed in the lower portion 20a of the sclera 20 and/or the suprachoroidal space 36, in one embodiment, the method further includes delivering a drug formulation through a lumen 1014 defined by the microneedle 1010 and into the lower portion 20a of the sclera 20 and/or the suprachoroidal space 36. More specifically, with the distal end portion 1012 of the microneedle 1010 disposed in the lower portion 20a of the sclera and/or the suprachoroidal space 36, the cartridge 1040 can be moved within the cartridge housing 1030, as described in detail with reference to FIG. 5. Furthermore, a plunger 1045 included in the cartridge 1040 can be moved relative to a cartridge body (not shown in FIGS. 17 and 18) to increase a pressure within an inner volume of the cartridge body, as indicated by the arrow AA in FIG. 17. The increase in pressure can be such that a drug formulation disposed within the inner volume of the cartridge is expelled through the lumen 1014 defined by the microneedle 1010, as indicated by the arrow BB in FIG. 18.

With the distal end portion 1012 of the microneedle 1010 disposed within the lower portion 20a of the sclera 20 and/or the suprachoroidal space 36, the drug formulation can be expelled from the lumen 1014 of the microneedle 1010 to substantially expand a volume of the suprachoroidal space 36, as described above. In this manner, the drug can flow circumferentially within the suprachoroidal space 36 to be delivered to, for example, the posterior region of the eye 10, as indicated by the arrows CC in FIG. 18. The drug formulation can be any suitable medicament suitable to treat, for example, ocular disease, as described in further detail below. With the medicament delivered to the suprachoroidal space 36 the method includes moving the medicament delivery device 1000 in a direction opposite the arrow AA in FIG. 17 to remove the microneedle 1010 from the eye 10. As described above, the arrangement of the microneedle 1010 is such that the amount of damage to surrounding tissue (e.g., the retina or choroid) due to the insertion of the microneedle 1010 is substantially reduced or eliminated.

Although FIGS. 17 and 18 illustrate the entire microneedle 1010 being inserted into the eye 10 and into the suprachoroidal space. However, in other embodiments, only a portion of a microneedle 1010 need be inserted into the portion of the eye 10. For example, in some embodiments, only a bevel portion of a microneedle is disposed within ocular tissue, for example, through the sclera to place a lumen of the microneedle in fluid communication with the suprachoroidal space.

Some of the microneedles and methods described herein can be used to deliver a drug to the suprachoroidal space of the eye. Injection may be performed by inserting the microneedle anterior to, at, or posterior to, the equator of the eye. For example, in some embodiments, the microneedle is inserted approximately 0.3 mm to 0.6 mm posterior to the limbus. In the methods provided herein, the entire shaft of the microneedle or substantially the entire shaft of the microneedle is inserted into the eye without damaging the retina or lens.

In some embodiments, the eye is a pediatric eye. In the methods described herein, the entire microneedle shaft length or substantially the entire microneedle shaft length (H' or H", see FIGS. 7 and 8) is inserted into the eye, for example, into the vitreous or sclera, and the drug is infused through the microneedle into the vitreous or suprachoroidal space. In this regard, the microneedle shaft length controls the depth of microneedle insertion. This control mechanism allows for the user of the microneedle to achieve reliable and reproducible drug delivery to the vitreous or suprachoroidal space without further optimization. Furthermore, the shaft length can be reduced any suitable amount in accordance with pediatric ocular anatomy.

Although shown in FIGS. 17 and 18 as delivering a substance to the suprachoroidal space, in some embodiments, the microneedles described herein are used to deliver one or more drugs (e.g., VEGF inhibitor, topoisomerase inhibitor, diagnostic agent)to the vitreous of an eye of a pediatric patient in need thereof. In the methods described herein, the entire microneedle shaft or substantially the entire microneedle shaft (H' or H", see FIGS. 7 and 8) is inserted into the pediatric eye and the drug is delivered, injected and/or infused through the microneedle into the vitreous. In this regard, the microneedle shaft length controls the depth of microneedle insertion. This built in control mechanism allows for the user of the microneedle to achieve reliable and reproducible drug delivery to the vitreous. In one embodiment, the microneedle used in the methods provided herein includes a multi-angle bevel, such as, for example, a three facet bevel or a four facet bevel, as described above.

Provided herein are methods for treating retinoblastoma in a patient in need thereof, and in particular, a pediatric patient in need thereof. Retinoblastoma is one of the most common primary intraocular tumor in children and one of the five most common childhood cancers. There is approximately 1 child born with retinoblastoma in 18,000 live births in the United States. A retinoblastoma tumor may be heritable and bilateral, resulting from a second mutation in developing retinal cells in an infant who harbors a germline mutation in the retinoblastoma gene. The tumor may grow within the retina and develop neovascularization, extending toward the vitreous (endophytic growth pattern), toward the choroid (exophytic growth pattern), a combination of both, or diffusely infiltrate the retina. Retinoblastoma often forms spheroids of tumor and invades the avascular vitreous, in a process known as vitreous seeding. Therefore, a type of therapy which will locally deliver chemotherapeutic agents, including to vitreous tumor seeds, is desirable.

Intra-arterial chemotherapy (IAC) has been utilized via radiologic guided trans-arterial cannulization of the ophthalmic artery. More recently, trans-arterial chemotherapy of retinoblastoma using a microcatheter has been employed; however, this method requires multiple injections and is associated with risks such as stroke. Moreover, the technique has been shown to be ineffective in approximately 1/3 of cases. Reasons for failure of chemoreduction and IAC include most importantly failure to control vitreous seeds, followed by resistant well differentiated tumor, intraretinal tumor and subretinal tumor. Although a recent preliminary study showed evidence of retinoblastoma tumor control, including control of vitreous seeds with intravitreal injections of methotrexate using a 30 gauge needle, there are risks of seeding the tumor when utilizing a 30 gauge needle (Kivela et al. Intravitreal methotrexate for retinoblastoma. Ophthalmology 2011; 118, 1689-6; Karcioglu et al. Tumor seeding in ocular fine needle aspiration biopsy. Ophthalmology 1985; 92, 1763-1767). Accordingly, a local delivery method that reduces or eliminates the risks of tumor seeding is desirable in the field of retinoblastoma treatment and therapy.

In some embodiments, a method for treating retinoblastoma is provided, comprising inserting at least one microneedle into the vitreous of the eye of the patient (e.g., a pediatric patient), and delivering, injecting and/or infusing a drug formulation into the vitreous of the eye through the microneedle. In a further embodiment, the drug formulation comprises an effective amount of a topoisomerase inhibitor, e.g., topotecan. Any of the microneedle embodiments described herein can be configured to deliver an effective amount of a suitable medicament for the treatment of retinoblastoma, or other ocular cancer. For example, any of the embodiments described herein can deliver vincristine, paclitaxel, cisplatin, carboplatin, teniposide, etoposide, cyclophosphamide, ifosfamide, doxorubicin, idrubicin, topotecan, and/or any other suitable medicament. In one embodiment, topotecan is delivered to the vitreous with one of the methods described herein. In some instances, the patient is a pediatric patient.

In one embodiment, provided herein are methods for decreasing the size of an intraocular retinoblastoma tumor. In one embodiment, the method comprises infusing an effective amount of topotecan into an eye of the patient, wherein the eye comprises one or more retinoblastoma tumors, and the effective amount of topotecan is infused into the vitreous through the at least one microneedle. In a further embodiment, the patient is a pediatric patient. In one embodiment, the drug is infused into the vitreous according to an hourly, daily, or weekly dosing regimen. In one embodiment, the drug is infused into the eye once weekly. In a further embodiment, the drug is infused into the eye once weekly for two, three, four, five, or six weeks. In a further embodiment, the drug is infused into the eye once weekly for three weeks.

The dosage of topotecan can vary, as appreciated by those of skill in the art. For example, in one embodiment, the topotecan is administered at a dose of between about 1µg/50µL and about 100 µg/50µL per dose. In a further embodiment, topotecan is administered at a dosage of about 1µg, about 5µg, about 10µg, about 20µg, about 50µg, about 75µg, or about 100µg. In a further embodiment, topotecan is administered at a dose of about 10µg. In one embodiment, when the microneedle is used to infuse topotecan, the tumor area is reduced to a greater extent in comparison to the reduction in tumor area that occurs when the same dosage and dosing regimen of topotecan is infused into the vitreous using a 30 gauge needle.

In another embodiment, when the microneedle is used to infuse topotecan or other chemotherapeutic agent, the number of vitreous tumor seeds is reduced to a greater extent compared to the reduction in the number of vitreous tumor seeds that are present after infusion of topotecan using a 30 gauge needle under the same dosing regimen.

In one embodiment, the method comprises inserting a microneedle through the ciliary body of the pediatric eye wherein the entire shaft or substantially the entire shaft of the microneedle is inserted into the eye, and infusing a drug into the vitreous. In a further embodiment, the eye is a pediatric eye.

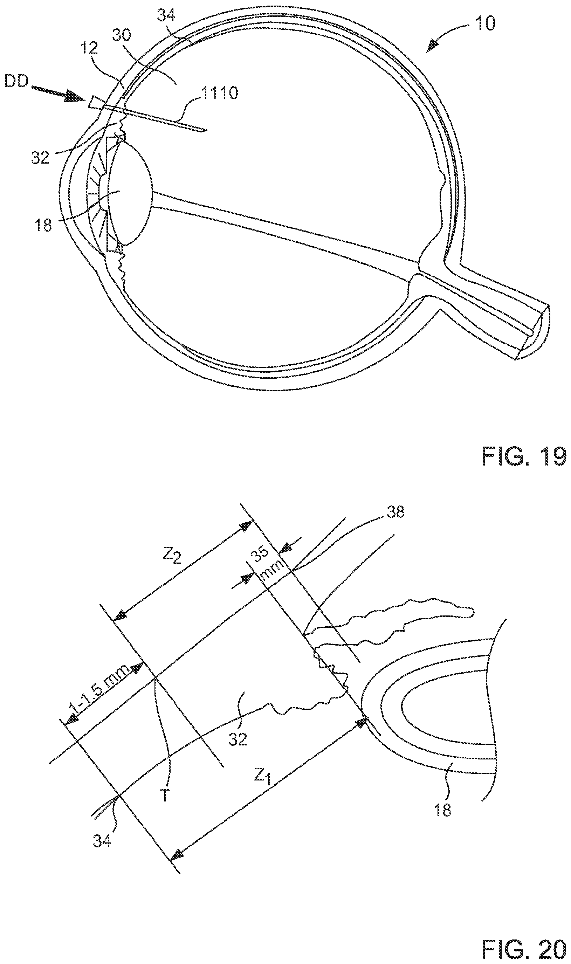

For example, in one embodiment, a method for treating an intraocular tumor in a child is provided (see FIGS. 19 and 20). As shown in Table 1 below and in the corresponding FIGS. 19 and 20, the length of the ciliary body 32 of a child is substantially smaller than the length of a ciliary body of an adult. Thus, the target location for the insertion of the microneedle into the sclera 20 is substantially reduced in the pediatric eye.

| Table 1 | | |
|---|---|---|
| Age | Length of Ciliary Body (mm) Zi | Limbus to Sclerotomy Distance (mm) Z2 |
| < 6 months | 2.23±0.06 Nasal | 1.5** |
| | 2.48±0.07 Temporal | |
| 6 to 12 months | 2.69±0.01 Nasal | 2.0 |
| | 2.96±0.14 Temporal | |
| 1 year to 2 years | 2.98±0.09 Nasal | 2.5 |
| | 3.15±0.09 Temporal | |
| 2 years to 6 years | 3.25±0.11 Nasal | 3.0 |
| | 3.85±0.12 Temporal | |
| Adult | 3.64±0.11 Nasal | 3.5 |
| | 4.32±0.13 Temporal | |

In this manner, the arrangement of any of the embodiments described above with reference to FIGS. 5-16 is such that the embodiments are suitable intravitreal drug delivery. Therefore, in some embodiments, the method includes inserting a microneedle 1110 into the sclera 20 of the eye 10 and advancing the microneedle 1110 through the ciliary body 32 such that the entire or substantially the entire shaft (H' or H") of the microneedle 1110 is disposed in the eye 10, as indicated by the arrow DD in FIG. 19. The method further includes delivering a medicament through a lumen (not shown) defined by the microneedle 1110 and into the vitreous 30. The medicament can be any suitable medicament suitable to treat, for example, retinoblastoma. With the medicament delivered to the vitreous the method includes moving the microneedle 1110 in a direction opposite the arrow DD to remove the microneedle 1110 from the eye 10.

In some embodiments, the microneedle 1110 is configured such that the tract produced by the insertion of the microneedle 1110 is sufficiently small so that tumor (e.g., retinoblastoma) seeds within the vitreous 30 cannot substantially move within the tract. Therefore, the risk of seeding (e.g., spreading) the tumor is greatly reduced if not eliminated all together. Furthermore, the microneedle 1110 is fabricated such that the amount of damage to surrounding tissue (e.g., the lens 18 or retina 34) due to the insertion of the microneedle 1110 is substantially reduced or eliminated. For example, in some embodiments, the microneedle 1110 can include a bevel of the types shown and described herein, which can contribute to and/or result in a reduced needle tract.

FIG. 19 illustrates the entire microneedle 1110 being inserted into the eye 10 and into the vitreous. However, in other embodiments, only a portion of a microneedle need be inserted into the portion of the eye 10. For example, in some embodiments, only a bevel portion of a microneedle is disposed within ocular tissue, for example, within the sclera, the suprachoroidal space, and/or the vitreous.

While the embodiments and methods herein describe delivering a medicament to a target tissue, in other embodiments, the embodiments described herein can be configured to facilitate a biopsy or other aspiration procedure. Some biopsy techniques include creation of a scleral flap and suturing of the scleral flap, two procedures that put the eye at risk for perforation and tumor seeding. In some instances, fine needle aspiration biopsy of the tumor with commercially available 27 and 30 gauge needles and methods can still result in tumor seeding. This tumor seeding increases the risk for metastasis and mortality from the tumor. Therefore, in some instances, a microneedle can be inserted into the eye in order to extract a biological tissue, fluid, or molecule from the sclera or corneal stroma.

In other instances, a microneedle can be inserted into the vitreous of an eye to core a target tissue such as, for example, a tumor. In some instances, the tumor is a retinoblastoma. The arrangement of the embodiments described herein can be such that the extraction of the biological tissue, fluid, or molecule sample results in less accumulation of the biological tissue, fluid, or molecule in comparison to the accumulation of the biological tissue, fluid, or molecule in the needle tract that occurs following a extraction of a biological tissue, fluid, or molecule sample using a 30 gauge needle. For example, in some embodiments, a retinoblastoma is extracted from an eye and less retinoblastoma seeds accumulate in the needle tract in comparison to extraction of retinoblastoma using a 30 gauge needle. In one embodiment, retinoblastoma cells are not present in the needle tract following extraction of a retinoblastoma tumor, or portion thereof, using one of the microneedles described herein.

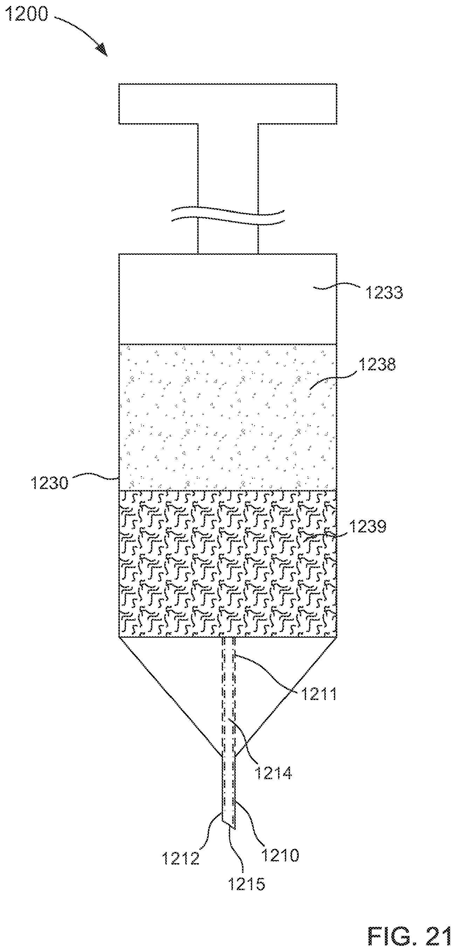

Any of the embodiments described herein can be used in any suitable system and/or with any suitable method for administration of a fluid drug formulation to, or withdrawal of fluid from, one or more biological tissues. For example, FIG. 21 illustrates medicament delivery device 1200 that includes a microneedle 1210 according to an embodiment. The microneedle 1210 defines a lumen 1214 through which a fluid drug formulation can be delivered to the eye or through which a biological fluid can be withdrawn from the eye. The microneedle 1210 has a proximal end portion 1211 and a distal end portion 1212. The distal end portion 1212 includes and/or otherwise defines a beveled tip of the types shown and described herein, and defines an opening 1215 of the lumen 1214. The microneedle 1210 is configured extend from a drug housing 1230 defining an inner volume 1233 for containing a fluid drug formulation. The inner volume 1233 is in operable communication (e.g., fluid communication) with the lumen 1214 defined by the microneedle 1210.

As shown, in some embodiments, the inner volume 1233 can include two fluid drug formulations 1238 and 1239 for injection into the eye. Although not shown, in other embodiments, more than two or less than two fluid drug formulations can be disposed in the inner volume 1233. In some instances, the first fluid drug formulation 1238 can have a different viscosity than the second fluid drug formulation 1239, such that the fluid drug formulations can be injected in series through the lumen 1214 and out the opening 1215 of the microneedle into the biological tissue. In one embodiment, the first fluid drug formulation 1238 has a greater viscosity than that of the second fluid drug formulation 1239 such that the second fluid drug formulation 1239 facilitates delivery of the first fluid drug formulation 1238 to the target biological tissue.

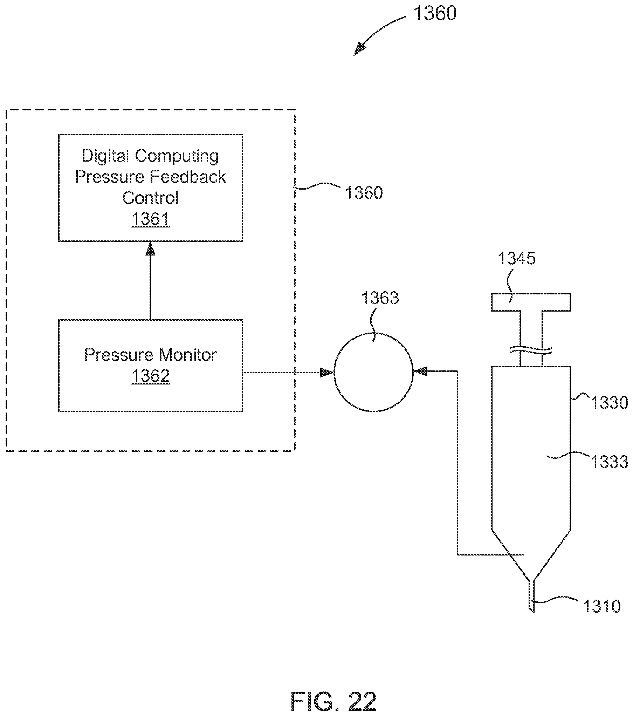

The apparatus of the present invention and as defined in the appended claims comprises a pressure sensor and a pressure feedback control system. FIG. 22 illustrates system 1360 includes a drug housing 1330 that defines an inner volume 1333, a plunger 1345, and a microneedle 1310 extending from the syringe in fluid communication with the reservoir. The system 1360 further includes a sensor 1363 and pressure feedback control system 1360 operably connected to the drug housing 1330. The pressure feedback control system 1360 includes a pressure monitor 1362 and digital computing pressure feedback control 1361. The pressure feedback control system 1360 can further include one or more valves, pumps, sensors, actuators, microprocessors, and/or memories (not shown in FIG. 22). For example, in an embodiment, the pressure feedback control system 1360 can include a valve that automatically closes in response to the pressure reaching a pre-determined value.

The pressure feedback control systems 1360 is used to monitor and control the pressure being applied to the drug housing 1330 during the insertion process, thereby monitoring and controlling the position of the microneedle 1310 in the biological tissue. In one embodiment, the pressure feedback control system 1360 is used to monitor and control the pressure being applied to the plunger 1345 during the injection process. In another embodiment, the pressure feedback control system 1360 is used for combinations of the foregoing. For example, injection into the suprachoroidal space generally requires a lower pressure than injection into the sclera. Thus, the pressure feedback control system 1360 can be utilized to facilitate placement of the microneedle 1310 and injection of the fluid drug formulation into the desired target biological tissue (e.g., by injecting some nominal amount of fluid to determine the position and proper placement of the microneedle).

Although not shown, any other type of control system or combination thereof can be used to control the transport of drug formulation or biological fluid through the hollow microneedle 1310. For instance, in one embodiment, the system 1360 can include a micropump, a microvalve, and a positioner, with a microprocessor programmed to control a pump or valve to control the rate of delivery of a drug formulation through the microneedle 1310 and into the ocular tissue. The flow through a microneedle 1310 may be driven by diffusion, capillary action, mechanical displacement, electrosmosis, electrophoresis, convection, or other driving forces. Devices and microneedle designs can be tailored using known pumps and other devices to utilize these drivers. In one embodiment, the system 1360 can further include an iontophoretic apparatus, similar to that described in U.S. Patent 6,319,240 to Beck, for enhancing the delivery of the drug formulation to the ocular tissue. In another embodiment, the system 1360 can further include a flowmeter or other means to monitor flow through the microneedle 1310 and to coordinate use of the pumps and valves.

The flow of drug formulation or biological fluid can be regulated using various valves or gates known in the art. The valve may be one which can be selectively and repeatedly opened and closed, or it may be a single-use type, such as a fracturable barrier. Other valves or gates used in the system 1360 can be activated thermally, electrochemically, mechanically, or magnetically to selectively initiate, modulate, or stop the flow of material through the microneedle 1310. In one embodiment, the flow is controlled with a rate-limiting membrane acting as the valve.

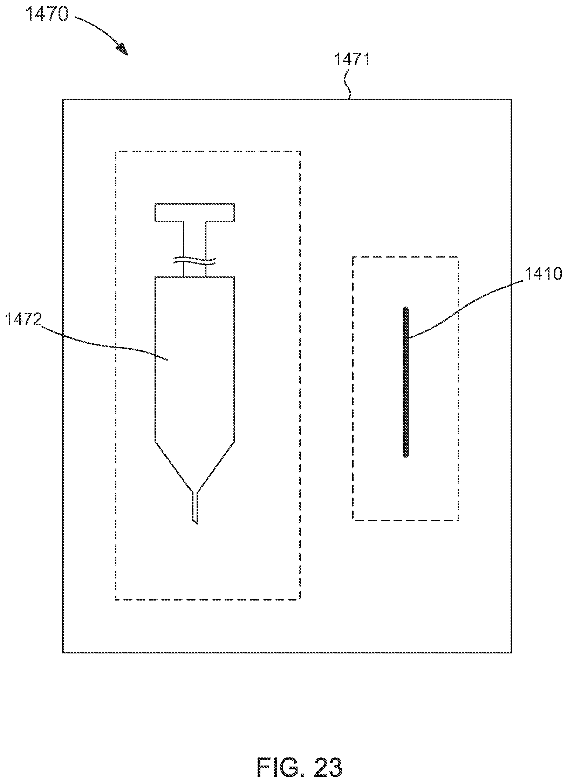

Any of the embodiments described herein can be included in any suitable kit and/or packaging. In some examples not forming part of the invention, portions of a kit can be packaged together or separately. For example, FIG. 23 illustrates a kit 1470 according to an example not forming part of the invention. The kit 1470 includes a sterile package 1471 having one or more microneedles 1410 and one or more infusion devices 1472 (e.g., syringes) disposed therein. The infusion device 1472 defines a fluid drug reservoir (not shown) into which a drug formulation optionally can be pre-loaded. Alternatively, in other embodiments, the kit 1470 can include one or more adapters (not shown) either in place of or in addition to the infusion device 1472 to facilitate attachment of the microneedle 1410 to the packaged infusion device 1472 or any other conventional infusion device and/or syringe. The one or more microneedles 1410 in the kit 1471 may comprise a range of different lengths or geometries in accordance with the embodiments described herein.

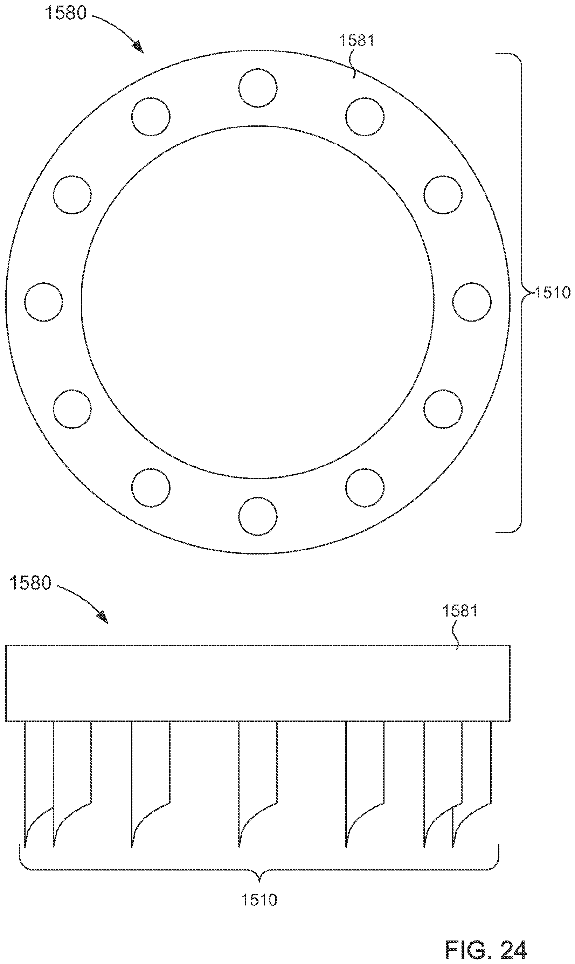

In some embodiments, any number of microneedles can be stored in, for example, an array or the like. For example, FIG. 24 illustrates a top view and a front view of a microneedle array 1580 according to an embodiment. The microneedle array 1580 includes an annular-shaped base 1581 having an array of microneedles 1510 extending therefrom. A fluid drug reservoir (not shown) may be incorporated directly into the annular-shaped base 1581 or in fluid connection thereto, such that a fluid drug formulation may be injected through the annular array of microneedles 1510. Each of the microneedles included in the array of microneedles 1510 can include one or more beveled distal ends, as described herein. In one embodiment, the annular array 1581 of microneedles 1510 has a diameter substantially similar to the diameter of the cornea. For example, if the array 1581 diameter is somewhat smaller than the diameter of the cornea, then the microneedle array 1581 can be positioned to make injections into the cornea along the corneal edge of the limbus. Similarly, if the array 1581 diameter is somewhat larger than the diameter of the cornea, then the microneedle array 1581 can be positioned to make injections into the conjunctiva, sclera or subconjunctival space along the sclera edge of the limbus. In this manner, the microneedle array 1580 can be a component of treatments of glaucoma, especially for targeting of the trabecular meshwork.

Any of the embodiments described herein can be used in any suitable method for administering a drug into an eye of a patient, wherein these methods are not part of the invention. In some embodiments, a method can include inserting a microneedle into an outer tissue of the eye and a inserting a fluid drug formulation through the channel of the microneedle and into the outer tissue of the eye. The outer tissue includes but is not limited to the sclera, cornea, corneal stroma, choroid, suprachoroidal space, conjunctiva, subconjunctival space, and subretinal space. The microneedle systems can also be used to deliver drug to tissues and sites proximal to the outer tissue, including trabecular meshwork, ciliary body, aqueous humour or vitreous humour. In some embodiments, the fluid drug formulation released from the microneedle into the outer tissue subsequently spreads to one or more tissues proximal to the outer tissue. For example, the fluid drug formulation may subsequently spread to the trabecular meshwork, the inter photo receptor space between the rod and cone outer segments and/or the pigment epithelium, the aqueous humour or vitreous humour, or the ciliary muscle. As used herein, the term "spread" refers to transport or movement of the fluid away from the initial site of injection, where the movement may occur due to one or more forces, including diffusion. In other embodiments, the fluid drug formulation remains substantially at the site of injection and does not substantially spread to other tissues at the time of injection. In such embodiments, there may be subsequent movement of the drug from the site of injection after the injection.

In some embodiments, a method can further include using a pressure-guided feedback system to determine the location of the opening of the microneedle in the eye and/or for controlling the fluid drug formulation infusion. For example, the method can include a pressure-guided feedback system including measuring (i) the pressure applied to the microneedle during its insertion into the eye and/or (ii) the pressure of the fluid drug formulation during its infusion into the sclera, cornea, corneal stroma, choroid, suprachoroidal space, conjunctiva, or subretinal space. In an embodiment, a drop in the pressure resisting infusion of the fluid drug formulation is used to signal that the microneedle is inserted an amount effective to place the opening of the microneedle in fluid communication with the suprachoroidal space.

In another aspect, a method can include aspirating a fluid from an eye of a patient. The method can include inserting at least one hollow microneedle into the tissue of the eye at an insertion site, and aspirating fluid from the insertion site into the at least one microneedle. For example, the at least one hollow microneedle may be inserted into the sclera of the eye at an insertion site to remove fluid from the suprachoroidal space. Aspiration of fluid from the eye can be particularly advantageous prior to surgical intervention to determine appropriate therapeutic treatment. For example, in an embodiment the aspiration and analysis of fluid from the suprachoroidal space of a patient may be desirable prior to conducting retinal reattachment surgery to identify appropriate cytokine and/or inflammatory mediators for individual treatment prior to reattachment of the retina. As another example, the at least one hollow microneedle can be inserted into the cornea of the eye at an insertion site to remove fluid from the cornea and/or from the anterior chamber.

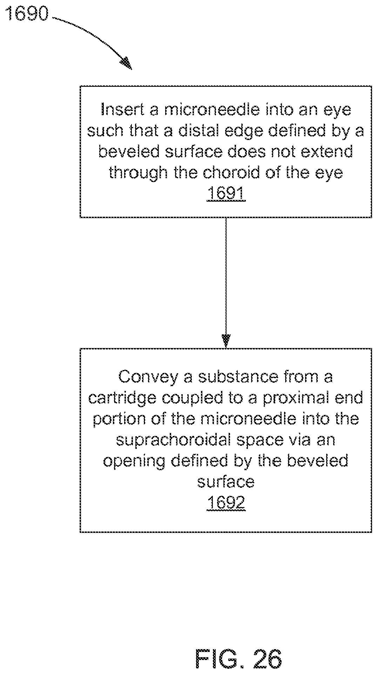

FIG. 26 is a flowchart illustrating a method 1690 of delivering an effective amount of a drug to a target ocular tissue, according to an embodiment. The method 1690 includes inserting a microneedle into an eye such that a distal edge defined by a beveled surface of the microneedle does not extend through the choroid of the eye, at 1691. The microneedle can be any of the microneedles described herein (e.g., the microneedles 310, 410, 510, 610, 710, 810, 910, 1010, 1110, 1210, 1310, 1410, and/or 1510). As such, the microneedle can have a proximal end portion that is configured to be operably coupled to a delivery device (e.g., the medicament delivery devices 200, 1000, and/or 1200) and a distal end portion that includes and/or forms the beveled surface that defines an opening. In some embodiments, the microneedle can include a beveled surface that is substantially similar to the beveled surface 416 of the microneedle 410 (see e.g., FIGS. 8-10) and/or the beveled surface 616 of the microneedle 610. Thus, the beveled surface can define a tip angle that can be less than, for example, about 20 degrees and an inside angle that can be greater than the tip angle.

As described above with reference to the microneedles 410, 610 and 1010, the beveled surface can have a height such that when the microneedle is inserted into the eye, the beveled surface is within at least one of a suprachoroidal space or a lower portion of the sclera (see e.g., FIG. 18). In some embodiments, the arrangement of the microneedle can be such that the entire shaft or substantially the entire shaft of the microneedle is disposed within the ocular tissue. Similarly stated, the microneedle can be inserted into the eye to a place a surface of the infusion device in contact with a surface of the eye. In some embodiments, the microneedle can be inserted into the ocular tissue at an angle that is between about 80 degrees and about 100 degrees relative to a tangential surface of the insertion site of the eye. In some embodiments, the microneedle can be inserted into the ocular tissue at about 90 degrees relative to the tangential surface of the insertion site of the eye. Furthermore, the microneedle can be inserted into the ocular tissue at any suitable angular orientation relative to a centerline of the microneedle. In other words, the beveled surface can be in any radial orientation relative to the tangential surface of the insertion site of the eye.

With the proximal end portion of the microneedle operably coupled to, for example, a medicament delivery device, a substance is conveyed from a cartridge coupled to the proximal end portion of the microneedle and into the suprachoroidal space via the opening defined by the beveled surface, at 1692. More specifically, the cartridge (e.g., the cartridge 240 included in the medicament delivery device 200 of FIG. 6) can be manipulated (e.g., by a plunger or the like) within an infusion device to increase a pressure within an inner volume of the cartridge. The increase in pressure can be such that a substance (e.g., a drug formulation) disposed within the inner volume of the cartridge is expelled through the opening defined by the microneedle. With the beveled surface disposed within the lower portion of the sclera and/or the suprachoroidal space, the substance can be expelled from the opening to substantially expand a volume of the suprachoroidal space, as described above. In this manner, the substance can flow circumferentially within the suprachoroidal space to be delivered to a target ocular tissue. In some embodiments, the substance can be any suitable medicament suitable to treat, for example, ocular disease, as described in further detail below.

### EXAMPLES

The examples below are for illustrative purposes only.

### Example 1. Inhibition of retinoblastoma cell growth using microneedle injection

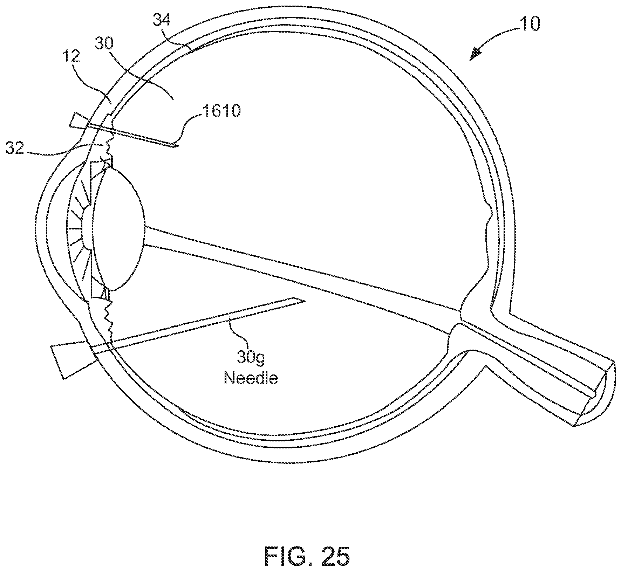

The delivery of substances to the eye and the inhibition of the growth of retinoblastoma cells using the microneedles described herein compared to standard needles was evaluated. FIG. 25 is a schematic depiction of the microneedle 1610 of the invention versus a 30 gauge needle, both inserted into an eye. Specifically, the microneedle 1610 and the 30 gauge needle are shown inserted into the sclera, through the ciliary body, and into the vitreous.

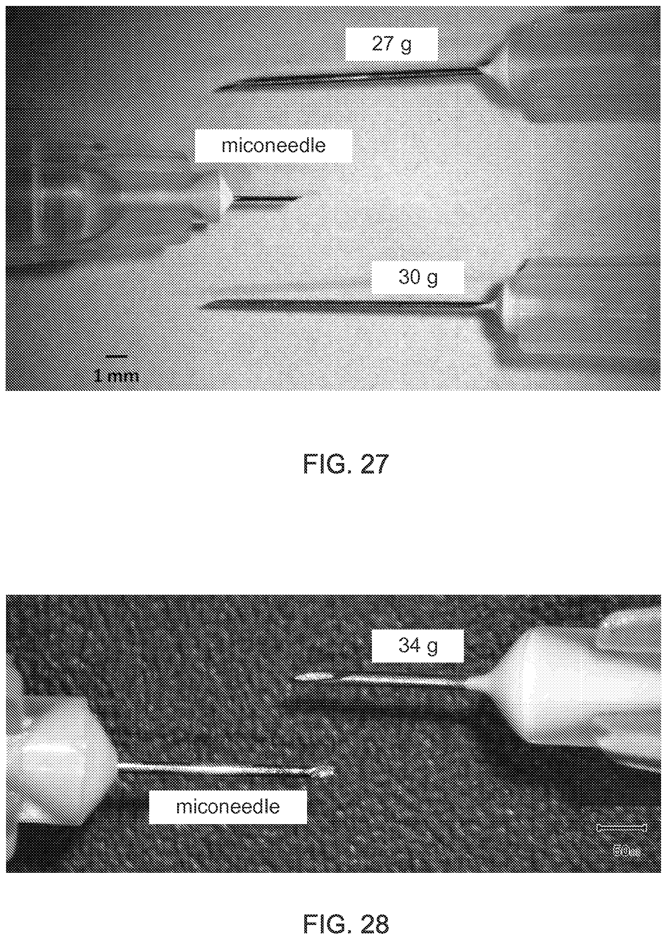

FIG. 27 shows a microneedle such as those described herein (shown in the middle) in comparison to 28 gauge (shown at the top) and 30 gauge (showed at the bottom) standard needles. FIG. 28 shows the (shown at the bottom) in comparison to a 34 gauge standard needle (shown at the top). The microneedle is shorter (4mm) and narrower than standard 26 and 30 gauge needles, each of which have a length of approximately 10 mm. Thus, the size of the microneedle is more appropriate for the pediatric eye than 26 gauge or 30 gauge needles. In addition, the microneedle has a shallower bevel (aspect ratio) with a smaller opening than the standard 34 gauge needle.

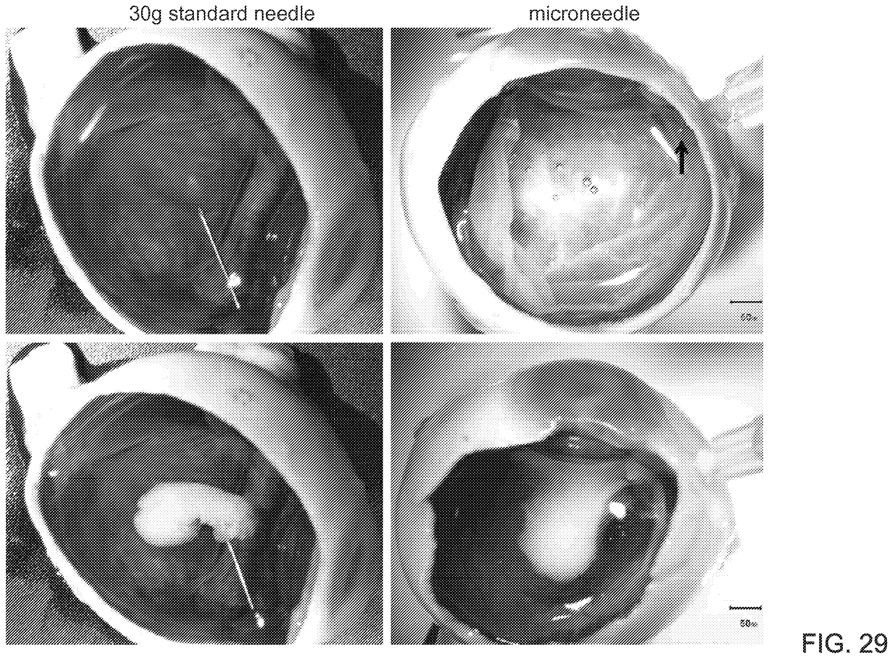

Human cadaver eyes were used to compare 30 gauge standard needle (FIG. 29, left panels) and microneedle (FIG. 29, right panels) injections of triamcinolone. As shown in FIG. 29, the microneedle is inserted into the cadaver eye and extends minimally into the vitreous (FIG. 29, top right). Although the microneedle extends minimally into the vitreous, triamcinolone was still successfully administered into the vitreous (FIG. 29, bottom right).

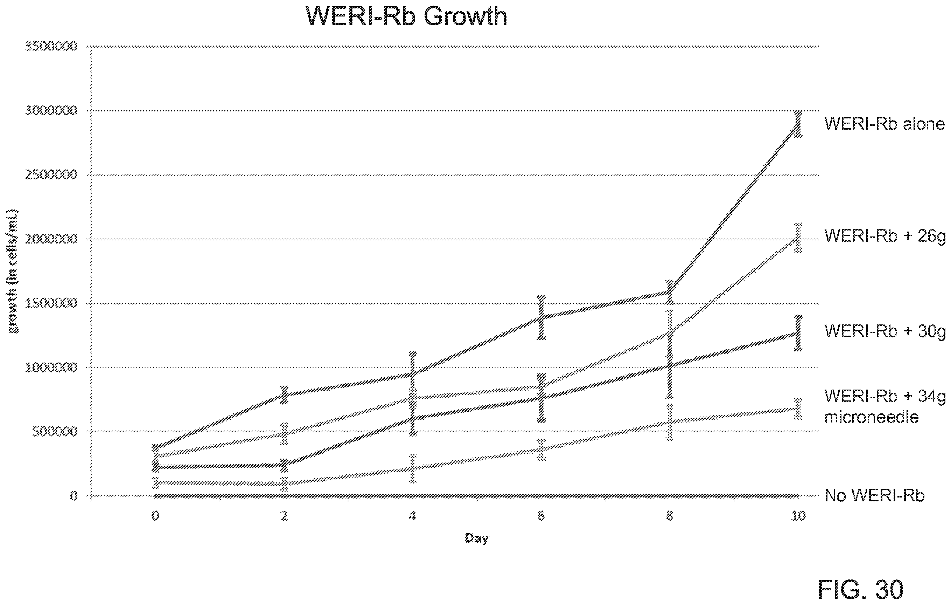

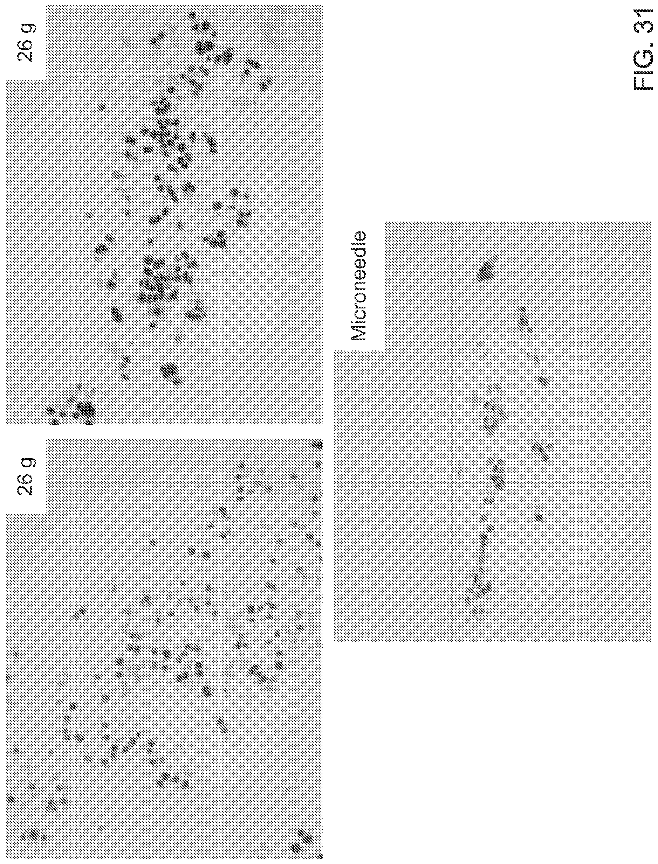

The number of cells that are allowed passage and that survived with the use of 34 gauge microneedles, in comparison to 26 or 30 gauge standard needles, was assessed. WERI human retinoblastoma (WERI-Rb) cells were aspirated and injected into culture media. At days 0, 2, 4, 6, 8, and 10 after aspiration and re-plating, the WERI-Rb cells that were viable were counted in triplicate. As shown in FIG. 30, the use of the 34 gauge microneedle significantly decreased the spread of WERI-Rb cells in comparison to 26 gauge or 30 gauge standard needles. Morphology and concentration of WERI-Rb cells after aspiration and passage in the 26 gauge, 30 gauge, and microneedle groups are shown in FIG. 31.

### Example 2. Inhibition of retinoblastoma cell growth using microneedle with baffle

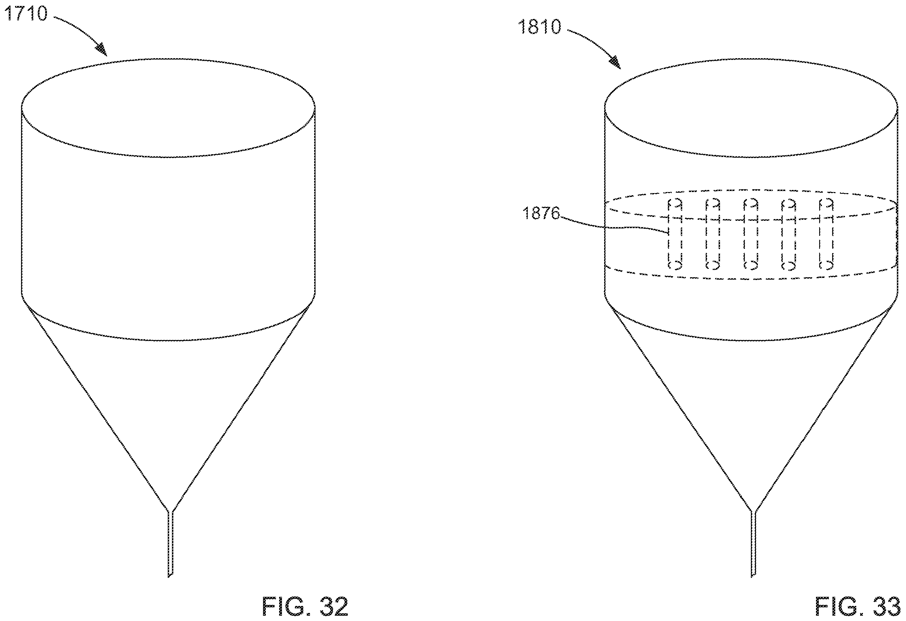

FIG. 32 is an illustration of a microneedle 1710 without a baffle according to an embodiment. FIG. 33 is an illustration of a microneedle 1810 with a baffle 1876 (e.g., inserted into the hub of the microneedle 1810), which creates a chamber that traps aspirated cells. A similar baffled microneedle 1810 was prepared. The baffle 1876 was made from a thin sheet of plastic which cut in the shape of a circle that snugly fit into the hub of the microneedle 1810. Approximately 8 holes of about 50 to150 µms in diameter were made in the baffle 1876 with the 34 gauge microneedle 1810.

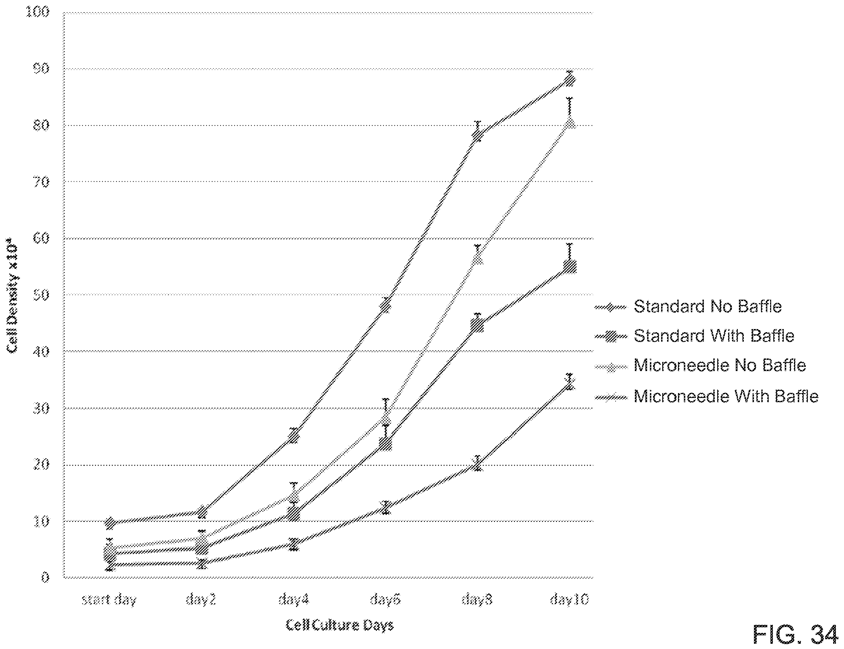

The baffle 1876 was inserted into the microneedle 1810, which allows for drug to pass through via the channels present in the baffle 1876. In order to assess the spread of retinoblastoma cells from needles, human WERI-Rb cells were aspirated and re-plated into 96 well plates using a standard 34 gauge, a standard 34 gauge with a baffle, the microneedle 1710, and the microneedle 1810 with the baffle 1876. The least amount of retinoblastoma cell growth occurred with the microneedle 1810 with the baffle 1876, followed by the 34 gauge needle with baffle, the microneedle 1710, and finally the 34 gauge needle (FIG. 34).

### Example 3. Targeted drug delivery using microneedle

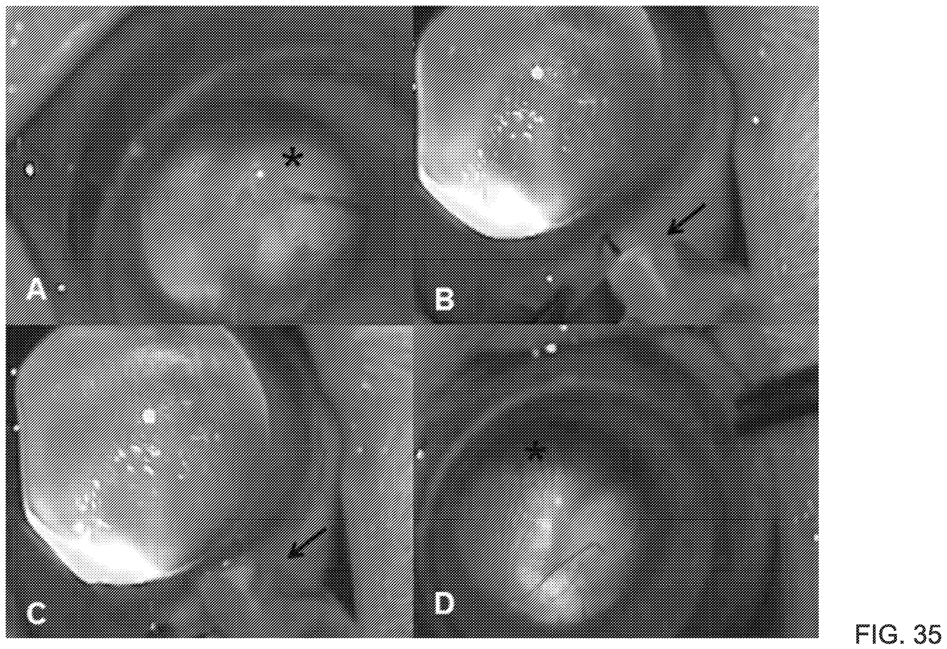

An experiment was conducted in order to assess whether microneedles are suitable for targeted drug delivery to vitreous seeds in a rabbit retinoblastoma model. New Zealand white rabbits received subretinal injections of retinoblastoma cells, which resulted in the establishment of tumors in the subretinal space near the optic nerve and in the vitreous (Kang and Grossniklaus, J Biomed Biotech 2011, Article ID 394730). In this model, similar to human retinoblastoma, vitreous seeds of viable tumor are present (indicated in FIG. 35 by the star in the top left). Topotecan was injected into the eye via a microneedle, which was inserted at the pars plana (indicated in FIG. 35 by the arrow in the top right) to its hub (indicated in FIG. 35 by the arrow in the bottom left). Twenty µg of topotecan was injected weekly for 3 weeks. As shown in FIG. 35 (bottom right), after 3 weekly injections of topotecan with the microneedle, the vitreous seeds have disappeared.

### Example 4. Aspiration of retinoblastoma cells using a microneedle.

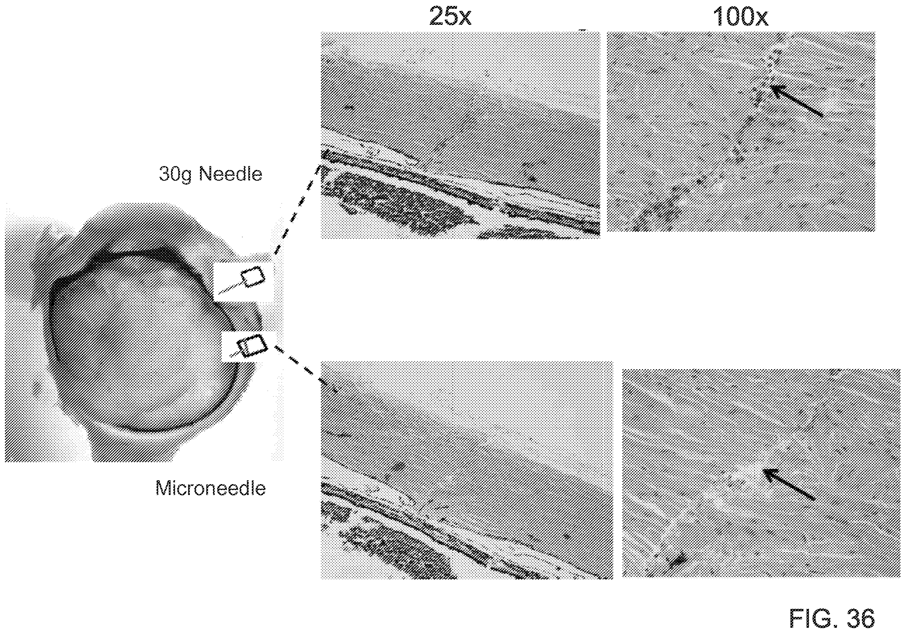

Either a 30 gauge needle or a microneedle with baffle was used to aspirate retinoblastoma tumors in enucleated eyes. An enucleated eye and a schematic depiction of the 30 gauge needle and the microneedle are shown in the left panel of FIG. 36.

Following aspiration, enucleated eyes were prepared and sectioned, and sections were stained with hematoxylin-eosin. As shown in FIG. 36, tumor cells were readily visible within the 30 gauge needle tract in the sclera (FIG. 36, top two panels), whereas tumor cells were not evident within the needle tract in the sclera from the microneedle with baffle (FIG. 36, bottom two panels). Thus, a microneedle with baffle can be used to aspirate retinoblastoma cells without causing tumor cells to be present in the needle tract following aspiration.

### Example 5. Topotecan delivery via a microneedle reduces vitreous seeds and tumor area in a rabbit retinoblastoma model.

New Zealand white rabbits received subretinal injections of retinoblastoma cells in order to establish tumors in the subretinal space near the optic nerve and in the vitreous space, as described above in Example 3. Fifty µL of PBS (control), 5µg/50uL of topotecan ("low dose"), or 10µg/50uL of topotecan ("high dose") were injected once weekly for three weeks into the eye. Injections were conducted via insertion of a microneedle at the pars plana up to the microneedle hub.

In order to enumerate vitreous seeds before and after topotecan treatment, fundus examination was conducted just prior to the first injection and one week after the third injection. Vitreous seeds were graded as a 1 plus (+) if seeds filled less than one third of the vitreous; 2 plus (++) if seeds filled one third to two thirds of the vitreous; and 3 plus (+++) if seeds filled the entire vitreous (i.e., two thirds of the vitreous up to the entire vitreous). Scores of +, ++, and +++ were expressed as scores of 1, 2, and 3, respectively, and the scores for rabbits in each group were averaged. As shown in FIG. 37, both low and high doses of topotecan delivered via microneedle reduced vitreous seed score. The high dose of topotecan delivered via microneedle resulted in a significant reduction in vitreous seeds after weekly treatment.

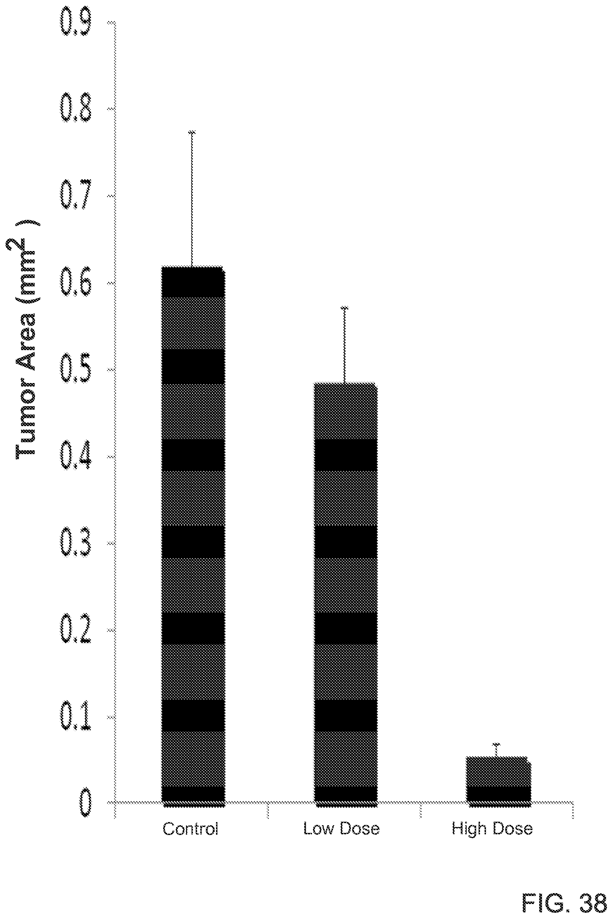

One week after the third injection of topotecan, animals were euthanized and eyes were removed. All enucleated eyes were fixed in 10% formalin, dehydrated in increasing concentrations of alcohol, and cleared in xylene. Serial sections of 8 microns were prepared, and every third slide was stained with hematoxylin-eosin. Five sections with the largest tumor area in each eye were photographed at 40X magnification (DP10; Olympus, Tokyo, Japan). Tumor size was determined with ImageJ software (developed by Wayne Rasband, National Institutes of Health, Bethesda, MD) and expressed as tumor area in mm². As shown in FIG. 38, topotecan injected via a microneedle decreased tumor area. In particular, three weekly treatments of high dose (i.e., 10µg/50uL) topotecan significantly reduced the tumor area in comparison to control treatment or low dose topotecan treatment.

Although the embodiments have been described above as being used with a given set of drug formulations to treat specific ocular diseases, the embodiments described herein can be used with any suitable drug formulation to treat any suitable ocular disease. Nonlimiting examples of ocular diseases include uveitis, glaucoma, diabetic macular edema or retinopathy, macular degeneration, retinoblastoma, and genetic diseases. The methods described herein are particularly useful for the local delivery of drugs that need to be administered to the posterior region of the eye, for example the retinochoroidal tissue, macula, and optic nerve in the posterior segment of the eye. In one embodiment, the delivery methods and devices described herein may be used in gene-based therapy applications. For example, the methods may administer a fluid drug formulation into the suprachoroidal space to deliver select DNA, RNA, or oligonucleotides to targeted ocular tissues.

The microneedles can be used to target delivery to specific tissues or regions within the eye or in neighboring tissue. In various embodiments, the methods may be designed for drug delivery specifically to the sclera, the choroid, the Brach's membrane, the retinal pigment epithelium, the subretinal space, the retina, the macula, the optic disk, the optic nerve, the ciliary body, the trabecular meshwork, the aqueous humor, the vitreous humor, and other ocular tissue or neighboring tissue in need of treatment.

A wide range of drugs may be formulated for delivery to ocular tissues with the present microneedle devices and methods. Moreover, any of the delivery devices and/or methods described herein can involve, include and/or contain any of the drugs described herein. For example, in some embodiments, the cartridge **240** or any other cartridge or medicament container described herein can contain any of the drugs and/or formulations described herein. As used herein, the term "drug" refers to any prophylactic, therapeutic, or diagnostic agent (e.g., a contrast agent). The drug may be selected from suitable proteins, peptides and fragments thereof, which can be naturally occurring, synthesized or recombinantly produced. Representative examples of types of drugs for delivery to ocular tissues include antibodies, anti-viral agents, chemotherapeutic agents (e.g., topoisomerase inhibitors), analgesics, anesthetics, aptamers, antihistamines, anti-inflammatory agents, and anti-neoplastic agents. In one embodiment, the drug is triamcinolone or triamcinolone acetonide.

The term "antibody" is intended to refer broadly to any immunologic binding agent such as IgG, IgM, IgA, IgD and IgE. An antibody can be monoclonal or polyclonal, and in one embodiment, is a humanized antibody. The term "antibody" is also used to refer to any antibody-like molecule that has an antigen binding region, and includes antibody fragments such as Fab', Fab, F(ab')₂, single domain antibodies (DABs), Fv, scFv (single chain Fv), and engineering multivalent antibody fragments such as dibodies, tribodies and multibodies. The techniques for preparing and using various antibody-based constructs and fragments are well known in the art (see, e.g., Antibodies: A Laboratory Manual, Cold Spring Harbor Laboratory, 1988).

Non-limiting examples of specific drugs and classes of drugs include β-adrenoceptor antagonists (e.g., carteolol, cetamolol, betaxolol, levobunolol, metipranolol, timolol), miotics (e.g., pilocarpine, carbachol, physostigmine), sympathomimetics (e.g., adrenaline, dipivefrine), carbonic anhydrase inhibitors (e.g., acetazolamide, dorzolamide), topoisomerase inhibitors (e.g., topotecan, irinotecan, camptothecin, lamellarin D, etoposide, teniposide, doxorubicin, mitoxantrone, amsacrine), prostaglandins, anti-microbial compounds, including anti-bacterials and anti-fungals (e.g., chloramphenicol, chlortetracycline, ciprofloxacin, framycetin, fusidic acid, gentamicin, neomycin, norfloxacin, ofloxacin, polymyxin, propamidine, tetracycline, tobramycin, quinolines), anti-viral compounds (e.g., acyclovir, cidofovir, idoxuridine, interferons), aldose reductase inhibitors, anti-inflammatory and/or anti-allergy compounds (e.g., steroidal compounds such as betamethasone, clobetasone, dexamethasone, fluorometholone, hydrocortisone, prednisolone and non-steroidal compounds such as antazoline, bromfenac, diclofenac, indomethacin, lodoxamide, saprofen, sodium cromoglycate), artificial tear/dry eye therapies, local anesthetics (e.g., amethocaine, lignocaine, oxbuprocaine, proxymetacaine), cyclosporine, diclofenac, urogastrone and growth factors such as epidermal growth factor, mydriatics and cycloplegics, mitomycin C, and collagenase inhibitors and treatments of age-related macular degeneration such as pegagtanib sodium, ranibizumab, aflibercept and bevacizumab.

In one embodiment, the drug is an integrin antagonist, a selectin antagonist, an adhesion molecule antagonist (e.g., intercellular adhesion molecule (ICAM)-1, ICAM-2, ICAM-3, platelet endothelial adhesion molecule (PCAM), vascular cell adhesion molecule (VCAM)), a leukocyte adhesion-inducing cytokine or growth factor antagonist (e.g., tumor necrosis factor-a (TNF-α), interleukin-1β (IL-1β), monocyte chemotatic protein-1 (MCP-1), or a vascular endothelial growth factor (VEGF)), as described in U.S. Patent No. 6,524,581 to Adamis. In some embodiments, a vascular endothelial growth factor (VEGF) inhibitor is administered with one of the microneedles described herein.

In some embodiments, two drugs are delivered by the methods described herein. The compounds may be administered in one formulation, or administered serially, in two separate formulations. For example, both a VEGF inhibitor and VEGF are provided. In some embodiments, the VEGF inhibitor is an antibody, for example a humanized monoclonal antibody. In further embodiments, the VEGF antibody is bevacizumab. In another embodiment, the VEGF inhibitor is ranibizumab, aflibercept or pegaptanib. In still other embodiments, the devices and methods described herein can be used to deliver one or more of the following VEGF antagonists: AL8326, 2C3 antibody, AT001 antibody, HyBEV, bevacizumab (Avastin), ANG3070, APX003 antibody, APX004 antibody, ponatinib (AP24534), BDM-E, VGX100 antibody (VGX100 CIRCADIAN), VGX200 (c-fos induced growth factor monoclonal antibody), VGX300, COSMIX, DLX903/1008 antibody, ENMD2076, Sutent (sunitinib malate), INDUS815C, R84 antibody, KD019, NM3, allogenic mesenchymal precursor cells combined with an anti-VEGF agent or antibody, MGCD265, MG516, VEGF-Receptor kinase inhibitors, MP0260, NT503, anti-DLL4/VEGF bispecific antibody, PAN90806, Palomid 529, BD0801 antibody, XV615, lucitanib (AL3810, E3810), AMG706 (motesanib diphosphate), AAV2-sFLT01, soluble Flt1 receptor, Cediranib (Recentin), AV-951 (Tivozanib, KRN-951), Stivarga (regorafenib), Volasertib (BI6727), CEP11981, KH903, Lenvatinib (E7080), terameprocol (EM1421), ranibizumab (Lucentis), Votrient (pazopanib hydrochloride), PF00337210, PRS050, SP01 (curcumin), Carboxyamidotriazole orotate, hydroxychloroquine, linifanib (ABT869, RG3635), Iluvien (fluocinolone acetonide), ALG1001, AGN150998, DARPin MP0112, AMG386, ponatinib (AP24534), AVA101, Vargatef (nintedanib), BMS690514, KH902, golvatinib (E7050), Afinitor (everolimus), Dovitinib lactate (TKI258, CHIR258), ORA101, ORA102, Axitinib (Inlyta, AG013736), Plitidepsin (Aplidin), Lenvatinib mesylate, PTC299, aflibercept (Zaltrap, Eylea), pegaptanib sodium (Macugen, LI900015), Visudyne (verteporfin), bucillamine (Rimatil, Lamin, Brimani, Lamit, Boomiq), R3 antibody, AT001/r84 antibody, troponin (BLS0597), EG3306, vatalanib (PTK787), Bmab100, GSK2136773, Anti-VEGFR Alterase, Avila, CEP7055, CLT009, ESBA903, HuMax-VEGF antibody, GW654652, HMPL010, GEM220, HYB676, JNJ17029259, TAK593, XtendVEGF antibody, Nova21012, Nova21013, CP564959, Smart Anti-VEGF antibody, AG028262, AG13958, CVX241, SU14813, PRS055, PG501, PG545, PTI101, TG100948, ICS283, XL647, enzastaurin hydrochloride (LY317615), BC194, quinolines, COT601M06.1, COT604M06.2, MabionVEGF, SIR-Spheres coupled to anti-VEGF or VEGF-R antibody, Apatinib (YN968D1), and AL3818. In addition, delivery of a VEGF inhibitor or VEGF antagonist using the microneedle devices and methods disclosed herein may be combined with one or more agents listed herein or with other agents known in the art.

In one embodiment, the devices and methods described herein are used for delivery of an effective amount of a VEGF antagonist to the suprachoroidal space of the eye of a patient in need thereof.

In a further embodiment, the VEGF antagonist is used to treat, prevent and/or ameliorate diabetic macular edema, visual impairment due to diabetic macular edema, diabetic retinopathy, dry eye syndrome (inflammation and corneal tissue damage of dry Eye), neovascular (wet) age-related macular degeneration (AMD)), ocular neovascularization, retinal detachment, a retinal disorder, retinitis pigmentosa, retinal vein occlusion, branch retinal vein occlusion, central retinal vein occlusion, eye cancer, subfoveal neovascular age-related macular degeneration, macular edema, macular edema associated with branch retinal vein occlusion, macular edema following retinal vein occlusion, macular edema with retinal vein occlusion (RVO)

In one another embodiment, the devices and methods described herein are used for delivery of an effective amount of a VEGF antagonist to the suprachoroidal space of the eye of a patient in need thereof. In a further embodiment, the VEGF antagonist is used to treat, prevent and or ameliorate a disease or disorder selected from leukemia, relapsed/refractory leukemia, acute lymphoblastic leukemia, acute myelogenous leukemia, relapsed or refractory acute myeloid leukemia, atopic dermatitis, recurrent or metastatic carcinoma of the urothelium, advanced urothelial carcinoma, blood disorders, myelofibrosis, brain tumor, glioblastoma, glioma, meningioma, cancer, carcinomatous meningitis (neoplastic meningitis), choroidal neovascularization (CNV), subfoveal choroidal neovascularization, chronic lymphocytic leukemia, chronic myelogenous leukemia, refractory chronic myelogenous leukemia, degenerative nerve diseases, neurodegenerative diseases, endometrial cancer, neurofibromatosis type II, head and neck cancer, hematological malignancies, Kaposi's Sarcoma, hepatocellular carcinoma, lung cancer, macular degeneration, age related macular degeneration, exudative age-related macular degeneration, multiple myeloma, relapsed or refractory multiple myeloma, multiple sclerosis, myopia, pathological myopia, neuroendocrine tumor, carcinoid tumor, neuroendocrine tumor, non-Hodgkin's Lymphoma, Diffuse Large B-Cell Lymphoma, corneal graft rejection, osteoarthritis, recurrent symptomatic malignant ascites, peripheral T-cell lymphoma, androgen independent psoriasis, pulmonary fibrosis, idiopathic pulmonary fibrosis, respiratory diseases, rheumatoid arthritis, sarcoma, alveolar soft part sarcoma, soft tissue sarcoma, scleroderma/systemic sclerosis, solid tumors, refractory germ cell tumors, thyroid cancer, differentiated or medullar thyroid cancer, and West Syndrome (Infantile Spasm).

In certain embodiments, the drug delivered to the suprachoroidal space using the devices and methods disclosed herein is rapamycin (Sirolimus, Rapamune). In one embodiment, the devices (e.g., microneedle devices) and methods disclosed herein are used in conjunction with rapamycin to treat, prevent and/or ameliorate a wide range of diseases or disorders including, but not limited to: abdominal neoplasms, acquired immunodeficiency syndrome, acute coronary syndrome, acute lymphoblastic leukemia, acute myelocytic leukemia, acute non-lymphoblastic leukemia, adenocarcinoma, adenoma, adenomyoepithelioma, adnexal diseases, anaplastic astrocytoma, anaplastic large cell lymphoma, anaplastic plasmacytoma, anemia, angina pectoris, angioimmunoblastic lymphadenopathy with dysproteinemia, angiomyolipoma, arterial occlusive diseases, arteriosclerosis, astrocytoma, atherosclerosis, autoimmune diseases, B-cell lymphomas, blood coagulation disorders, blood protein disorders, bone cancer, bone marrow diseases, brain diseases, brain neoplasms, breast beoplasms, bronchial neoplasms, carcinoid syndrome, carcinoid Tumor, carcinoma, squamous cell carcinoma, central nervous system diseases, central nervous system neoplasms, choroid diseases, choroid plexus neoplasms, choroidal neovascularization, choroiditis, chronic lymphocytic leukemia, chronic myeloid leukemia, chronic myelomonocytic leukemia, chronic myeloproliferative disorders, chronic neutrophilic leukemia, clear cell renal cell carcinoma, colonic diseases, colonic neoplasms, colorectal neoplasms, coronary artery disease, coronary disease, coronary Occlusion, coronary restenosis, coronary stenosis, coronary thrombosis, cutaneous T-cell lymphoma, diabetes mellitus, digestive system neoplasms, dry eye syndromes, ear diseases, edema, endocrine gland neoplasms, endocrine system diseases, endometrial neoplasms, Endometrial stromal tumors, Ewing's sarcoma, exanthema, eye neoplasms, fibrosis, follicular lymphoma, gastrointestinal diseases, gastrointestinal neoplasms, genital neoplasms, glioblastoma, glioma, gliosarcoma, graft vs host disease, hematologic diseases, hematologic neoplasms, hemorrhagic disorders, hemostatic disorders, Hodgkin disease, Hodgkin lymphoma, homologous wasting disease, immunoblastic lymphadenopathy, immunologic deficiency syndromes, immunoproliferative disorders, infarction, inflammation, intestinal diseases, intestinal neoplasms, ischemia, kidney cancer, kidney diseases, kidney neoplasms, leukemia, B-Cell, leukemia, lymphoid, liver cancer, liver diseases, lung diseases, lymphatic diseases, lymphoblastic lymphoma, lymphoma, macular degeneration, macular edema, melanoma, mouth neoplasms, multiple myeloma, myelodysplastic syndromes, myelofibrosis, myeloproliferative disorders, neuroectodermal tumors, neuroendocrine tumors, neuroepithelioma, neurofibroma, renal cancer, respiratory tract diseases, retinal degeneration, retinal diseases, retinal neoplasms, retinoblastoma, rhabdomyosarcoma, thoracic neoplasms, uveitis, vascular diseases, Waldenstrom Macroglobulinemia, and wet macular degeneration. In addition, delivery of rapamycin using the microneedle devices and methods disclosed herein may be combined with one or more agents listed herein or with other agents known in the art.

In one embodiment, the drug delivered to ocular tissue, for example the sclera or suprachoroidal space, using the microneedle devices and methods disclosed herein reduces, inhibits, prevents and/or ameliorates inflammation. Examples of drugs that reduce, inhibit, prevent and/or ameliorate inflammation include (but are not limited to): 19AV Agonists, 19GJ agonists, 2MD Analogs, 4SC101, 4SC102, 57-57, 5-HT2 Receptor Antagonist, 64G12, A804598, A967079, AAD2004, AB1010, AB224050, abatacept, Abegrin, Aabevac, AbGn134, AbGn168, Abki, ABN912, ABR215062, ABR224050, Abrammune, Abreva, ABS15, ABS4, ABS6, ABT122, ABT325, ABT494, ABT874, ABT963, ABXIL8, ABXRB2, AC430, Accenetra, Acdeam, ACE772, Acebid, Acebloc, aceclofenac, acetaminophen, chlorzoxazone, serrapeptase, tizanidine hydrochloride, betadex, Aceclogesic Plus, Aceclon, Acecloren, Aceclorism, acecrona, Aceffein, acemetacin, Acenac, Acenterine, Acetal-SP, ibuprofen, Acetyl-G, acetylsalicylate dl-lysine, acetylsalicylic acid, Acicot, Acifine, Acik, Aclocen, Acloflam-P, Aclomore, Aclon, A-CQ, ACS15, actarit, Actemra, Acthelea liofilizado, Actifast, Actimab-B, Actiquim, Actirin, Actis PLUS, activated leukocyte cell adhesion molecule antibody, Acular X, AD452, adalimumab, ADAMTS5 Inhibitor, ADC1001, Adco-Diclofenac, Adco-Indomethacin, Adco-Meloxicam, Adco-Naproxen, Adco-Piroxicam, Adcort, Adco-Sulindac, adenosine triphosphate disodium, AdenosineA2a Receptor Agonist, Adimod, Adinos, Adioct, Adiodol, Adipoplus, adipose derived stem and/or regenerative cells, Adizen, Adpep, Advacan, Advagraf, Advel, Adwiflam, AEB071, Aental, Afenac, Affen Plus, Afiancen, Afinitor, Aflamin, Aflazacort, Aflogen, Afloxan, AFM15, AFM16, AFM17, AFM23, Afpred-Dexa, AFX200, AG011, Agafen, aganirsen, AGI1096, Agidex, AGS010, Agudol, A-Hydrocort, AIK1, AIN457, Airtal, AIT110, AJM300, ajulemic acid, AK106, AL-24-2A1, AL4-1A1, Ala Cort, Alanz, Albumin immune-globulin, alclometasone dipropionate, ALD518, aldesleukin, Aldoderma, alefacept, alemtuzumab, Alequel, Alergolon, Alergosone, Aletraxon, Alfenac, Algason, Algin vek coat, Algioflex, Algirex, Algivin Plus, alicaforsen sodium, Alin, Alinia, Aliviodol, Aliviosin, alkaline phosphatase, ALKS6931, allantoin, Allbupen, Allmol, Allochrysine, allogeneic endothelial cells, allogeneic mesenchymal precursor cells, allogeneic mesenchymal stem cells, alminoprofen, alpha 1 antitrypsin, Alpha 7 nicotinic agonists, alpha amylase, alpha chymotrypsin, alpha fetoprotein, alpha linolenic acid, Alpha-1-antitrypsin, Alpha2Beta1 Integrin Inhibitors, Alphacort, Alphafen, alpha-hexidine, alpha-trypsin, Alphintern, Alpinamed mobility omega 3, Alpoxen, AL-Rev1, Alterase, ALX0061, ALX0761, ALXN1007, ALXN1102, AM3840, AM3876, AMAB, AMAP102, Amason, Ambene, AmbezimG, amcinonide, AME133v, Amecin, Ameloteks, A-Methapred, Amevive, AMG108, AMG139, AMG162, AMG181, AMG191, AMG220, AMG623, AMG674, AMG714, AMG719, AMG729, AMG827, Amidol, amifampridine phosphate, Amifenac, Amimethacin, amiprilose hydrochloride, Amiprofen, Ammophos, Amoflam, AMP110, Ampikyy, Ampion, ampiroxicam, amtolmetin guacil, AMX256, AN6415, ANA004, ANA506, Anabu, Anacen, Anaflam, Anaflex ACI, Anaida, anakinra, Analgen Artritis, Anapan, Anaprox, Anavan, Anax, Anco, andrographis, Aneol, Anergix, Anervax.RA, Anflene, ANG797, Anilixin, Anmerushin, Annexin 1 peptides, annexin A5, Anodyne, Ansaid, Anspirin, Antarene, Anti BST2 antibody, Anti C5a MAb, Anti ILT7 antibody, Anti VLA1 antibody, Anti-alphal 1 antibody, Anti-CD4 802-2, Anti-CD86 Monoclonal Antibody, Anti-chemokine, Anti-DC-SIGN, Anti-HMGB-1 MAb, Anti-IL-18 Mab, Anti-IL-1R MAb, Anti-IL-1R MAb, Anti-IL23 BRISTOL, Anti-inflammatory Peptides, Anti-interleukin 1Beta antibody, Anti-LIGHT antibody, Anti-LIGHT antibody, Anti-MIF Antibody, Anti-MIF Antibody, Anti-miR181a, antioxidant inflammation modulators, Antiphlamine, AntiRAGE MAb, antithrombin III, Anti-TIRC-7 MAb, Anusol-HC, Anyfen, AP105, AP1089, AP1189, AP401, AP501, apazone, APD334, Apentac, APG103, Apidone, apilimod mesylate, Apitac, Apitoxin, Apizel, APN Inhibitor, apo-Azathioprine, Apo-Dexamethasone, ApoE mimetics, ApoFasL, apo-Indomethacin, apo-mefenamic, apo-methotrexate, apo-nabumetone, Apo-Napro-NA, apo-Naproxen, aponidin, apo-Phenylbutazone, apo-Piroxicam, apo-Sulin, Apo-Tenoxicam, apo-Tiaprofenic, Apranax, apremilast, apricoxib, Aprofen, Aprose, Aproxen, APX001 antibody, APX007 antibody, APY0201, AqvoDex, AQX108, AQX1125, AQX131135, AQX140, AQX150, AQX200, AQX356, AQXMN100, AQXMN106, ARA290, Arava, Arcalyst, Arcoxia, Arechin, Arflur, ARG098, ARG301, arginine aescin, arginine deiminase (pegylated), ARGX109 antibody, ARGX110, Arheuma, Aristocort, Aristospan, Ark-AP, ARN4026, Arofen, Aroff EZ, Arolef, Arotal, Arpibru, Arpimune, Arpu Shuangxin, ARQ101, Arrestin SP, Arrox, ARRY162, ARRY371797, ARRY614, ARRY872, ART621, Artamin, Arthfree, Artho Tech, Arthrexin, Arthrispray, Arthrotec, Arthrovas, Artifit, Artigo, Artin, Artinor, Artisid, Artoflex, Artren Hipergel, Artridol, Artrilase, Artrocaptin, Artrodiet, Artrofen, Artropan, Artrosil, Artrosilene, Artrotin, Artrox, Artyflam, Arzerra, AS604850, AS605858, Asacol, ASA-Grindeks, Asazipam, Aseclo, ASF1096, ASF1096, ASK8007, ASKP1240, ASLAN003, Asmo ID, Asonep, ASP015K, ASP2408, ASP2409, Aspagin, Aspeol, Aspicam, Aspirimex, aspirin, AST120, astaxanthin, AstroCort, Aszes, AT002 antibody, AT007, AT008 antibody, AT008 antibody, AT010, AT1001, atacicept, Ataspin, Atepadene, Atgam, ATG-Fresenius, Athrofen, ATI003, atiprimod, ATL1222, ATN103, ATN192, ATR107, Atri, Atrmin, Atrosab antibody, ATX3105, AU801, auranofin, Aurobin, Auropan, Aurothio, aurotioprol, autologous adipose derived regenerative cells, Autonec, Avandia, AVE9897, AVE9940, Avelox, Avent, AVI3378, Avloquin, AVP13546, AVP13748, AVP28225, AVX002, Axcel Diclofenac, Axcel Papain, Axen, AZ17, AZ175, Azacortid, AZA-DR, Azafrine, Azamun, Azanin, Azap, Azapin, Azapren, Azaprin, Azaram, Azasan, azathioprine, AZD0275, AZD0902, AZD2315, AZD5672, AZD6703, AZD7140, AZD8309, AZD8566, AZD9056, Azet, Azintrel, azithromycin, Az-od, Azofit, Azolid, Azoran, Azulene, Azulfidine, Azulfin, B1 antagonists, Baclonet, BAF312, BAFF Inhibitor, Bages, Baily S.P., Baleston, Balsolone, baminercept alfa, bardoxolone methyl, baricitinib, Barotase, Basecam, basiliximab, Baxmune, Baxo, BAY869766, BB2827, BCX34, BCX4208, Becfine, Beclate-C, Beclate-N, Beclolab Q, beclomethasone dipropionate, Beclorhin, Becmet-CG, Begita, Begti, belatacept, belimumab, Belosalic, Bemetson, Ben, Benevat, Benexam, Benflogin, Benisan, Benlysta, Benlysta, benorilate, Benoson, benoxaprofen, Bentol, benzydamine hydrochloride, Benzymin, Beofenac, Berafen, Berinert, Berlofen, Bertanel, Bestamine, Bestofen, Beta Nicip, Betacort, Betacorten G, Betafoam, beta-glucan, Betalar, Beta-M, Betamed, Betamesol, betamethasone, betamethasone dipropionate, betamethasone sodium, betamethasone sodium phosphate, betamethasone valerate, Betane, Betanex, Betapanthen, Betapar, Betapred, Betason, Betasonate, Betasone, Betatrinta, Betaval, Betazon, Betazone, Betesil, Betnecort, Betnesol, Betnovate, Bextra, BFPC13, BFPC18, BFPC21, BFPT6864, BG12, BG9924, BI695500, BI695501, BIA12, Big-Joint-D, BIIB023 antibody, Bi-ksikam, Bingo, BioBee, Bio-Cartilage, Bio-C-Sinkki, Biodexone, Biofenac, Bioreucam, Biosone, Biosporin, BIRB796, Bitnoval, Bitvio, Bivigam, BKT140, BKTP46, BL2030, BL3030, BL4020, BL6040, BL7060, BLI1300, blisibimod, Blokium B12, Blokium Gesic, Blokium, BMS066, BMS345541, BMS470539, BMS561392, BMS566419, BMS582949, BMS587101, BMS817399, BMS936557, BMS945429, BMS-A, BN006, BN007, BNP166, Bonacort, Bonas, bone marrow stromal cell antigen 2 antibody, Bonflex, Bonifen, Boomiq, Borbit, Bosong, BR02001, BR3-FC, Bradykinin B1 Receptor Antagonist, Bredinin, Brexecam, Brexin, Brexodin, briakinumab, Brimani, briobacept, Bristaflam, Britten, Broben, brodalumab, Broen-C, bromelains, Bromelin, Bronax, Bropain, Brosiral, Bruace, Brufadol, Brufen, Brugel, Brukil, Brusil, BT061, BTI9, BTK kinase inhibitors, BTT1023 antibody, BTT1507, bucillamine, Bucillate, Buco Reigis, bucolome, Budenofalk, budesonide, Budex, Bufect, Bufencon, Bukwang Ketoprofen, Bunide, Bunofen, Busilvex, busulfan, Busulfex, Busulipo, Butartrol, Butarut B12, Butasona, Butazolidin, Butesone, Butidiona, BVX10, BXL628, BYM338, B-Zone, C1 esterase inhibitor, C243, c4462, c5997, C5aQb, c7198, c9101, C9709, c9787, CAB101, cadherin 11 antibody, caerulomycin A, CAL263, Calcort, Calmatel, CAM3001, Camelid Antibodies, Camlox, Camola, Campath, Camrox, Camtenam, canakinumab, candida albicans antigen, Candin, cannabidiol, CAP1.1, CAP1.2, CAP2.1, CAP2.2, CAP3.1, CAP3.2, Careram, Carimune, Cariodent, Cartifix, CartiJoint, Cartilago, Cartisafe-DN, Cartishine, Cartivit, Cartril-S, Carudol, CaspaCIDe, CaspaCIDe, Casyn, CAT1004, CAT1902, CAT2200, Cataflam, Cathepsin S inhibitor, Catlep, CB0114, CB2 agonist, CC0478765, CC10004, CC10015, CC1088, CC11050, CC13097, CC15965, CC16057, CC220, CC292, CC401, CC5048, CC509, CC7085, CC930, CCR1 Antagonist, CCR6 Inhibitor, CCR7 Antagonist, CCRL2 antagonist, CCX025, CCX354, CCX634, CD Diclofenac, CD102, CD103 Antibody, CD103 Antibody, CD137 antibody, CD16 antibody, CD18 antibody, CD19 antibody, CD1d Antibody, CD20 antibody, CD200Fc, CD209 antibody, CD24, CD3 antibody, CD30 antibody, CD32A antibody, CD32B antibody, CD4 antibody, CD40 ligand, CD44 antibody, CD64 antibody, CDC839, CDC998, CDIM4, CDIM9, CDK9-Inhibitor, CDP146, CDP323, CDP484, CDP6038, CDP870, CDX1135, CDX301, CE224535, Ceanel, Cebedex, Cebutid, Ceclonac, Ceex, CEL2000, Celact, Celbexx, Celcox, Celebiox, Celebrex, Celebrin, Celecox, celecoxib, Celedol, Celestone, Celevex, Celex, CELG4, Cell adhesion molecule antagonists, CellCept, Cellmune, Celosti, Celoxib, Celprot, Celudex, cenicriviroc mesylate, cenplacel-1, CEP11004, CEP37247, CEP37248, Cephyr, Ceprofen, Certican, certolizumab pegol, Cetofenid, Cetoprofeno, cetylpyridinium chloride, CF101, CF402, CF502, CG57008, CGEN15001, CGEN15021, CGEN15051, CGEN15091, CGEN25017, CGEN25068, CGEN40, CGEN54, CGEN768, CGEN855, CGI1746, CGI560, CGI676, Cgtx-Peptides, CH1504, CH4051, CH4446, chaperonin 10, chemokine C-C motif ligand 2, chemokine C-C motif ligand 2 antibody, chemokine C-C motif ligand 5 antibody, chemokine C-C motif receptor 2 antibody, chemokine C-C motif receptor 4 antibody, chemokine C-X-C motif ligand 10 antibody, chemokine C-X-C motif ligand 12 aptamer, Chemotaxis Inhibitor, Chillmetacin, chitinase 3-like 1, Chlocodemin, Chloquin, chlorhexidine gluconate, chloroquine phosphate, choline magnesium trisalicylate, chondroitin sulfate, Chondroscart, CHR3620, CHR4432, CHR5154, Chrysalin, Chuanxinlian, Chymapra, Chymotase, chymotrypsin, Chytmutrip, CI202, CI302, Cicloderm-C, Ciclopren, Cicporal, Cilamin, Cimzia, cinchophen, cinmetacin, cinnoxicam, Cinoderm, Cinolone-S, Cinryze, Cipcorlin, cipemastat, Cipol-N, Cipridanol, Cipzen, Citax F, Citogan, Citoken T, Civamide, CJ042794, CJ14877, c-Kit monoclonal antibody, cladribine, Clafen, Clanza, Claversal, clazakizumab, Clearoid, Clease, Clevegen, Clevian, Clidol, Clindac, Clinoril, Cliptol, Clobenate, Clobequad, clobetasol butyrate, clobetasol propionate, Clodol, clofarabine, Clofen, Clofenal LP, Clolar, Clonac, Clongamma, clonixin lysine, Clotasoce, Clovacort, Clovana, Cloxin, CLT001, CLT008, C-MAF Inhibitor, CMPX1023, Cnac, CNDO201, CNI1493, CNTO136, CNTO148, CNTO1959, Cobefen, CoBenCoDerm, Cobix, Cofenac, Cofenac, COG241, COL179, colchicine, Colchicum Dispert, Colchimax, Colcibra, Coledes A, Colesol, Colifoam, Colirest, collagen, type V, Comcort, complement component (3b/4b) receptor 1, Complement Component C1s Inhibitors, complement component C3, complement factor 5a receptor antibody, complement factor 5a receptor antibody, complement factor D antibody, Condrosulf, Condrotec, Condrothin, conestat alfa, connective tissue growth factor antibody, Coolpan, Copaxone, Copiron, Cordefla, Corhydron, Cort S, Cortan, Cortate, Cort-Dome, Cortecetine, Cortef, Corteroid, Corticap, Corticas, Cortic-DS, corticotropin, Cortiderm, Cortidex, Cortiflam, Cortinet M, Cortinil, Cortipyren B, Cortiran, Cortis, Cortisolu, cortisone acetate, Cortival, Cortone acetate, Cortopin, Cortoral, Cortril, Cortypiren, Cosamine, Cosone, cosyntropin, COT Kinase Inhibitor, Cotilam, Cotrisone, Cotson, Covox, Cox B, COX-2/5-LO Inhibitors, Coxeton, Coxflam, Coxicam, Coxitor, Coxtral, Coxypar, CP195543, CP412245, CP424174, CP461, CP629933, CP690550, CP751871, CPSI2364, C-quin, CR039, CR074, CR106, CRA102, CRAC channel inhibitor, CRACM Ion Channel Inhibitor, Cratisone, CRB15, CRC4273, CRC4342, C-reactive protein 2-methoxyethyl phosphorothioate oligonucleotide, CreaVax-RA, CRH modulators, critic-aid, Crocam, Crohnsvax, Cromoglycic acid, cromolyn sodium, Cronocorteroid, Cronodicasone, CRTX803, CRx119, CRx139, CRx150, CS502, CS670, CS706, CSF1R Kinase Inhibitors, CSL324, CSL718, CSL742, CT112, CT1501R, CT200, CT2008, CT2009, CT3, CT335, CT340, CT5357, CT637, CTP05, CTP10, CT-P13, CTP17, Cuprenil, Cuprimine, Cuprindo, Cupripen, Curaquin, Cutfen, CWF0808, CWP271, CX1020, CX1030, CX1040, CX5011, Cx611, Cx621, Cx911, CXC chemokine receptor 4 antibody, CXCL13 antibodies, CXCR3 antagonists, CXCR4 antagonist, Cyathus 1104 B, Cyclo-2, Cyclocort, cyclooxygenase-2 inhibitor, cyclophosphamide, Cyclorine, Cyclosporin A Prodrug, Cyclosporin analogue A, cyclosporine, Cyrevia, Cyrin CLARIS, CYT007TNFQb, CYT013IL1bQb, CYT015IL17Qb, CYT020TNFQb, CYT107, CYT387, CYT99007, cytokine inhibitors, Cytopan, Cytoreg, CZC24832, D1927, D9421C, daclizumab, danazol, Danilase, Dantes, Danzen, dapsone, Dase-D, Daypro, Daypro Alta, Dayrun, Dazen, DB295, DBTP2, D-Cort, DD1, DD3, DE096, DE098, Debio0406, Debio0512, Debio0615, Debio0618, Debio1036, Decaderm, Decadrale, Decadron, Decadronal, Decalon, Decan, Decason, Decdan, Decilone, Declophen, Decopen, Decorex, Decorten, Dedema, Dedron, Deexa, Defcort, De-flam, Deflamat, Deflan, Deflanil, Deflaren, Deflaz, deflazacort, Defnac, Defnalone, Defnil, Defosalic, Defsure, Defza, Dehydrocortison, Dekort, Delagil, delcasertib, delmitide, Delphicort, Deltacorsolone, Deltacortril, Deltafluorene, Deltasolone, Deltasone, Deltastab, Deltonin, Demarin, Demisone, Denebola, denileukin diftitox, denosumab, Denzo, Depocortin, Depo-medrol, Depomethotrexate, Depopred, Deposet, Depyrin, Derinase, Dermol, Dermolar, Dermonate, Dermosone, Dersone, Desketo, desonide, desoxycorticosterone acetate, Deswon, Dexa, Dexabene, Dexacip, Dexacort, Dexacortisone, Dexacotisil, Dexadic, Dexadrin, Dexadron, Dexafar, Dexahil, Dexalab, Dexalaf, Dexalet, Dexalgen, Dexallion, Dexalocal, Dexalone, Dexa-M, Dexamecortin, Dexamed, Dexamedis, Dexameral, Dexameta, Dexamethasone, dexamethasone acetate, dexamethasone palmitate, dexamethasone phosphate, dexamethasone sodium metasulfobenzoate, dexamethasone sodium phosphate, Dexamine, Dexapanthen, Dexa-S, Dexason, Dexatab, Dexatopic, Dexaval, Dexaven, Dexazolidin, Dexazona, Dexazone, Dexcor, Dexibu, dexibuprofen, Dexico, Dexifen, Deximune, dexketoprofen, dexketoprofen trometamol, Dexmark, Dexomet, Dexon I, Dexonalin, Dexonex, Dexony, Dexoptifen, Dexpin, Dextan-Plus, dextran sulfate, Dezacor, Dfz, diacerein, Diannexin, Diastone, Dicarol, Dicasone, Dicknol, Diclo, Diclobon, Diclobonse, Diclobonzox, Diclofast, Diclofen, diclofenac, diclofenac beta-dimethylaminoethanol, diclofenac deanol, diclofenac diethylamine, diclofenac epolamine, diclofenac potassium, diclofenac resinate, diclofenac sodium, Diclogen AGIO, Diclogen Plus, Diclokim, Diclomed, Diclo-NA, Diclonac, Dicloramin, Dicloran, Dicloreum, Diclorism, Diclotec, Diclovit, Diclowal, Diclozem, Dico P, Dicofen, Dicoliv, Dicorsone, Dicron, Dicser, Difena, Diffutab, diflunisal, dilmapimod, Dilora, dimethyl sulfone, Dinac, D-Indomethacin, Dioxaflex Protect, Dipagesic, Dipenopen, Dipexin, Dipro AS, Diprobeta, Diprobetasone, Diproklenat, Dipromet, Dipronova, Diprosone, Diprovate, Diproxen, Disarmin, Diser, Disopain, Dispain, Dispercam, Distamine, Dizox, DLT303, DLT404, DM199, DM99, DMI9523, dnaJP1, DNX02070, DNX04042, DNX2000, DNX4000, docosanol, Docz-6, Dolamide, Dolaren, Dolchis, Dolex, Dolflam, Dolfre, Dolgit, Dolmax, Dolmina, Dolo Ketazon, Dolobest, Dolobid, Doloc, Dolocam, Dolocartigen, Dolofit, Dolokind, Dolomed, Dolonac, Dolonex, Dolotren, Dolozen, Dolquine, Dom0100, Dom0400, Dom0800, Domet, Dometon, Dominadol, Dongipap, Donica, Dontisanin, doramapimod, Dorixina Relax, Dormelox, Dorzine Plus, Doxatar, Doxtran, DP NEC, DP4577, DP50, DP6221, D-Penamine, DPIV/APN Inhibitors, DR1 Inhibitors, DR4 Inhibitors, DRA161, DRA162, Drenex, DRF4848, DRL15725, Drossadin, DSP, Duexis, Duo-Decadron, Duoflex, Duonase, DV1079, DV1179, DWJ425, DWP422, Dymol, DYN15, Dynapar, Dysmen, E5090, E6070, Easy Dayz, Ebetrexat, EBI007, EC0286, EC0565, EC0746, Ecax, echinacea purpurea extrack, EC-Naprosyn, Econac, Ecosprin 300, Ecosprin 300, Ecridoxan, eculizumab, Edecam, efalizumab, Efcortesol, Effigel, Eflagen, Efridol, EGFR Antibody, EGS21, eIF5A1 siRNA, Ekarzin, elafin, Eldoflam, Elidel, Eliflam, Elisone, Elmes, Elmetacin, ELND001, ELND004, elocalcitol, Elocom, elsibucol, Emanzen, Emcort, Emifen, Emifenac, emorfazone, Empynase, emricasan, Emtor, Enable, Enbrel, Enceid, EncorStat, Encortolon, Encorton, Endase, Endogesic, Endoxan, Enkorten, Ensera, Entocort, Enzylan, Epanova, Eparang, Epatec, Epicotil, epidermal growth factor receptor 2 antibody, epidermal growth factor receptor antibody, Epidixone, Epidron, Epiklin, EPPA1, epratuzumab, EquiO, Erac, Erazon, ERB041, ERB196, Erdon, EryDex, escherichia coli enterotoxin B subunit, Escin, E-Selectin Antagonists, Esfenac, ESN603, esonarimod, Esprofen, estetrol, Estopein, Estrogen Receptor beta agonist, etanercept, etaracizumab, ETC001, ethanol propolis extrack, ETI511, etiprednol dicloacetate, Etodin, Etodine, Etodol, etodolac, Etody, etofenamate, Etol Fort, Etolac, Etopin, etoricoxib, Etorix, Etosafe, Etova, Etozox, Etura, Eucob, Eufans, eukaryotic translation initiation factor 5A oligonucleotide, Eunac, Eurocox, Eurogesic, everolimus, Evinopon, EVT401, Exaflam, EXEL9953, Exicort, Expen, Extra Feverlet, Extrapan, Extrauma, Exudase, F16, F991, Falcam, Falcol, Falzy, Farbovil, Farcomethacin, Farnerate, Farnezone, Farnezone, Farotrin, fas antibody, Fastflam, FasTRACK, Fastum, Fauldmetro, FcgammaRIA antibody, FE301, Febrofen, Febrofid, felbinac, Feldene, Feldex, Feloran, Felxicam, Fenac, Fenacop, Fenadol, Fenaflan, Fenamic, Fenaren, Fenaton, Fenbid, fenbufen, Fengshi Gutong, Fenicort, Fenopine, fenoprofen calcium, Fenopron, Fenris, Fensupp, Fenxicam, fepradinol, Ferovisc, Feverlet, fezakinumab, FG3019, FHT401, FHTCT4, FID114657, figitumumab, Filexi, filgrastim, Fillase, Final, Findoxin, fingolimod hydrochloride, firategrast, Firdapse, Fisiodar, Fivasa, FK778, Flacoxto, Fladalgin, Flagon, Flamar, Flamcid, Flamfort, Flamide, Flaminase, Flamirex Gesic, Flanid, Flanzen, Flaren, Flaren, Flash Act, Flavonoid Anti-inflammatory Molecule, Flebogamma DIF, Flenac, Flex, Flexafen 400, Flexi, Flexidol, Flexium, Flexon, Flexono, Flogene, Flogiatrin B12, Flogomin, Flogoral, Flogosan, Flogoter, Flo-Pred, Flosteron, Flotrip Forte, Flt3 inhibitors, fluasterone, Flucam, Flucinar, fludrocortisone acetate, flufenamate aluminum, flumethasone, Flumidon, flunixin, fluocinolone, fluocinolone acetonide, fluocinonide, fluocortolone, Fluonid, fluorometholone, Flur, flurbiprofen, Fluribec, Flurometholone, Flutal, fluticasone, fluticasone propionate, Flutizone, Fluzone, FM101 antibody, fms-related tyrosine kinase 1 antibody, Folitrax, fontolizumab, formic acid, Fortecortin, Fospeg, fostamatinib disodium, FP1069, FP13XX, FPA008, FPA031, FPT025, FR104, FR167653, Framebin, Frime, Froben, Frolix, FROUNT Inhibitors, Fubifen PAP, Fucole ibuprofen, Fulamotol, Fulpen, Fungifin, Furotalgin, fusidate sodium, FX002, FX141L, FX201, FX300, FX87L, Galectin modulators, gallium maltolate, Gamimune N, Gammagard, Gamma-I.V., GammaQuin, Gamma-Venin, Gamunex, Garzen, Gaspirin, Gattex, GBR500, GBR500 antibody, GBT009, G-CSF, GED0301, GED0414, Gefenec, Gelofen, Genepril, Gengraf, Genimune, Geniquin, Genotropin, Genz29155, Gerbin, Gerbin, gevokizumab, GF01564600, Gilenia, Gilenya, givinostat, GL0050, GL2045, glatiramer acetate, Globulin, Glortho Forte, Glovalox, Glovenin-I, GLPG0259, GLPG0555, GLPG0634, GLPG0778, GLPG0974, Gluco, Glucocerin, glucosamine, glucosamine hydrochloride, glucosamine sulfate, Glucotin, Gludex, Glutilage, GLY079, GLY145, Glycanic, Glycefort up, Glygesic, Glysopep, GMCSF Antibody, GMI1010, GMI1011, GMI1043, GMR321, GN4001, Goanna Salve, Goflex, gold sodium thiomalate, golimumab, GP2013, GPCR modulator, GPR15 Antagonist, GPR183 antagonist, GPR32 antagonist, GPR83 antagonist, G-protein Coupled Receptor Antagonists, Graceptor, Graftac, granulocyte colony-stimulating factor antibody, granulocyte-macrophage colony-stimulating factor antibody, Gravx, GRC4039, Grelyse, GS101, GS9973, GSC100, GSK1605786, GSK1827771, GSK2136525, GSK2941266, GSK315234, GSK681323, GT146, GT442, Gucixiaotong, Gufisera, Gupisone, gusperimus hydrochloride, GW274150, GW3333, GW406381, GW856553, GWB78, GXP04, Gynestrel, Haloart, halopredone acetate, Haloxin, HANALL, Hanall Soludacortin, Havisco, Hawon Bucillamin, HB802, HC31496, HCQ 200, HD104, HD203, HD205, HDAC inhibitor, HE2500, HE3177, HE3413, Hecoria, Hectomitacin, Hefasolon, Helen, Helenil, HemaMax, Hematom, hematopoietic stem cells, Hematrol, Hemner, Hemril, heparinoid, Heptax, HER2 Antibody, Herponil, hESC Derived Dendritic Cells, hESC Derived Hematopoietic stem cells, Hespercorbin, Hexacorton, Hexadrol, hexetidine, Hexoderm, Hexoderm Salic, HF0220, HF1020, HFT-401, hG-CSFR ED Fc, Hiberna, high mobility group box 1 antibody, Hiloneed, Hinocam, hirudin, Hirudoid, Hison, Histamine H4 Receptor Antagonist, Hitenercept, Hizentra, HL036, HL161, HMPL001, HMPL004, HMPL004, HMPL011, HMPL342, HMPL692, honey bee venom, Hongqiang, Hotemin, HPH116, HTI101, HuCAL Antibody, Human adipose mesenchymal stem cells, anti-MHC class II monoclonal antibody, Human Immunoglobulin, Human Placenta Tissue Hydrolysate, HuMaxCD4, HuMax-TAC, Humetone, Humicade, Humira, Huons Betamethasone sodium phosphate, Huons dexamethasone sodium phosphate, Huons Piroxicam, Huons Talniflumate, Hurofen, Huruma, Huvap, HuZAF, HX02, Hyalogel, hyaluronate sodium, hyaluronic acid, hyaluronidase, Hyaron, Hycocin, Hycort, Hy-Cortisone, hydrocortisone, hydrocortisone acetate, hydrocortisone butyrate, hydrocortisone hemisuccinate, hydrocortisone sodium phosphate, hydrocortisone sodium succinate, Hydrocortistab, Hydrocortone, Hydrolin, Hydroquine, Hydro-Rx, Hydrosone HIKMA, hydroxychloroquine, hydroxychloroquine sulfate, Hylase Dessau, HyMEX, Hypen, HyQ, Hysonate, HZN602, I.M.75, IAP Inhibitors, Ibalgin, Ibalgin, Ibex, ibrutinib, IBsolvMIR, Ibu, Ibucon, Ibudolor, Ibufen, Ibuflam, Ibuflex, Ibugesic, Ibu-Hepa, Ibukim, Ibumal, Ibunal, Ibupental, Ibupril, Ibuprof, ibuprofen, Ibuscent, Ibusoft, Ibusuki Penjeong, Ibususpen, Ibutard, Ibutop, Ibutop, Ibutrex, IC487892, ichthammol, ICRAC Blocker, IDEC131, IDECCE9.1, Ides, Idicin, Idizone, IDN6556, Idomethine, IDR1, Idyl SR, Ifen, iguratimod, IK6002, IKK-beta inhibitor, IL17 Antagonist, IL-17 Inhibitor, IL-17RC, IL18, II1Hy1, IL1R1, IL-23 Adnectin, IL23 Inhibitor, IL23 Receptor Antagonist, IL-31 mAb, IL-6 Inhibitor, IL6Qb, Ilacox, Ilaris, ilodecakin, ILV094, ILV095, Imaxetil, IMD0560, IMD2560, Imesel Plus, Iminoral, Immodin, IMMU103, IMMU106, Immucept, Immufine, Immunex Syrup, immunoglobulin, immunoglobulin G, Immunoprin, ImmunoRel, Immurin, IMO8400, IMP731 antibody, Implanta, Imunocell, Imuran, Imurek, Imusafe, Imusporin, Imutrex, IN0701, Inal, INCB039110, INCB18424, INCB28050, INCB3284, INCB3344, Indexon, Indic, Indo, Indo-A, Indobid, Indo-Bros, Indocaf, Indocarsil, Indocid, Indocin, Indomehotpas, Indomen, Indomet, Indometacin, indomethacin, Indomethasone, Indometin, Indomin, Indopal, Indoron, Indotroxin, INDUS830, INDUS83030, Infladase, Inflamac, Inflammasome inhibitor, Inflavis, Inflaxen, Inflectra, infliximab, Ingalipt, Inicox dp, Inmecin, Inmunoartro, Innamit, InnoD06006, INO7997, Inocin, Inoten, Inovan, Inpra, Inside Pap, Insider-P, Instacyl, Instracool, Intafenac, Intaflam, Inteban, Inteban Spansule, integrin, alpha 1 antibody, integrin, alpha 2 antibody, Intenurse, interferon alfa, interferon beta-la, interferon gamma, interferon gamma antibody, Interking, interleukin 1 Hy1, interleukin 1 antibody, interleukin 1 receptor antibody, interleukin 1, beta antibody, interleukin 10, interleukin 10 antibody, interleukin 12, interleukin 12 antibody, interleukin 13 antibody, interleukin 15 antibody, interleukin 17 antibody, interleukin 17 receptor C, interleukin 18, interleukin 18 binding protein, interleukin 18 antibody, interleukin 2 receptor, alpha antibody, interleukin 20 antibody, Interleukin 21 mAb, interleukin 23 aptamer, interleukin 31 antibody, interleukin 34, Interleukin 6 Inhibitor, interleukin 6 antibody, interleukin 6 receptor antibody, interleukin 7, interleukin 7 receptor antibody, interleukin 8, interleukin 8 antibody, interleukin-18 antibody, Intidrol, Intradex, Intragam P, Intragesic, Intraglobin F, Intratect, Inzel, Iomab B, IOR-T3, IP751, IPH2201, IPH2301, IPH24, IPH33, IPI145, Ipocort, IPP201007, I-Profen, Iprox, Ipson, Iputon, IRAK4 Inhibitor, Iremod, Irtonpyson, IRX3, IRX5183, ISA247, ISIS104838, ISIS2302, ISISCRPRx, Ismafron, IsoQC inhibitor, Isox, ITF2357, Iveegam EN, Ivepred, IVIG-SN, IW001, Izilox, J607Y, J775Y, JAK Inhibitor, JAK3 inhibitor, JAK3 kinase inhibitor, JI3292, JI4135, Jinan Lida, JNJ10329670, JNJ18003414, JNJ26528398, JNJ27390467, JNJ28838017, JNJ31001958, JNJ38518168, JNJ39758979, JNJ40346527, JNJ7777120, JNT-Plus, Joflam, Joint Glucosamin, Jointec, Jointstem, Joinup, JPE1375, JSM10292, JSM7717, JSM8757, JTE051, JTE052, JTE522, JTE607, Jusgo, K412, K832, Kaflam, KAHR101, KAHR102, KAI9803, Kalymin, Kam Predsol, Kameton, KANAb071, Kappaproct, KAR2581, KAR3000, KAR3166, KAR4000, KAR4139, KAR4141, KB002, KB003, KD7332, KE298, keliximab, Kemanat, Kemrox, Kenacort, Kenalog, Kenaxir, Kenketsu Venoglobulin-IH, Keplat, Ketalgipan, Keto Pine, Keto, Ketobos, Ketofan, Ketofen, Ketolgan, Ketonal, Ketoplus Kata Plasma, ketoprofen, Ketores, Ketorin, ketorolac, ketorolac tromethamine, Ketoselect, Ketotop, Ketovail, Ketricin, Ketroc, Ketum, Keyi, Keyven, KF24345, K-Fenac, K-Fenak, K-Gesic, Kifadene, Kilcort, Kildrol, KIM127, Kimotab, Kinase Inhibitor 4SC, Kinase N, Kincort, Kindorase, Kineret, Kineto, Kitadol, Kitex, Kitolac, KLK1 Inhibitor, Klofen-L, Klotaren, KLS-40or, KLS-40ra, KM277, Knavon, Kodolo orabase, Kohakusanin, Koide, Koidexa, Kolbet, Konac, Kondro, Kondromin, Konshien, Kontab, Kordexa, Kosa, Kotase, KPE06001, KRP107, KRP203, KRX211, KRX252, KSB302, K-Sep, Kv 1.3 Blocker, Kv1.3 4SC, Kv1.3 inhibitor, KVK702, Kynol, L156602, Labizone, Labohydro, Labopen, Lacoxa, Lamin, Lamit, Lanfetil, laquinimod, larazotide acetate, LAS186323, LAS187247, LAS41002, Laticort, LBEC0101, LCP3301, LCP-Siro, LCP-Tacro, LCsA, LDP392, Leap-S, Ledercort, Lederfen, Lederlon, Lederspan, Lefenine, leflunomide, Leflux, Lefno, Lefra, Leftose, Lefumide, Lefunodin, Lefva, lenalidomide, lenercept, LentiRA, LEO15520, Leodase, Leukine, Leukocyte function-associated antigen-1 antagonist, leukocyte immunoglobulin-like receptor, subfamily A, member 4 antibody, Leukothera, leuprolide acetate, levalbuterol, levomenthol, LFA-1 Antagonist, LFA451, LFA703, LFA878, LG106, LG267 Inhibitors, LG688 Inhibitors, LGD5552, Li Life, LidaMantle, Lidex, lidocaine, lidocaine hydrochloride, Lignocaine hydrochloride, LIM0723, LIM5310, Limethason, Limus, Limustin, Lindac, Linfonex, Linola acute, Lipcy, lisofylline, Listran, Liver X Receptor modulator, Lizak, LJP1207, LJP920, Lobafen, Lobu, Locafluo, Localyn, Locaseptil-Neo, Locpren, Lodine, Lodotra, Lofedic, Loflam, Lofnac, Lolcam, Lonac, lonazolac calcium, Loprofen, Loracort, Lorcam, Lorfenamin, Lorinden Lotio, Lorncrat, lornoxicam, Lorox, losmapimod, loteprednol etabonate, Loteprednol, Lotirac, Low Molecular Ganoderma Lucidum Polysaccharide, Loxafen, Loxfenine, Loxicam, Loxofen, Loxonal, Loxonin, loxoprofen sodium, Loxoron, LP183A1, LP183A2, LP204A1, LPCN1019, LT1942, LT1964, LTNS101, LTNS103, LTNS106, LTNS108, LTS1115, LTZMP001, Lubor, lumiracoxib, Lumitect, LX2311, LX2931, LX2932, LY2127399, LY2189102, LY2439821, LY294002, LY3009104, LY309887, LY333013, lymphocyte activation gene 3 antibody, Lymphoglobuline, Lyser, lysine aspirin, Lysobact, Lysoflam, Lysozyme hydrochloride, M3000, M834, M923, mAb hG-CSF, MABP1, macrophage migration inhibitory factor antibody, Maitongna, Majamil prolongatum, major histocompatibility complex class II DR antibody, major histocompatibility complex class II antibody, Malidens, Malival, mannan-binding lectin, mannan-binding lectin-associated serine protease-2 antibody, MapKap Kinase 2 Inhibitor, maraviroc, Marlex, masitinib, Maso, MASP2 antibody, MAT304, Matrix Metalloprotease Inhibitor, mavrilimumab, Maxiflam, Maxilase, Maximus, Maxisona, Maxius, Maxpro, Maxrel, Maxsulid, Maxy12, Maxy30, MAXY4, Maxy735, Maxy740, Mayfenamic, MB11040, MBPY003b, MCAF5352A, McCam, McRofy, MCS18, MD707, MDAM, MDcort, MDR06155, MDT012, Mebicam, Mebuton, meclofenamate sodium, Meclophen, Mecox, Medacomb, Medafen, Medamol, Medesone, MEDI2070, MEDI5117, MEDI541, MEDI552, MEDI571, Medicox, Medifen, Medisolu, Medixon, Mednisol, Medrol, Medrolon, medroxyprogesterone acetate, Mefalgin, mefenamic acid, Mefenix, Mefentan, Meflen, Mefnetra forte, Meftagesic-DT, Meftal, Megakaryocyte Growth and Development Factor, Megaspas, Megaster, megestrol acetate, Meite, Meksun, Melbrex, Melcam, Melcam, Melflam, Melic, Melica, Melix, Melocam, Melocox, Mel-One, Meloprol, Melosteral, Melox, Meloxan, Meloxcam, Meloxic, Meloxicam, Meloxifen, Meloxin, Meloxiv, Melpred, Melpros, Melurjin, Menamin, Menisone, Menthomketo, Menthoneurin, Mentocin, Mepa, Mepharen, meprednisone, Mepresso, Mepsolone, mercaptopurine, Mervan, Mesadoron, mesalamine, Mesasal, Mesatec, Mesenchymal Precursor Cells, mesenchymal stem cell, Mesipol, Mesren, Mesulan, Mesulid, Metacin, Metadaxan, Metaflex, Metalcaptase, metalloenzyme inhibitors, Metapred, Metax, Metaz, Meted, Metedic, Methacin, Methaderm, Methasone, Methotrax, methotrexate, methotrexate sodium, Methpred, Methyl prednisolone acetate, methyl salicylate, methyl sulphonyl methane, Methylon, Methylpred, methylprednisolone, methylprednisolone acetate, methylprednisolone sodium succinate, methylprednisolone succinate, Methylprednisolone, Methysol, Metindol, Metoart, Metoject, Metolate, Metoral, Metosyn, Metotab, Metracin, Metrex, metronidazole, Metypred, Mevamox, Mevedal, Mevilox, Mevin SR, Mexilal, Mexpharm, Mext, Mextran, MF280, M-FasL, MHC class II beta chain peptide, Micar, Miclofen, Miclofenac, Micofenolato Mofetil, Micosone, Microdase, microRNA 181a-2 oligonucleotide, MIF Inhibitors, MIFQb, MIKA-Ketoprofen, Mikametan, milodistim, Miltax, Minafen, Minalfen, Minalfene, Minesulin, Minocort, Mioflex, Miolox, Miprofen, Miridacin, Mirloks, Misoclo, Misofenac, MISTB03, MISTB04, Mitilor, mizoribine, MK0359, MK0812, MK0873, MK2 Inhibitors, MK50, MK8457, MK8808, MKC204, MLN0002, MLN0415, MLN1202, MLN273, MLN3126, MLN3701, MLN3897, MLNM002, MM093, MM7XX, MN8001, Mobic, Mobicam, Mobicox, Mobifen Plus, Mobilat, Mobitil, Mocox, Modigraf, Modrasone, Modulin, Mofecept, Mofetyl, mofezolac sodium, Mofilet, Molace, molgramostim, Molslide, Momekin, Momen Gele, Moment 100, Momesone, Momesun, Mometamed, mometasone, mometasone furoate, Monimate, monosodium alpha-luminol, Mopik, MOR103, MOR104, MOR105, MOR208 antibody, MORAb022, Moricam, morniflumate, Mosuolit, Motoral, Movaxin, Mover, Movex, Movix, Movoxicam, Mox Forte, Moxen, moxifloxacin hydrochloride, Mozobil, MP, MP0210, MP0270, MP1000, MP1031, MP196, MP435, MPA, mPGES-1 inhibitor, MPSS, MRX7EAT, MSL, MT203, MT204, mTOR Inhibitor, MTRX1011A, Mucolase, Multicort, MultiStem, muramidase, muramidase, muramidase hydrochloride, muromonab-CD3, Muslax, Muspinil, Mutaze, Muvera, MX68, Mycept, Mycocell, Mycocept, Mycofenolatmofetil Actavis, Mycofet, Mycofit, Mycolate, Mycoldosa, Mycomun, Myconol, mycophenolate mofetil, mycophenolate sodium, mycophenolic acid, Mycotil, myeloid progenitor cells, Myfenax, Myfetil, Myfortic, Mygraft, Myochrysine, Myocrisin, Myprodol, Mysone, nab-Cyclosporine, Nabentac, nabiximols, Nabton, Nabuco, Nabucox, Nabuflam, Nabumet, nabumetone, Nabuton, Nac Plus, Nacta, Nacton, Nadium, Naklofen SR, NAL1207, NAL1216, NAL1219, NAL1268, NAL8202, Nalfon, Nalgesin S, namilumab, Namsafe, nandrolone, Nanocort, Nanogam, Nanosomal Tacrolimus, Napageln, Napilac, Naprelan, Napro, Naprodil, Napronax, Napropal, Naproson, Naprosyn, Naproval, Naprox, naproxen, naproxen sodium, Naproxin, Naprozen, Narbon, Narexsin, Naril, Nasida, natalizumab, Naxdom, Naxen, Naxin, Nazovel, NC2300, ND07, NDC01352, Nebumetone, NecLipGCSF, Necsulide, Necsunim, Nelsid-S, Neo Clobenate, Neo Swiflox FC, Neocoflan, Neo-Drol, Neo-Eblimon, Neo-Hydro, Neoplanta, Neoporine, Neopreol, Neoprox, Neoral, Neotrexate, Neozen, Nepra, Nestacort, Neumega, Neupogen, Neuprex, Neurofenac, Neurogesic, Neurolab, Neuroteradol, Neuroxicam, Neutalin, neutrazumab, Neuzym, New Panazox, Newfenstop, NewGam, Newmafen, Newmatal, Newsicam, NEX1285, sFcRIIB, Nextomab, NF-kappaB Inhibitor, NF-kB inhibitor, NGD20001, NHP554B, NHP554P, NI0101 antibody, NI0401, NI0501 antibody, NI0701, NI071, NI1201 antibody, NI1401, Nicip, Niconas, Nicool, NiCord, Nicox, Niflumate, Nigaz, Nikam, Nilitis, Nimace, Nimaid, Nimark-P, Nimaz, Nimcet Juicy, Nime, Nimed, Nimepast, nimesulide, Nimesulix, Nimesulon, Nimica Plus, Nimkul, Nimlin, Nimnat, Nimodol, Nimpidase, Nimsaid-S, Nimser, Nimsy-SP, Nimupep, Nimusol, Nimutal, Nimuwin, Nimvon-S, Nincort, Niofen, Nipan, Nipent, Nise, Nisolone, Nisopred, Nisoprex, Nisulid, nitazoxanide, Nitcon, nitric oxide, Nizhvisal B, Nizon, NL, NMR1947, NN8209, NN8210, NN8226, NN8555, NN8765, NN8828, NNC014100000100, NNC051869, Noak, Nodevex, Nodia, Nofenac, Noflagma, Noflam, Noflamen, Noflux, Non-antibacterial Tetracyclines, Nonpiron, Nopain, Normferon, Notpel, Notritis, Novacort, Novagent, Novarin, Novigesic, NOXA12, NOXD19, Noxen, Noxon, NPI1302a-3, NPI1342, NPI1387, NPI1390, NPRCS1, NPRCS2, NPRCS3, NPRCS4, NPRCS5, NPRCS6, NPS3, NPS4, nPT-ery, NU3450, nuclear factor NF-kappa-B p65 subunit oligonucleotide, Nucort, Nulojix, Numed-Plus, Nurokind Ortho, Nusone-H, Nutrikemia, Nuvion, NV07alpha, NX001, Nyclobate, Nyox, Nysa, Obarcort, OC002417, OC2286, ocaratuzumab, OCTSG815, Oedemase, Oedemase-D, ofatumumab, Ofgyl-O, Ofvista, OHR118, OKi, Okifen, Oksamen, Olai, olokizumab, Omeprose E, Omnacortil, Omneed, Omniclor, Omnigel, Omniwel, onercept, ONO4057, ONS1210, ONS1220, Ontac Plus, Ontak, ONX0914, OPC6535, opebacan, OPN101, OPN201, OPN302, OPN305, OPN401, oprelvekin, OPT66, Optifer, Optiflur, OptiMIRA, Orabase Hca, Oradexon, Oraflex, OralFenac, Oralog, Oralpred, Ora-sed, Orasone, orBec, Orbone forte, Orcl, ORE10002, ORE10002, Orencia, Org214007, Org217993, Org219517, Org223119, Org37663, Org39141, Org48762, Org48775, Orgadrone, Ormoxen, Orofen Plus, Oromylase Biogaran, Orthal Forte, Ortho Flex, Orthoclone OKT3, Orthofen, Orthoflam, Orthogesic, Orthoglu, Ortho-II, Orthomac, Ortho-Plus, Ortinims, Ortofen, Orudis, Oruvail, OS2, Oscart, Osmetone, Ospain, Ossilife, Ostelox, Osteluc, Osteocerin, osteopontin, Osteral, otelixizumab, Otipax, Ou Ning, OvaSave, OX40 Ligand Antibody, Oxa, Oxagesic CB, Oxalgin DP, oxaprozin, OXCQ, Oxeno, Oxib MD, Oxibut, Oxicam, Oxiklorin, Oximal, Oxynal, oxyphenbutazone, Oxyphenbutazone, ozoralizumab, P13 peptide, P1639, P21, P2X7 Antagonists, p38 Alpha Inhibitor, p38 Antagonist, p38 MAP kinase inhibitor, p38alpha MAP Kinase Inhibitor, P7 peptide, P7170, P979, PA401, PA517, Pabi-dexamethasone, PAC, PAC10649, paclitaxel, Painoxam, Paldon, Palima, pamapimod, Pamatase, Panafcort, Panafcortelone, Panewin, PanGraf, Panimun Bioral, Panmesone, Panodin SR, Panslay, Panzem, Panzem NCD, PAP1, papain, Papirzin, Pappen K Pap, Paptinim-D, paquinimod, PAR2 Antagonist, Paracetamol, Paradic, Parafen TAJ, Paramidin, Paranac, Parapar, Parci, parecoxib, Parixam, Parry-S, Partaject Busulfan, pateclizumab, Paxceed, PBI0032, PBI1101, PBI1308, PBI1393, PBI1607, PBI1737, PBI2856, PBI4419, PBI4419, P-Cam, PCI31523, PCI32765, PCI34051, PCI45261, PCI45292, PCI45308, PD360324, PD360324, PDA001, PDE4 inhibitor, PDE-IV Inhibitor, PDL241 antibody, PDL252, Pediapred, Pefree, pegacaristim, Peganix, Peg-Interleukin 12, pegsunercept, Pegsunercept, PEGylated arginine deiminase, peldesine, pelubiprofen, Penacle, penicillamine, Penostop, Pentalgin, Pentasa, Pentaud, pentostatin, Peon, Pepdase, Pepser, Peptirase, Pepzen, Pepzol, Percutalgine, Periochip, Peroxisome Proliferator Activated Receptor gamma modulators, Petizene, PF00344600, PF04171327, PF04236921, PF04308515, PF05230905, PF05280586, PF251802, PF3475952, PF3491390, PF3644022, PF4629991, PF4856880, PF5212367, PF5230896, PF547659, PF755616, PF9184, PG27, PG562, PG760564, PG8395, PGE3935199, PGE527667, PH5, PH797804, PHA408, Pharmaniaga Mefenamic acid, Pharmaniaga Meloxicam, Pheldin, Phenocept, phenylbutazone, PHY702, PI3K delta inhibitor, PI3K Gamma/Delta Inhibitor, PI3K Inhibitor, Picalm, pidotimod, piketoprofen, Pilelife, Pilopil, Pilovate, pimecrolimus, Pipethanen, Piractam, Pirexyl, Pirobet, Piroc, Pirocam, Pirofel, Pirogel, Piromed, Pirosol, Pirox, Piroxen, Piroxicam, piroxicam betadex, Piroxifar, Piroxil, Piroxim, Pixim, Pixykine, PKC Theta Inhibitor, PL3100, PL5100 Diclofenac, Placenta Polypeptide, Plaquenil, plerixafor, Plocfen, PLR14, PLR18, Plutin, PLX3397, PLX5622, PLX647, PLX-BMT, pms-Diclofenac, pms-Ibuprofen, pms-Leflunomide, pms-Meloxicam, pms-Piroxicam, pms-Prednisolone, pms-Sulfasalazine, pms-Tiaprofenic, PMX53, PN0615, PN100, PN951, podofilox, POL6326, Polcortolon, Polyderm, Polygam S/D, Polyphlogin, Poncif, Ponstan, Ponstil Forte, Porine-A Neoral, Potaba, potassium aminobenzoate, Potencort, Povidone, povidone iodine, pralnacasan, Prandin, Prebel, Precodil, Precortisyl Forte, Precortyl, Predfoam, Predicort, Predicorten, Predilab, Predilone, Predmetil, Predmix, Predna, Prednesol, Predni, prednicarbate, Prednicort, Prednidib, Prednifarma, Prednilasca, prednisolone, prednisolone acetate, prednisolone sodium phosphate, prednisolone sodium succinate, prednisolone sodium succinate, prednisone, prednisone acetate, Prednitop, Prednol-L, Prednox, Predone, Predonema, Predsol, Predsolone, Predsone, Predval, Preflam, Prelon, Prenaxol, Prenolone, Preservex, Preservin, Presol, Preson, Prexige, Priliximab, Primacort, Primmuno, Primofenac, prinaberel, Privigen, Prixam, Probuxil, Procarne, Prochymal, Procider-EF, Proctocir, Prodase, Prodel B, Prodent, Prodent Verde, Proepa, Profecom, Profenac L, Profenid, Profenol, Proflam, Proflex, Progesic Z, proglumetacin, proglumetacin maleate, Prograf, Prolase, Prolixan, promethazine hydrochloride, Promostem, Promune, PronaB, pronase, Pronat, Prongs, Pronison, Prontoflam, Propaderm-L, Propodezas, Propolisol, Proponol, propyl nicotinate, Prostaloc, Prostapol, Protacin, Protase, Protease Inhibitors, Protectan, Proteinase Activated Receptor 2 Inhibitor, Protofen, Protrin, Proxalyoc, Proxidol, Proxigel, Proxil, Proxym, Prozym, PRT062070, PRT2607, PRTX100, PRTX200, PRX106, PRX167700, Prysolone, PS031291, PS375179, PS386113, PS540446, PS608504, PS826957, PS873266, Psorid, PT, PT17, PTL101, P-Transfer Factor peptides, PTX3, Pulminiq, Pulsonid, Purazen, Pursin, PVS40200, PX101, PX106491, PX114, PXS2000, PXS2076, PYM60001, Pyralvex, Pyranim, pyrazinobutazone, Pyrenol, Pyricam, Pyrodex, Pyroxi-Kid, QAX576, Qianbobiyan, QPI1002, QR440, qT3, Quiacort, Quidofil, R107s, R125224, R1295, R132811, R1487, R1503, R1524, R1628, R333, R348, R548, R7277, R788, rabeximod, Radix Isatidis, Radofen, Raipeck, Rambazole, Randazima, Rapacan, Rapamune, Raptiva, Ravax, Rayos, RDEA119, RDEA436, RDP58, Reactine, Rebif, REC200, Recartix-DN, receptor for advanced glycation end products antibody, Reclast, Reclofen, recombinant HSA-TIMP-2, recombinant human alkaline Phosphatase, recombinant Interferon Gamma, Recominant human alkaline phosphatase, Reconil, Rectagel HC, Recticin, Recto Menaderm, Rectos, Redipred, Redolet, Refastin, Regenica, REGN88, Relafen, Relaxib, Relev, Relex, Relifen, Relifex, Relitch, Rematof, remestemcel-1, Remesulidum, Remicade,, Remsima, Remsima, Remsima, ReN1869, Renacept, Renfor, Renodapt, Renodapt-S, Renta, Reosan, Repare-AR, Reparilexin, reparixin, Repertaxin, Repisprin, Resochin, Resol, resolvin E1, Resurgil, Re-tin-colloid, Retoz, Reumacap, Reumacon, Reumadolor, Reumador, Reumanisal, Reumazin, Reumel, Reumotec, Reuquinol, revamilast, Revascor, Reviroc, Revlimid, Revmoksikam, Rewalk, Rexalgan, RG2077, RG3421, RG4934 antibody, RG7416, RG7624, Rheila, Rheoma, Rheprox, Rheudenolone, Rheufen, Rheugesic, Rheumacid, Rheumacort, Rheumatrex, Rheumesser, Rheumid, Rheumon, Rheumox, Rheuoxib, Rhewlin, Rhucin, RhuDex, Rhulef, Ribox, Ribunal, Ridaura, rifaximin, rilonacept, rimacalib, Rimase, Rimate, Rimatil, Rimesid, risedronate sodium, Ritamine, Rito, Rituxan, rituximab, RNS60, RO1138452, Ro313948, RO3244794, RO5310074, Rob803, Rocamix, Rocas, Rofeb, rofecoxib, Rofee, Rofewal, Roficip Plus, Rojepen, Rokam, Rolodiquim, Romacox Fort, Romatim, romazarit, Ronaben, ronacaleret, Ronoxcin, ROR Gamma T Antagonist, ROR gamma t inverse agonists, Rosecin, rosiglitazone, Rosmarinic acid, Rotan, Rotec, Rothacin, Roxam, Roxib, Roxicam, Roxopro, Roxygin DT, RP54745, RPI78, RPI78M, RPI78MN, RPIMN, RQ00000007, RQ00000008, RTA402, R-Tyflam, Rubicalm, Rubifen, Ruma pap, Rumalef, Rumidol, Rumifen, Runomex, rusalatide acetate, ruxolitinib, RWJ445380, RX10001, Rycloser MR, Rydol, SIP Receptor Agonists, SIP Receptor Modulators, S1P1 Agonist, S1P1 receptor agonist, S2474, S3013, SA237, SA6541, Saaz, S-adenosyl-L-methionine-sulfate-p-toluene sulfonate, Sala, Salazidin, Salazine, Salazopyrin, Salcon, Salicam, salsalate, Sameron, SAN300, Sanaven, Sandimmun, Sandoglobulin, Sanexon, SangCya, SAR153191, SAR302503, SAR479746, Sarapep, sargramostim, Sativex, Savantac, Save, Saxizon, Sazo, SB1578, SB210396, SB217969, SB242235, SB273005, SB281832, SB683698, SB751689, SBI087, SC080036, SC12267, SC409, Scaflam, SCD ketoprofen, SCIO323, SCIO469, SD-15, SD281, SDP051 antibody, Sd-rxRNA, secukinumab, Sedase, Sedilax, Sefdene, Seizyme, SEL113, Seladin, Selecox, selectin P ligand antibody, Glucocorticoid Receptor Agonist, Selectofen, Selektine, SelK1 antibody, Seloxx, Selspot, Selzen, Selzenta, Selzentry, semapimod, semapimod hydrochloride, semparatide, Semparatide, Senafen, Sendipen, Senterlic, SEP119249, Sepdase, Septirose, Seractil, Serafen-P, Serase, Seratid D, Seratiopeptidase, Serato-M, Seratoma Forte, Serazyme, Serezon, Sero, Serodase, Serpicam, Serra, serrapeptase, Serratin, Serratiopeptidase, Serrazyme, Servisone, Seven E P, SGI1252, SGN30, SGN70, SGX203, shark cartilage extrack, Sheril, Shield, Shifazen, Shifazen-Fort, Shincort, Shincort, Shiosol, ShK186, Shuanghuangxiaoyan, SI615, SI636, Sigmasporin, Sigmasporin, SIM916, Simpone, Simulect, Sinacort, Sinalgia, Sinapol, Sinatrol, Sinsia, siponimod, Sirolim, sirolimus, Siropan, Sirota, Sirova, sirukumab, Sistal Forte, SKF105685, SKF105809, SKF106615, SKF86002, Skinalar, Skynim, Skytrip, SLAM family member 7 antibody, Slo-indo, SM101, SM201 antibody, SM401, SMAD family member 7 oligonucleotide, SMART Anti-IL-12 Antibody, SMP114, SNO030908, SNO070131, sodium aurothiomalate, sodium chondroitin sulfate, sodium deoxyribonucleotide, sodium gualenate, sodium naproxen, sodium salicylate, Sodixen, Sofeo, Soleton, Solhidrol, Solicam, Soliky, Soliris, Sol-Melcort, Solomet, Solondo, Solone, Solu-Cort, Solu-Cortef, Solu-Decortin H, Solufen, Solu-Ket, Solumark, Solu-Medrol, Solupred, Somalgen, somatropin, Sonap, Sone, sonepcizumab, Sonexa, Sonim, Sonim P, Soonil, Soral, Sorenil, sotrastaurin acetate, SP-10, SP600125, Spanidin, SP-Cortil, SPD550, Spedace, sperm adhesion molecule 1, Spictol, spleen tyrosine kinase oligonucleotide, Sporin, S-prin, SPWF1501, SQ641, SQ922, SR318B, SR9025, SRT2104, SSR150106, SSR180575, SSS07 antibody, ST1959, STA5326, stabilin 1 antibody, Stacort, Stalogesic, stanozolol, Staren, Starmelox, Stedex IND-SWIFT, Stelara, Stemin, Stenirol, Sterapred, Steriderm S, Sterio, Sterisone, Steron, stichodactyla helianthus peptide, Stickzenol A, Stiefcortil, Stimulan, STNM01, Store Operated Calcium Channel (SOCC) Modulator, STP432, STP900, Stratasin, Stridimmune, Strigraf, SU Medrol, Subreum, Subuton, Succicort, Succimed, Sulan, Sulcolon, Sulfasalazin Heyl, Sulfasalazin, sulfasalazine, Sulfovit, Sulidac, Sulide, sulindac, Sulindex, Sulinton, Sulphafine, Sumilu, SUN597, Suprafen, Supretic, Supsidine, Surgam, Surgamine, Surugamu, Suspen, Suton, Suvenyl, Suwei, SW Dexasone, Syk Family Kinase Inhibitor, Syn1002, Synacran, Synacthen, Synalar C, Synalar, Synavive, Synercort, Sypresta, T cell cytokine-inducing surface molecule antibody, T cell receptor antibody, T5224, T5226, TA101, TA112, TA383, TA5493, tabalumab, Tacedin, Tacgraf, TACIFc5, Tacrobell, Tacrograf, Tacrol, tacrolimus, Tadekinig alpha, Tadolak, TAFA93, Tafirol Artro, Taizen, TAK603, TAK715, TAK783, Takfa, Taksta, talarozole, Talfin, Talmain, talmapimod, Talmea, Talnif, talniflumate, Talos, Talpain, Talumat, Tamalgen, Tamceton, Tamezon, Tandrilax, tannins, Tannosynt, Tantum, tanzisertib, Tapain-beta, Tapoein, Tarenac, tarenflurbil, Tarimus, Tarproxen, Tauxib, Tazomust, TBR652, TC5619, T-cell, immune regulator 1, ATPase, H+ transporting, lysosomal V0 subunit A3 antibody, TCK1, T-cort, T-Dexa, Tecelac, Tecon, teduglutide, Teecort, Tegeline, Tementil, temoporfin, Tencam, Tendrone, Tenefuse, Tenfly, tenidap sodium, Tenocam, Tenoflex, Tenoksan, Tenotil, tenoxicam, Tenoxim, Tepadina, Teracort, Teradol, tetomilast, TG0054, TG1060, TG20, TG20, tgAAC94, Th1/Th2 Cytokine Synthase Inhibitor, Th-17 cell inhibitors, Thalido, thalidomide, Thalomid, Themisera, Thenil, Therafectin, Therapyace, thiarabine, Thiazolopyrimidines, thioctic acid, thiotepa, THR090717, THR0921, Threenofen, Thrombate III, Thymic peptide, Thymodepressin, Thymogam, Thymoglobulin, Thymoglobuline, Thymoject thymic peptides, thymomodulin, thymopentin, thymopolypetides, tiaprofenic acid, tibezonium iodide, Ticoflex, tilmacoxib, Tilur, T-immune, Timocon, Tiorase, Tissop, TKB662, TL011, TLR4 antagonists, TLR8 inhibitor, TM120, TM400, TMX302, TNF Alpha inhibitor, TNF alpha-TNF receptor antagonist, TNF antibody, TNF receptor superfamily antagonists, TNF TWEAK Bi-Specific, TNF-Kinoid, TNFQb, TNFR1 antagonist, TNR001, TNX100, TNX224, TNX336, TNX558, tocilizumab, tofacitinib, Tokuhon happ, TOL101, TOL102, Tolectin, ToleriMab, Tolerostem, Tolindol, toll-like receptor 4 antibody, toll-like receptor antibody, tolmetin sodium, Tongkeeper, Tonmex, Topflame, Topicort, Topleucon, Topnac, Toppin Ichthammol, toralizumab, Toraren, Torcoxia, Toroxx, Tory, Toselac, Totaryl, Touch-med, Touchron, Tovok, Toxic apis, Toyolyzom, TP4179, TPCA1, TPI526, TR14035, Tradil Fort, Traficet-EN, Tramace, tramadol hydrochloride, tranilast, Transimune, Transporina, Tratul, Trexall, Triacort, Triakort, Trialon, Triam, triamcinolone, triamcinolone acetate, triamcinolone acetonide, triamcinolone acetonide acetate, triamcinolone hexacetonide, Triamcort, Triamsicort, Trianex, Tricin, Tricort, Tricortone, TricOs T, Triderm, Trilac, Trilisate, Trinocort, Trinolone, Triolex, triptolide, Trisfen, Trivaris, TRK170, TRK530, Trocade, trolamine salicylate, Trolovol, Trosera, Trosera D, Troycort, TRX1 antibody, TRX4, Trymoto, Trymoto-A, TT301, TT302, TT32, TT32, TT33, TTI314, tumor necrosis factor, tumor necrosis factor 2-methoxyethyl phosphorothioate oligonucleotide, tumor necrosis factor antibody, tumor necrosis factor kinoid, tumor necrosis factor oligonucleotide, tumor necrosis factor receptor superfamily, member 1B antibody, tumor necrosis factor receptor superfamily1B oligonucleotide, tumor necrosis factor superfamily, member 12 antibody, tumor necrosis factor superfamily, member 4 antibody, tumor protein p53 oligonucleotide, tumour necrosis factor alpha antibody, TuNEX, TXA127, TX-RAD, TYK2 inhibitors, Tysabri, ubidecarenone, Ucerase, ulodesine, Ultiflam, Ultrafastin, Ultrafen, Ultralan, U-Nice-B, Uniplus, Unitrexate, Unizen, Uphaxicam, UR13870, UR5269, UR67767, Uremol-HC, Urigon, U-Ritis, ustekinumab, V85546, Valcib, Valcox, valdecoxib, Valdez, Valdixx, Valdy, Valentac, Valoxib, Valtune, Valus AT, Valz, Valzer, Vamid, Vantal, Vantelin, VAP-1 SSAO Inhibitor, vapaliximab, varespladib methyl, Varicosin, Varidase, vascular adhesion protein-1 antibody, VB110, VB120, VB201, VBY285, Vectra-P, vedolizumab, Vefren, VEGFR-1 Antibody, Veldona, veltuzumab, Vendexine, Venimmun N, Venoforte, Venoglobulin-IH, Venozel, Veral, Verax, vercirnon, vero-Dexamethasone, Vero-Kladribin, Vetazone, VGX1027, VGX750, Vibex MTX, vidofludimus, Vifenac, Vimovo, Vimultisa, Vincort, Vingraf, Vioform-HC, Vioxl, Vioxx, Virobron, visilizumab, Vivaglobin, Vivalde Plus, Vivian-A, VLST002, VLST003, VLST004, VLST005, VLST007, Voalla, voclosporin, Vokam, Vokmor, Volmax, Volna-K, Voltadol, Voltagesic, Voltanase, Voltanec, Voltaren, Voltarile, Voltic, Voren, vorsetuzumab, Votan-SR, VR909, VRA002, VRP1008, VRS826, VRS826, VT111, VT214, VT224, VT310, VT346, VT362, VTX763, Vurdon, VX30 antibody, VX467, VX5, VX509, VX702, VX740, VX745, VX745, VX850, W54011, Walacort, Walix, WC3027, Wilgraf, Winflam, Winmol, Winpred, Winsolve, Wintogeno, WIP901, Woncox, WSB711 antibody, WSB712 antibody, WSB735, WSB961, X071NAB, X083NAB, Xantomicin Forte, Xedenol, Xefo, Xefocam, Xenar, Xepol, X-Flam, Xibra, Xicam, Xicotil, Xifaxan, XL499, XmAb5483, XmAb5485, XmAb5574, XmAb5871, XOMA052, Xpress, XPro1595, XtendTNF, XToll, Xtra, Xylex-H, Xynofen SR, Yang Shu-IVIG, YHB14112, YM974, Youfeline, Youfenac, Yuma, Yumerol, Yuroben, YY piroxicam, Z104657A, Zacy, Zaltokin, zaltoprofen, Zap70 Inhibitor, Zeepain, Zeloxim Fort, Zema-Pak, Zempack, Zempred, Zenapax, Zenas, Zenol, Zenos, Zenoxone, Zerax, Zerocam, Zerospasm, ZFNs, zinc oxide, Zipsor, ziralimumab, Zitis, Zix-S, Zocort, Zodixam, Zoftadex, zoledronic acid, Zolfin, Zolterol, Zopyrin, Zoralone, ZORprin, Zortress, ZP1848, zucapsaicin, Zunovate, Zwitterionic polysaccharides, ZY1400, Zybodies, Zycel, Zyrofen, Zyrogen Inhibitors, Zyser, Zytrim, and Zywin-Forte. In addition, the anti-inflammatory drugs, as listed above, may be combined with one or more agents listed above or herein or with other agents known in the art.

In one embodiment, a drug that reduces, inhibits, prevents and/or ameliorates inflammation, for example, one of the drugs provided above, is delivered to the suprachoroidal space of the eye using the microneedle devices and methods disclosed herein, and is used to treat, prevent and/or ameliorate a disease or disorder selected from arthritis, degenerative arthritis, psoriatic arthritis, arthritic disorders, arthritic pain, arthrosis, autoimmune arthritis, autoimmune diseases, autoimmune disorders, axial spondyloarthritis, chronic prosthetic joint infection, collagen induced arthritis, osteoarthritis, rheumatoid arthritis, senile arthritis, seronegative oligoarthritis of the knee, allergic and autoimmune inflammatory diseases, inflammatory diseases, inflammatory disorders, collagen diseases, discoid Lupus Erythematosus, immune deficiencies, immune diseases, immune disorders, immunodeficiency diseases, immunodeficiency disorders, immunoglobulin (IgG2) deficiency, immunoglobulin deficiency, Inflammation, Lambert-Eaton myasthenia syndrome, polymyositis, dermatomyositis, polyneuritis, post-operative ocular inflammation, polychondritis, sporadic inclusion body myositis, Systemic Lupus Erythematosus, T cell deficiency, TNF-receptor associated periodic syndrome, tropical spastic paraparesis, Wegener Granulomatosis, X-linked severe combined immunodeficiency disease, Behcet's disease, Crohn's disease, Crohn's Fistula, cutaneous Lupus Erythematosus, acute inflammation, acute inflammatory edema, adrenocortical insufficiency, cerebral inflammation, chronic lung inflammation, corticoid-responsive inflammatory skin disorders, cutaneous inflammation, dermal inflammation, dry skin inflammatory disease, ear edema, ear inflammation, glossitis, inflammatory bowel disease, inflammatory degenerative disease, inflammatory disorders of the eye and/or ear, inflammatory lesions in fungal infections, inflammatory lesions, inflammatory pain, inflammatory skin diseases or disorders, mouth and gum inflammation, mouth and throat inflammation, musculoskeletal disorders, otitis, pelvic inflammatory disease, perianal inflammation, post operative inflammation, pulmonary inflammation, rectal inflammation, refractory idiopathic inflammatory myopathies, seborrhoeic dermatitis, swelling, aphthous ulcerations, chronic polyarthritis, juvenile rheumatoid arthritis, rheumatic diseases, Sjogren's syndrome, opthalmic for Sjogren's syndrome, transplant rejection, acute allograft rejection, chronic graft rejection, graft versus host disease, humoral rejection in heart transplantation, humoral rejection in kidney transplantation, organ rejection in renal transplantation, solid organ transplant rejection, bronchiolitis obliterans after lung transplantation, rejection of bone marrow transplant, chronic lung transplant rejection, Corneal graft rejection, delayed graft function in kidney transplantation, heart transplant rejection, Homotransplantation rejection, immune rejection of hESC-derived therapeutic grafts, kidney transplant rejection, liver transplant rejection, lung transplant rejection, organ rejection, pancreatic islet transplantation rejection in type I diabetes, renal transplant rejection and xenograft rejection.

In one embodiment, the drug delivered to the suprachoroidal space using the microneedle devices and methods disclosed herein treats, prevents, and/or ameliorates macular degeneration (e.g., age related macular degeneration, dry age related macular degeneration, exudative age-related macular degeneration, geographic atrophy associated with age related macular degeneration, neovascular (wet) age-related macular degeneration, neovascular maculopathy and age related macular degeneration, occult with no classic choroidal neovascularization (CNV) in age-related macular degeneration, Stargardt's disease, Subfoveal wet Age-Related macular degeneration, and Vitreomacular Adhesion (VMA) associated with Neovascular Age Related macular degeneration). Examples of drugs that treat, prevent and/or ameliorate macular degeneration that can be used in conjunction with the devices and methods described herein include, but are not limited to: A0003, A36 peptide, AAV2-sFLT01 , ACE041, ACU02, ACU3223, ACU4429, AdPEDF, aflibercept, AG13958, aganirsen, AGN150998, AGN745, AL39324, AL78898A, AL8309B, ALN-VEG01, alprostadil, AM1101, amyloid beta antibody, anecortave acetate, Anti-VEGFR-2 Alterase, Aptocine, APX003, ARC1905, ARC1905 with Lucentis, ATG3, ATP-binding cassette, subfamily A, member 4 gene, ATXS10, Avastin with Visudyne, AVT101, AVT2, bertilimumab, bevacizumab with verteporfin, bevasiranib sodium, bevasiranib sodium; with ranibizumab, brimonidine tartrate, BVA301, canakinumab, Cand5, Cand5 with Lucentis, CERE140, ciliary neurotrophic factor, CLT009, CNTO2476, collagen monoclonal antibody, complement component 5 aptamer (pegylated), complement component 5 aptamer (pegylated) with ranibizumab, complement component C3, complement factor B antibody, complement factor D antibody, copper oxide with lutein, vitamin C, vitamin E, and zinc oxide, dalantercept, DE109, dexamethasone with ranibizumab and verteporfin, disitertide, DNA damage inducible transcript 4 oligonucleotide, E10030, E10030 with Lucentis, EC400, eculizumab, EGP, EHT204, embryonic stem cells, human stem cells, endoglin monoclonal antibody, EphB4 RTK Inhibitor, EphB4 Soluble Receptor, ESBA1008, ETX6991, Evizon, Eyebar, EyePromise Five, Eyevi, Eylea, F200, FCFD4514S, fenretinide, fluocinolone acetonide, fluocinolone acetonide with ranibizumab, fms-related tyrosine kinase 1 oligonucleotide, fms-related tyrosine kinase 1 oligonucleotide with kinase insert domain receptor 169, fosbretabulin tromethamine, Gamunex, GEM220, GS101, GSK933776, HC31496, Human n-CoDeR, HYB676, IBI-20089 with Lucentis, iCo-008, Icon1, I-Gold, Ilaris, Iluvien, Iluvien with Lucentis, immunoglobulins, integrin alpha5beta1 immunoglobulin fragments, Integrin inhibitor, IRIS Lutein, I-Sense Ocushield, Isonep, isopropyl unoprostone, JPE1375, JSM6427, KH902, LentiVue, LFG316, LP590, LPOIOIOAM, Lucentis, Lucentis with Visudyne, Lutein ekstra, Lutein with myrtillus extrack, Lutein with zeaxanthin, M200, M200 with Lucentis, Macugen, MC1101, MCT355, mecamylamine, Microplasmin, motexafin lutetium, MP0112, NADPH oxidase inhibitors, Neoretna, neurotrophin 4 gene, Nova21012, Nova21013, NT501, NT503, Nutri-Stulln, ocriplasmin, OcuXan, Oftan Macula, Optrin, ORA102 with Avastin, P144, P17, Palomid 529, PAN90806, Panzem, Panzem, PARP Inhibitors, pazopanib hydrochloride, pegaptanib sodium, PF4523655, PG11047, piribedil, platelet-derived growth factor beta polypeptide aptamer (pegylated), platelet-derived growth factor beta polypeptide aptamer (pegylated) with ranibizumab, PLG101, PMX20005, PMX53, POT4, PRS055, PTK787, ranibizumab, ranibizumab with triamcinolone acetonide, ranibizumabwith verteporfin, ranibizumab with volociximab, RD27, Rescula, Retaane, retinal pigment epithelial cells, RetinoStat, RG7417, RN6G, RT101, RTU007, SB267268, serpin peptidase inhibitor, clade F, member 1 gene, shark cartilage extrack, Shef1, SIR1046, SIR1076, Sirna027, sirolimus, SMTD004, Snelvit, SOD Mimetics, Soliris, sonepcizumab, squalamine lactate, ST602, StarGen, T2TrpRS, TA106, talaporfin sodium, Tauroursodeoxycholic acid , TG100801, TKI , TLCx99, TRC093, TRC105, triamcinolone acetonide with verteporfin, Trivastal Retard, TT30, Ursa, ursodiol, Vangiolux, VAR10200, vascular endothelial growth factor antibody, vascular endothelial growth factor B, vascular endothelial growth factor kinoid, vascular endothelial growth factor oligonucleotide, VAST Compounds, vatalanib, VEGF Inhibitor, verteporfin, Visudyne, Visudyne with Lucentis and dexamethasone, Visudyne with triamcinolone acetonide, Vivis, volociximab, Votrient, XV615, zeaxanthin, ZFP TF, zinc-monocysteine and Zybrestat. In one embodiment, one or more of the macular degeneration treating drugs described above is combined with one or more agents listed above or herein or with other agents known in the art.

In one embodiment, the methods and devices provided hererin are used to delivery triamcinolone or triamcinolone acetonide to the suprachoroidal space of an eye of a patient in need thereof. In a further embodiment, the triamcinolone or triamcinolone acetonide is delivered for the treatment of sympathetic ophthalmia, temporal arteritis, uveitis and/or ocular inflammatory conditions. In one embodiment, triamcinolone or triamcinolone acetonide is delivered to the suprachoroidal space of the eye in a patient in need of treatment of sympathetic opthalmia with the methods and devices described herein. In another embodiment, triamcinolone or triamcinolone acetonide is delivered to the suprachoroidal space of the eye in a patient in need of treatment of temporal arteritis with the methods and devices described herein. In yet another embodiment, triamcinolone or triamcinolone acetonide is delivered to the suprachoroidal space of the eye in a patient in need of treatment of uveitis, with the methods and devices described herein. In another embodiment, triamcinolone or triamcinolone acetonide is delivered to the suprachoroidal space of the eye in a patient in need of treatment of one or more ocular inflammatory conditions, with the methods and devices described herein.

The triamcinolone composition provided herein, in one embodiment, is a suspension comprising microparticles or nanoparticles of triamcinolone or triamcinolone acetonide. The microparticles, in one embodiment, have a D₅₀ of about 3 µm or less. In a further embodiment, the D₅₀ is about 2 µm. In another embodiment, the D₅₀ is about 2 µm or less. In even another embodiment, the D₅₀ is about 1000 nm or less. The microparticles, in one embodiment, have a D₉₉ of about 10 µm or less. In another embodiment, the D₉₉ is about 10 µm. In another embodiment, the D₉₉ is less than about 10 µm or less than about 9 µm or less.

In one embodiment, the triamcinolone composition comprises triamcinolone microparticles. In a further embodiment, the composition comprises polysorbate 80. In another embodiment, the triamcinolone composition comprises one or more of CaCl₂, MgCl₂, sodium acetate and sodium citrate. In one embodiment, the composition comprises polysorbate 80 at a w/v% of 0.02% or about 0.02%, 0.015% or about 0.015%.

In certain embodiments the drug delivered to ocular tissues using the microneedle devices and methods disclosed herein treats, prevents, and/or ameliorates fibrosis (e.g. myelofibrosis, fibrosis in diabetic nephropathy, cystic fibrosis, scarring, and skin fibrosis).

In one embodiment, a drug that treats, prevents and/or ameliorates fibrosis is used in conjunction with the devices and methods described herein, and is delivered to the suprachoroidal space of the eye. In a further embodiment, the drug is Actimmune with Pirfenidone, ACUHTR028, AlphaVBeta5, aminobenzoate potassium, amyloid P, ANG1122, ANG1170, ANG3062, ANG3281, ANG3298, ANG4011, Anti-CTGF RNAi, Aplidin, astragalus membranaceus extrack with salvia and schisandra chinensis, atherosclerotic plaque blocker, Azol, AZX100, BB3, connective tissue growth factor antibody , CT140, danazol, Esbriet, EXC001, EXC002, EXC003, EXC004, EXC005, F647, FG3019, Fibrocorin, Follistatin, FT011, Galectin-3 inhibitors, GKT137831, GMCT01, GMCT02, GRMD01, GRMD02, GRN510, Heberon Alfa R, interferon alfa-2b, interferon gamma-lb with pirfenidone, ITMN520, JKB119, JKB121, JKB122, KRX168, LPA1 receptor antagonist, MGN4220, MIA2, microRNA 29a oligonucleotide, MMI0100, noscapine, PBI4050, PBI4419, PDGFR inhibitor, PF-06473871, PGN0052, Pirespa, Pirfenex, pirfenidone, plitidepsin, PRM151, Px102, PYN17, PYN22 with PYN17, Relivergen, rhPTX2 Fusion Proteins, RXI109, secretin, STX100, TGF-beta Inhibitor, transforming growth factor, beta receptor 2 oligonucleotide, VA999260 or XV615. In one embodiment, one or more of the fibrosis treating drugs described above is combined with one or more agents listed above or herein or with other agents known in the art.

In one embodiment, a drug that treats, prevents and/or ameliorates diabetic macular edema is used in conjunction with the devices and methods described herein, and is delivered to the suprachoroidal space of the eye. In a further embodiment, the drug is AKB9778, bevasiranib sodium, Cand5, choline fenofibrate, Cortiject, c-raf 2-methoxyethyl phosphorothioate oligonucleotide, DE109, dexamethasone, DNA damage inducible transcript 4 oligonucleotide, FOV2304, iCo007, KH902, MP0112, NCX434, Optina, Ozurdex, PF4523655, SAR1118, sirolimus, SK0503 or TriLipix. In one embodiment, one or more of the diabetic macular edema treating drugs described above is combined with one or more agents listed above or herein or with other agents known in the art.

In one embodiment, a drug that treats, prevents and/or ameliorates macular edema is used in conjunction with the devices and methods described herein, and is delivered to the suprachoroidal space of the eye. In a further embodiment, the drug is denufosol tetrasodium, dexamethasone, ecallantide, pegaptanib sodium, ranibizumab or triamcinolone. In addition, the drugs delivered to ocular tissues using the microneedle devices and methods disclosed herein which treat, prevent, and/or ameliorate macular edema, as listed above, may be combined with one or more agents listed above or herein or with other agents known in the art.

In one embodiment, a drug that treats, prevents and/or ameliorates ocular hypertension is used in conjunction with the devices and methods described herein and is delivered to the suprachoroidal space of the eye. In a further embodiment, the drug is 2-MeS-beta gamma-CCl2-ATP, Aceta Diazol, acetazolamide, Aristomol, Arteoptic, AZD4017, Betalmic, betaxolol hydrochloride, Betimol, Betoptic S, Brimodin, Brimonal, brimonidine, brimonidine tartrate, Brinidin, Calte, carteolol hydrochloride, Cosopt, CS088, DE092, DE104, DE111, dorzolamide, dorzolamide hydrochloride, Dorzolamide hydrochloride with Timolol maleate, Droptimol, Fortinol, Glaumol, Hypadil, Ismotic, isopropyl unoprostone, isosorbide, Latalux, latanoprost, Latanoprost with Timolol maleate, levobunolol hydrochloride, Lotensin, Mannigen, mannitol, metipranolol, mifepristone, Mikelan, Minims Metipranolol, Mirol, nipradilol, Nor Tenz, Ocupress, olmesartan, Ophtalol, pilocarpine nitrate, Piobaj, Rescula, RU486, Rysmon TG, SAD448, Saflutan, Shemol, Taflotan, tafluprost, tafluprost with timolol, Thiaboot, Timocomod, timolol, Timolol Actavis, timolol hemihydrate, timolol maleate, Travast, travoprost, Unilat, Xalacom, Xalatan or Zomilol. In addition, the drugs delivered to ocular tissues using the microneedle devices and methods disclosed herein which treat, prevent, and/or ameliorate ocular hypertension, as listed above, may be combined with one or more agents listed above or herein or with other agents known in the art.

In certain embodiments one or more drugs may be delivered to ocular tissues and/or into the suprachoroidal space via the microneedle device described herein. Delivery of one or more drugs into the suprachoroidal space using the microneedle device described herein may be accomplished by using one or more microneedles. In addition, combinations of one of more drugs may be delivered to the suprachoroidal space using the microneedle device described herein in combination with delivery of one or more drugs via intravitreal (IVT) administration (e.g., intravitreal injection, intravitreal implant or eye drops). Methods of IVT administration are well known in the art. Examples of drugs that can be administered via IVT include, but are not limited to: A0003, A0006, Acedolone, AdPEDF, aflibercept, AG13958, aganirsen, AGN208397, AKB9778, AL78898A, amyloid P, Angiogenesis Inhibitor Gene Therapy, ARC 1905, Aurocort, bevasiranib sodium, brimonidine, Brimonidine, brimonidine tartrate, bromfenac sodium, Cand5, CERE140, Ciganclor, CLT001, CLT003, CLT004, CLT005, complement component 5 aptamer (pegylated), complement factor D antibody, Cortiject, c-raf 2-methoxyethyl phosphorothioate oligonucleotide, cyclosporine, triamcinolone, DE109, denufosol tetrasodium, dexamethasone, dexamethasone phosphate, disitertide, DNA damage inducible transcript 4 oligonucleotide, E10030, ecallantide, EG3306, Eos013, ESBA1008, ESBA105, Eylea, FCFD4514S, fluocinolone acetonide, fms-related tyrosine kinase 1 oligonucleotide, fomivirsen sodium, fosbretabulin tromethamine, FOV2301, FOV2501, ganciclovir, ganciclovir sodium, GS101, GS156, hyaluronidase, IBI20089, iCo007, Iluvien, INS37217, Isonep, JSM6427, Kalbitor, KH902, lerdelimumab, LFG316, Lucentis, M200, Macugen, Makyueido, Microplasmin, MK0140, MP0112, NCX434, neurotrophin 4 gene, OC10X, ocriplasmin, ORA102, Ozurdex, P144, P17, Palomid 529, pazopanib hydrochloride, pegaptanib sodium, Plasma Kallikrein Inhibitors, platelet-derived growth factor beta polypeptide aptamer (pegylated), POT4, PRM167, PRS055, QPI1007, ranibizumab, resveratrol, Retilone, retinal pigment epithelium-specific protein 65kDa gene, Retisert, rod derived cone viability factor, RPE65 Gene Therapy, RPGR Gene Therapy, RTP801, Sd-rxRNA, serpin peptidase inhibitor clade F member 1 gene, Sirna027, sirolimus, sonepcizumab, SRT501, STP601, TG100948, Trabio, triamcinolone, triamcinolone acetonide, Trivaris, tumor necrosis factor antibody, VEGF/rGel-Op, verteporfin, Visudyne, Vitrase, Vitrasert, Vitravene, Vitreals, volociximab, Votrient, XG102, Xibrom, XV615, and Zybrestat. Accordingly, the methods of the present invention include administrating via IVT one or more of the drugs listed above in combination with one or more drugs disclosed herein administered into the suprachoroidal space using the microneedle device described herein.

In one embodiment, the drug is formulated for storage and delivery via the microneedle device described herein. The "drug formulation" is a formulation of a drug, which typically includes one or more pharmaceutically acceptable excipient materials known in the art. The term "excipient" refers to any non-active ingredient of the formulation intended to facilitate handling, stability, dispersibility, wettability, release kinetics, and/or injection of the drug. In one embodiment, the excipient may include or consist of water or saline.

In one embodiment, the fluid drug formulation includes microparticles or nanoparticles, either of which includes at least one drug. Desirably, the microparticles or nanoparticles provide for the controlled release of drug into the ocular tissue. As used herein, the term "microparticle" encompasses microspheres, microcapsules, microparticles, and beads, having a number average diameter of 1 to 100 µm, most preferably 1 to 25 µm. The term "nanoparticles" are particles having a number average diameter of 1 to 1000 nm. Microparticles may or may not be spherical in shape. "Microcapsules" are defined as microparticles having an outer shell surrounding a core of another material. The core can be liquid, gel, solid, gas, or a combination thereof. In one case, the microcapsule may be a "microbubble" having an outer shell surrounding a core of gas, wherein the drug is disposed on the surface of the outer shell, in the outer shell itself, or in the core. (Microbubbles may be respond to accoustic vibrations as known in the art for diagnosis or to burst the microbubble to release its payload at/into a select ocular tissue site.) "Microspheres" can be solid spheres, can be porous and include a sponge-like or honeycomb structure formed by pores or voids in a matrix material or shell, or can include multiple discrete voids in a matrix material or shell. The microparticle or nanoparticles may further include a matrix material. The shell or matrix material may be a polymer, amino acid, saccharride, or other material known in the art of microencapsulation.

The drug-containing microparticles or nanoparticles may be suspended in an aqueous or non-aqueous liquid vehicle. The liquid vehicle may be a pharmaceutically acceptable aqueous solution, and optionally may further include a surfactant. The microparticles or nanoparticles of drug themselves may include an excipient material, such as a polymer, a polysaccharide, a surfactant, etc., which are known in the art to control the kinetics of drug release from particles.

In one embodiment, the fluid drug formulation further includes an agent effective to degrade collagen or GAG fibers in the sclera, which may enhance penetration/release of the drug into the ocular tissues. This agent may be, for example, an enzyme, such a hyaluronidase, a collagenase, or a combination thereof. In a variation of this method, the enzyme is administered to the ocular tissue in a separate step from-preceding or following-infusion of the drug. The enzyme and drug are administered at the same site.

In another embodiment, the drug formulation is one which undergoes a phase change upon administration. For instance, a liquid drug formulation may be injected through hollow microneedles into the suprachoroidal space, where it then gels and the drug diffuses out from the gel for controlled release.

The embodiments described herein can be formed or constructed of one or more biocompatible materials. For example, any of the microneedles shown and described herein can be constructed of a substantially rigid material such that the microneedle does not substantially deform when used according to the methods described herein. Examples of suitable biocompatible materials include metals, glasses, ceramics, polymers, and/or nanotubes (e.g., carbon nanotubes). Examples of suitable metals include pharmaceutical grade stainless steel, gold, titanium, nickel, iron, platinum, tin, chromium, copper, and alloys thereof. The polymer may be biodegradable or non-biodegradable. Examples of suitable biodegradable polymers include polylactides, polyglycolides, polylactide-co-glycolides (PLGA), polyanhydrides, polyorthoesters, polyetheresters, polycaprolactones, polyesteramides, poly(butyric acid), poly(valeric acid), polyurethanes and copolymers and blends thereof. Examples of non-biodegradable polymers include nylons, polyesters, polycarbonates, polyacrylates, polymers of ethylene-vinyl acetates and other acyl substituted cellulose acetates, non-degradable polyurethanes, polystyrenes, polyvinyl chloride, polyvinyl fluoride, poly(vinyl imidazole), chlorosulphonate polyolefins, polyethylene oxide, blends and copolymers thereof.

The microneedles described herein can be fabricated by a variety of methods. For example, in some embodiments, the hollow microneedle is fabricated using a laser or similar optical energy source. In one example, a microneedle and/or microcannula may be cut using a laser to represent the desired microneedle length. The laser may also be use to shape single or multiple tip openings. Single or multiple cuts may be performed on a single microcannula to shape the desired microneedle structure. In one example, the microcannula may be made of metal such as stainless steel and cut using a laser with a wavelength in the infrared region of the light spectrum (0.7-300 µm). Further refinement may be performed using metal electropolishing techniques familiar to those in the field. In another embodiment, the microneedle length and optional bevel can be formed by a physical grinding process, which, for example, may include grinding a metal cannula against a moving abrasive surface. The fabrication process may further include precision grinding, micro-bead jet blasting and/or ultrasonic cleaning to form the shape of the desired precise tip of the microneedle.

While various embodiments have been described above, it should be understood that they have been presented by way of example only, and not limitation. Where methods described above indicate certain events occurring in certain order, the ordering of certain events may be modified. Additionally, certain of the events may be performed concurrently in a parallel process when possible, as well as performed sequentially as described above

Where schematics and/or embodiments described above indicate certain components arranged in certain orientations or positions, the arrangement of components may be modified. Similarly, where methods and/or events described above indicate certain events and/or procedures occurring in certain order, the ordering of certain events and/or procedures may be modified. While the embodiments have been particularly shown and described, it will be understood that various changes in form and details may be made.

For example, although the microneedles are shown and described herein as being substantially linear (i.e., having a linear center line) and being substantially rigid, in other embodiments a microneedle, such as, for example, the microneedles 310, 410 and 610 can be curved and/or can define a substantially curved lumen therethrough. In yet other embodiments, any of the microneedles described herein (e.g., the microneedles 310, 410 and 610) can be flexible.

Although various embodiments have been described as having particular features and/or combinations of components, other embodiments are possible having a combination of any features and/or components from any of embodiments as discussed above.

For example, although the microneedles 310, 410, 510 and 610 can have the diameters and wall thickness as specified by the microneedle 710 shown and described above. Moreover, any of the microneedles described herein can have a variable-thickness wall, similar to that shown in the microneedle 910.

## Claims

1. An apparatus (100, 200, 1000), comprising:
a housing (130, 230, 1030), a proximal end portion (231) of the housing defining an opening (235) configured to receive a portion of a medicament cartridge (140, 240, 1040) therein, a distal end portion (232) of the housing (130, 230, 1030) including a base surface configured to contact a surface of a target tissue; and
a microneedle (110, 210, 1010) having a distal end portion (212) including a beveled surface (416) and a proximal end portion (211), and defining a lumen (214), wherein the microneedle is coupled to the distal end portion (232) of the housing (130, 230, 1030), the proximal end portion of the microneedle (110, 210, 1010) configured to be in fluid communication with the medicament cartridge (140, 240, 1040) when the portion of the medicament cartridge is disposed within the opening,
the base surface circumscribing the proximal end portion (211) of the microneedle (110, 210, 1010); **characterized in that** the apparatus comprises:
a pressure sensor (1363) and a pressure feedback control system (1360) operably coupled to the housing (130, 230, 1030) and configured to provide an indication of a pressure applied at the housing during an injection procedure; and
wherein the beveled surface (416) defines a first segment arranged at a first angle relative to a centerline (CL) of the lumen (214) defined by the microneedle (110, 210, 1010) and a second segment arranged at a second angle relative to the centerline (CL) of the lumen (214).

2. The apparatus of claim 1, wherein the microneedle (110, 210, 1010) is physically coupled to the distal end portion (232) of the housing (130, 230, 1030).

3. The apparatus of claim 1, wherein the base surface is substantially normal to the centerline of the lumen defined by the microneedle; and the microneedle is fixedly coupled to the base surface.

4. The apparatus of claim 1, wherein the beveled surface is defined in at least two planes.

5. The apparatus of claim 1, wherein the microneedle (110, 210, 1010) is rigid and is 30 gauge or smaller.

6. The apparatus of claim 1, wherein the housing (130, 230, 1030) is coupled to the medicament cartridge (140, 240, 1040) such that at least a portion of the medicament cartridge is disposed within the housing.

7. The apparatus of claim 1, wherein the base surface is substantially normal to the center line of the lumen defined by the microneedle.

8. The apparatus of claim 1, wherein the microneedle (110) is physically and/or fluidically coupled to the housing.

9. The apparatus of claim 1, wherein the housing (130) includes a set of annular walls that define an inner volume that is in fluid communication with the microneedle (110).

10. The apparatus of claim 1, wherein the medicament cartridge (140) is a prefilled syringe.

11. The apparatus of claim 1, wherein the medicament cartridge (140) can be moved from a first configuration a second configuration to expel the medicament through the lumen of the microneedle.

12. The apparatus of claim 1, wherein the first angle is from about 0.1 degree to about 30 degrees or from or from about 1 degree to about 25 degrees or from about 2 degrees to about 20 degrees or from about 10 degrees to about 20 degrees.

13. The apparatus of claim 1, wherein the medicament cartridge (240) includes a seal (246) and a plunger (245) configured to move the seal (246) within the medicament cartridge (240), and the pressure sensor (1363) is configured to provide an indication of a pressure applied to the plunger (245) during the injection procedure.

## Patentansprüche

1. Vorrichtung (100, 200, 1000), umfassend:
ein Gehäuse (130, 230, 1030), wobei ein proximaler Endabschnitt (231) des Gehäuses eine Öffnung (235) definiert, die so konfiguriert ist, dass sie einen Teil einer Medikamentenkartusche (140, 240, 1040) darin aufnimmt, wobei ein distaler Endabschnitt (232) des Gehäuses (130, 230, 1030) eine Grundfläche aufweist, die so konfiguriert ist, dass sie eine Oberfläche eines Zielgewebes berührt; und
eine Mikronadel (110, 210, 1010), die einen distalen Endabschnitt (212) mit einer abgeschrägten Oberfläche (416) und einen proximalen Endabschnitt (211) aufweist und ein Lumen (214) definiert, wobei die Mikronadel mit dem distalen Endabschnitt (232) des Gehäuses (130, 230, 1030) gekoppelt ist, wobei der proximale Endabschnitt der Mikronadel (110, 210, 1010) so konfiguriert ist, dass er in Fluidverbindung mit der Medikamentenkartusche (140, 240, 1040) steht, wenn der Abschnitt der Medikamentenkartusche innerhalb der Öffnung angeordnet ist,
wobei die Grundfläche den proximalen Endabschnitt (211) der Mikronadel (110, 210, 1010) abgrenzt; **dadurch gekennzeichnet, dass** die Vorrichtung umfasst:
einen Drucksensor (1363) und ein Druckrückkopplungssteuersystem (1360), das funktionsfähig mit dem Gehäuse (130, 230, 1030) gekoppelt und so konfiguriert ist, dass es eine Anzeige eines Drucks liefert, der während eines Injektionsvorgangs auf das Gehäuse ausgeübt wird; und
wobei die abgeschrägte Oberfläche (416) ein erstes Segment definiert, das in einem ersten Winkel relativ zu einer Mittellinie (CL) des Lumens (214) angeordnet ist, das durch die Mikronadel (110, 210, 1010) definiert ist, und ein zweites Segment, das in einem zweiten Winkel relativ zu der Mittellinie (CL) des Lumens (214) angeordnet ist.

2. Vorrichtung nach Anspruch 1, wobei die Mikronadel (110, 210, 1010) physisch mit dem distalen Endabschnitt (232) des Gehäuses (130, 230, 1030) verbunden ist.

3. Vorrichtung nach Anspruch 1, wobei die Grundfläche im Wesentlichen senkrecht zur Mittellinie des durch die Mikronadel definierten Lumens verläuft und die Mikronadel fest mit der Grundfläche verbunden ist.

4. Vorrichtung nach Anspruch 1, wobei die abgeschrägte Oberfläche in mindestens zwei Ebenen definiert ist.

5. Vorrichtung nach Anspruch 1, wobei die Mikronadel (110, 210, 1010) starr ist und eine Stärke von 30 Gauge oder weniger hat.

6. Vorrichtung nach Anspruch 1, wobei das Gehäuse (130, 230, 1030) mit der Medikamentenkartusche (140, 240, 1040) gekoppelt ist, so dass mindestens ein Teil der Medikamentenkartusche innerhalb des Gehäuses angeordnet ist.

7. Vorrichtung nach Anspruch 1, wobei die Grundfläche im Wesentlichen senkrecht zur Mittellinie des durch die Mikronadel definierten Lumens ist.

8. Vorrichtung nach Anspruch 1, wobei die Mikronadel (110) physikalisch und/oder fluidisch mit dem Gehäuse gekoppelt ist.

9. Vorrichtung nach Anspruch 1, wobei das Gehäuse (130) einen Satz ringförmiger Wände aufweist, die ein Innenvolumen definieren, das in Fluidverbindung mit der Mikronadel (110) steht.

10. Vorrichtung nach Anspruch 1, wobei die Medikamentenkartusche (140) eine vorgefüllte Spritze ist.

11. Vorrichtung nach Anspruch 1, wobei die Medikamentenkartusche (140) von einer ersten Konfiguration in eine zweite Konfiguration bewegt werden kann, um das Medikament durch das Lumen der Mikronadel auszustoßen.

12. Vorrichtung nach Anspruch 1, wobei der erste Winkel von etwa 0,1 Grad bis etwa 30 Grad oder von oder von etwa 1 Grad bis etwa 25 Grad oder von etwa 2 Grad bis etwa 20 Grad oder von etwa 10 Grad bis etwa 20 Grad beträgt.

13. Vorrichtung nach Anspruch 1, wobei die Medikamentenkartusche (240) eine Dichtung (246) und einen Kolben (245) aufweist, der so konfiguriert ist, dass er die Dichtung (246) innerhalb der Medikamentenkartusche (240) bewegt, und der Drucksensor (1363) so konfiguriert ist, dass er eine Anzeige eines Drucks liefert, der während des Injektionsverfahrens auf den Kolben (245) ausgeübt wird.

## Revendications

1. Appareil (100, 200, 1000), comportant :
un logement (130, 230, 1030), une partie d'extrémité proximale (231) du logement définissant une ouverture (235) configurée pour recevoir une partie d'une cartouche de médicament (140, 240, 1040) dans celle-ci, une partie d'extrémité distale (232) du logement (130, 230, 1030) comprenant une surface de base configurée pour entrer en contact avec une surface d'un tissu cible ; et
une micro-aiguille (110, 210, 1010) ayant une partie d'extrémité distale (212) comprenant une surface biseautée (416) et une partie d'extrémité proximale (211), et définissant une lumière (214), la micro-aiguille étant couplée à la partie d'extrémité distale (232) du logement (130, 230, 1030), la partie d'extrémité proximale de la micro-aiguille (110, 210, 1010) étant configurée pour être en communication fluidique avec la cartouche de médicament (140, 240, 1040) lorsque la partie de la cartouche de médicament est disposée dans l'ouverture,
la surface de base circonscrivant la partie d'extrémité proximale (211) de la micro-aiguille (110, 210, 1010) ; **caractérisé en ce que** l'appareil comporte :
un capteur de pression (1363) et un système de commande de rétroaction de pression (1360), couplés de manière opérationnelle au logement (130, 230, 1030) et configurés pour fournir une indication d'une pression appliquée au niveau du logement pendant une procédure d'injection ; et
la surface biseautée (416) définissant un premier segment disposé dans un premier angle par rapport à une ligne centrale (CL) de la lumière (214) définie par la micro-aiguille (110, 210, 1010) et un second segment disposé dans un second angle par rapport à la ligne centrale (CL) de la lumière (214).

2. Appareil selon la revendication 1, dans lequel la micro-aiguille (110, 210, 1010) est physiquement couplée à la partie d'extrémité distale (232) du boîtier (130, 230, 1030).

3. Appareil selon la revendication 1, dans lequel la surface de base est sensiblement normale à la ligne centrale de la lumière définie par la micro-aiguille, et la micro-aiguille est couplée de manière fixe à la surface de base.

4. Appareil selon la revendication 1, dans lequel la surface biseautée est définie dans au moins deux plans.

5. Appareil selon la revendication 1, dans lequel la micro-aiguille (110, 210, 1010) est rigide et est de calibre 30 ou inférieur.

6. Appareil selon la revendication 1, dans lequel le logement (130, 230, 1030) est couplé à la cartouche de médicament (140, 240, 1040) de sorte qu'au moins une partie de la cartouche de médicament est disposée à l'intérieur du logement.

7. Appareil selon la revendication 1, dans lequel la surface de base est sensiblement normale à la ligne centrale de la lumière définie par la micro-aiguille.

8. Appareil selon la revendication 1, dans lequel la micro-aiguille (110) est couplée au logement de manière physique et/ou fluidique.

9. Appareil selon la revendication 1, dans lequel le boîtier (130) comporte un ensemble de parois annulaires qui définissent un volume intérieur qui est en communication fluidique avec la micro-aiguille (110).

10. Appareil selon la revendication 1, dans lequel la cartouche de médicament (140) est une seringue préremplie.

11. Appareil selon la revendication 1, dans lequel la cartouche de médicament (140) peut être déplacée d'une première configuration à une seconde configuration pour expulser le médicament à travers la lumière de la micro-aiguille.

12. Appareil selon la revendication 1, dans lequel le premier angle est compris entre environ 0,1 degré et environ 30 degrés ou entre environ 1 degré et environ 25 degrés ou entre environ 2 degrés et environ 20 degrés ou entre environ 10 degrés et environ 20 degrés.

13. Appareil selon la revendication 1, dans lequel la cartouche de médicament (240) comporte un joint (246) et un piston (245) configuré pour déplacer le joint (246) à l'intérieur de la cartouche de médicament (240), et le capteur de pression (1363) est configuré pour fournir une indication d'une pression appliquée au piston (245) pendant la procédure d'injection.
